(19) Europäisches Patentamt
European Patent Office
Office européen des brevets

(11) EP 4 177 255 A1

(12) EUROPEAN PATENT APPLICATION
published in accordance with Art. 153(4) EPC

(43) Date of publication:
**10.05.2023 Bulletin 2023/19**

(21) Application number: **21834664.1**

(22) Date of filing: **29.06.2021**

(51) International Patent Classification (IPC):
***C07D 413/14*** (2006.01) ***C07D 413/12*** (2006.01)
***C07D 271/06*** (2006.01) ***A61K 31/4245*** (2006.01)
***A61P 3/00*** (2006.01)

(52) Cooperative Patent Classification (CPC):
**A61K 31/4245; A61K 31/4439; A61K 31/4725; A61K 31/498; A61K 31/675; A61P 1/04; A61P 1/16; A61P 3/00; A61P 3/06; A61P 3/10; A61P 9/10; A61P 17/00; A61P 37/02; C07D 271/06; C07D 413/10;** (Cont.)

(86) International application number:
**PCT/CN2021/103263**

(87) International publication number:
**WO 2022/002078 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 CN 202010622053**

(71) Applicant: **Shanghai Institute of Materia Medica, Chinese Academy of Sciences
Pudong, Shanghai 201203 (CN)**

(72) Inventors:
- **LIU, Hong**
  **Shanghai 201203 (CN)**
- **WANG, Jiang**
  **Shanghai 201203 (CN)**
- **XIE, Cen**
  **Shanghai 201203 (CN)**
- **LIU, Yameng**
  **Shanghai 201203 (CN)**
- **HU, Shulei**
  **Shanghai 201203 (CN)**
- **LI, Cuina**
  **Shanghai 201203 (CN)**
- **GAO, Feng**
  **Shanghai 201203 (CN)**
- **WANG, Kanglong**
  **Shanghai 201203 (CN)**
- **WANG, Yong**
  **Shanghai 201203 (CN)**
- **ZHONG, Xianchun**
  **Shanghai 201203 (CN)**
- **SHI, Yuqiang**
  **Shanghai 201203 (CN)**
- **JIANG, Hualiang**
  **Shanghai 201203 (CN)**
- **CHEN, Kaixian**
  **Shanghai 201203 (CN)**

(74) Representative: **Lavoix
Bayerstraße 83
80335 München (DE)**

# (54) OXADIAZOLE COMPOUND, PREPARATION METHOD THEREFOR, PHARMACEUTICAL COMPOSITION AND USE THEREOF

(57) The present invention relates to the fields of pharmaceutical chemistry and pharmacotherapeutics,and in particular to a compound as represented by general formula I, a racemate, an R-isomer, an S-isomer and a pharmaceutically acceptable salt thereof and a mixture of same, a preparation method therefor, a pharmaceutical composition containing the compound and the use thereof as an SIP receptor agonist. The oxadiazole compound involved in the present invention can be used for treating SIP receptor agonist related diseases.

EP 4 177 255 A1

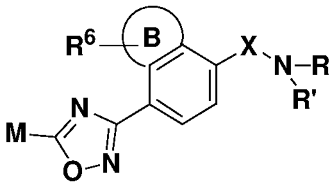

I

(52) Cooperative Patent Classification (CPC): (Cont.)
**C07D 413/12; C07D 413/14; C07F 9/6558**

## Description

### Technical field

The invention relates to the field of pharmaceutical chemistry and drug therapy, in particular to a class of oxadiazole compounds, a preparation method therefor, pharmaceutical compositions containing such compounds and as S1P receptor agonists, especially the use in the preparation a drug for the treatment of non-alcoholic fatty liver, liver fibrosis, diabetes, hyperlipidemia, multiple sclerosis (includingrelapsing multiple sclerosis, relapsing-remitting multiple sclerosis, active secondary progressive multiple sclerosis), psoriasis, ulcerative colitis, lupus erythematosus, Crohn's disease, immune disorders, wet age-related macular degeneration, atopic dermatitis, inflammatory bowel disease, clinical isolated syndromeand other diseases.

### Background

Non-alcoholic fatty liver disease (NAFLD) is a common chronic liver disease that is a serious threat to the national health of China.Epidemiological surveys show that the prevalence of NAFLD in China has increased dramatically from 18% in 2008 to 29.2% in 2018, more than twice the growth rate of western countries, and about 58.9% of patients with biopsy-confirmed NAFLD present with non-alcoholic steatohepatitis (NASH) form.Currently, NAFLD has replaced chronic viral liver disease as the top chronic liver disease in China.It is estimated that by 2030, China will have the fastest incidence growth rate in the world, reaching 314.58 million people.In the foreseeable future,NASH will become a major public health challenge in China.

Lipid metabolism is one of the core of hepatic metabolism, and there is increasing evidence that triglycerides deposited in hepatocytes are not the main cause of lipotoxicity, and that lowering triglycerides alone is not effective in improving NASH, so new interventions to improve lipid disorders are urgently needed. Triglycerides are the predominant form of lipids in simple fatty liver, and about 25% of patients progress from simple fatty liver to NASH with a more pronounced lipotoxic phenotype, in which reprogramming of the lipid metabolic network may be one of the most important driving factors, but little is known about it.In the last decade, new insights into disorders of lipid metabolism have emerged, with cholesterol, free fatty acids, lysophosphatidylcholine and sphingolipids being identified as important lipids causing lipotoxicity. Among them, sphingolipids are a class of lipids containing a sphingosine backbone, and the metabolites ceramide and sphingosine 1-phosphate (S1P) are two of the most studied sphingolipid bioactive molecules.In vivo, ceramide is generally synthesized through the three pathways of de novo synthesis, sphingomyelinase hydrolysis and remedial synthesis, and then acetylated under the action of ceramidase (ceramidase,CDase) to produce sphingosine, which is further phosphorylated by sphingosine kinase (sphingosine kinase,SphK) to S 1P.The intracellular S1P can function as a second messenger for bioregulation, while the S1P secreted outside the cell activates downstream signaling pathways such as PI3K/Akt, Ras/ERK, Rho, Rac, etc. through five cell surface G protein-coupled receptors (GPCRs) S1P receptors 1-5 (S1PR1-5), causing a series of biological effects such as cell survival, proliferation, differentiation and migration.The five isoforms are distributed differently in each tissue, S1PR1, S1PR2 and S1PR3 are widely present in numerous tissues, including the liver, while S1PR4 is widely expressed in lymphoid and lung tissues, and S1PR5 is expressed in the brain and skin, thus performing different functions.Normal levels of S 1P are involved in a variety of vital physiological processes, such as cell growth, survival and migration, and vascular neogenesis and maturation, but excess S1P can also induce many pathological changes, including inflammation and fibrosis, two pathological processes closely associated with NASH progression.Data from clinical studies have found that sphingolipid levels in vivo show a positive correlation with NAFLD progression, particularly ceramide and S 1P levels in the liver, which rise dramatically upon progression to NASH, and that lowering ceramide levels or modulating downstream S1P signaling pathways can ameliorate metabolic disturbances.

Intrinsic immune activation is a key factor in triggering and amplifying hepatic inflammation, which plays a central role in promoting the transition from simple fatty liver to NASH.Kupffer cells (KCs) are macrophages located in the hepatic sinusoids, account for 20 - 35% of all non-parenchymal cells in the liver and are the most important class of cells in hepatic intrinsic immunity. The cytokines released by KCs can mediate the inflammatory cascade response, induce hepatocyte death and lipid degeneration, and promote the activation of hepatic stellate cells (HSCs).HSCs are the main source of extracellular matrix and play a key role in the formation of liver fibrosis. HSCs differentiate into myofibroblast-like cells upon activation, which have contractile, pro-inflammatory effects as well as pro-fibrotic properties.S1P can accelerate NASH progression by acting on S1PR1 and S1PR3 on the surface of liver macrophages to initiate an intrinsic immune response.S 1P can also directly induce the activation, proliferation and contraction of HSCs by acting on S1PR1-3 on the surface of HSCs and promote the secretion of extracellular matrix[8].Studies have shown that regulating the activities of S1PR1 and S1PR3 in mice through drugs or gene knockout can effectively alleviate the progress of inflammation and fibrosis in various liver disease models. Therefore, S1P receptors are a promising new target against metabolic diseases such as NASH.In particular, it is a new target for type II diabetes with a fasting

hyperglycemic phenotype.

In summary, there is an urgent need in the art to develop more S1P receptor regulators in the field.

## SUMMARY OF THE INVENTION

The purpose of the present invention is to provide an S1P receptor agonist.

The first aspect of the present invention provides a compound as shown in the following formula I, a pharmaceutically acceptable salt thereof, a racemate, a R-isomer, an S-isomer or a mixture thereof:

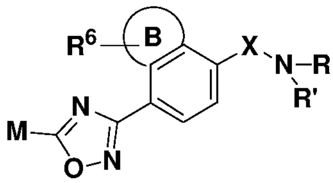

I

wherein,

M is selected from the group consisting of substituted or unsubstituted C1-C6 alkyl, substituted or unsubstituted C1-C6 alkenyl, substituted or unsubstituted 3- to 12-membered saturated aliphatic ring, substituted or unsubstituted 3- to 12-membered unsaturated aliphatic ring, substituted or unsubstituted 3- to 12-membered aliphatic ring containing 1 to 8 heteroatoms, substituted or unsubstituted 9- to 12-membered aromatic fused ring, substituted or unsubstituted C6-C10 aromatic ring (preferably a benzene ring) and substituted or unsubstituted 5-12-membered aromatic heterocycle containing 1 to 4 heteroatoms selected from group consisting of oxygen, sulfur and nitrogen; wherein the substituted in M group means the group may be substituted by one or more R1 groups; and

each $R^1$ is independently selected from the group consisting of: hydrogen, deuterium, tritium, halogen, cyano, amino, hydroxyl, nitro, aldehyde group, substituted or unsubstituted guanyl, substituted or unsubstituted C1 to C6 alkyl (including trifluoromethyl), substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl 5- to 7-membered heteroaryl, substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^4$, $-OCOR^4$, and $SO_2R^4$;

X is selected from the group consisting of: $CHR^2$, $NR^2$, O and S;

$R^2$ is selected from the group consisting of: hydrogen, deuterium, tritium, halogen, cyano, amino, hydroxyl, nitro, aldehyde group, substituted or unsubstituted guanyl, substituted or unsubstituted guanidino, substituted or unsubstituted C1 to C6 alkyl (containing trifluoromethyl), C1 to C3 alkyl substituted by 1-7 fluorine atoms, substituted or unsubstituted C1 to C6 alkoxy, substituted or unsubstituted C6 to C10 aryl, substituted or unsubstituted 5- to 7 -membered heterocycle, substituted or unsubstituted C1 to C6 alkyl phenyl, substituted or unsubstituted C1 to C6 alkyl 5- to 7- membered heteroaryl, substituted or unsubstituted C3 to C12 cycloalkyl, substituted or unsubstituted C2 to C10 acyl, substituted or unsubstituted C2 to C10 ester, substituted or unsubstituted C1 to C6 amide, - $SO_2R^5$, and $-COR^5$;

R and R' are independently selected from the group consisting of: H, substituted or unsubstituted C1-C6 alkyl, substituted or unsubstituted 3-12 member saturated aliphatic ring; or R, R' and the N atom to which they connect together form the following structure:

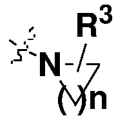

n is 1, 2, 3, 4, 5, 6, or 7;

$R^3$ is selected from the group consisting of: carboxyl, sulfonic acid group ($-SO_2H$), phosphate group ($-PO_3H$), substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^5$, - $OCOR^5$, $-C(O)R^5$,$-C(O)OR^5$,$-SO_2R^5$, and $-PO(OR^5)_2$;

ring B is selected from the group consisting of: 5- to 10-membered saturated or unsaturated carbon-ring, 5- to 10-membered saturated or unsaturated heterocycle; wherein the heterocycle has one or more N atoms as ring member; $R^4$ and $R^5$ are independently selected from the group consisting of: hydrogen, deuterium, tritium, amino, hydroxyl, substituted or unsubstituted C1 to C6 alkyl (including trifluoromethyl), C1 to C3 alkyl containing 1-7 fluorine atoms, substituted or unsubstituted C6 to C10 aryl, substituted or unsubstituted 5-7 membered heterocycle, substituted or unsubstituted C1 to C6 alkyl phenyl, substituted or unsubstituted C1 to C6 alkyl 5-7 membered heteroaryl, substituted or unsubstituted C3 to C12 cycloalkyl, substituted or unsubstituted C2 to C10 acyl, substituted or unsubstituted C2 to C10 ester, and substituted or unsubstituted C1 to C6 amide;

$R^6$ is one or more groups on the ring

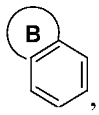

and selected from the group consisting of: hydrogen, deuterium, tritium, substituted or unsubstituted C1 to C6 alkyl; wherein, the substituted means one or more hydrogen atoms on the group being substituted by a substituent selected from the group consisting of: halogen, hydroxyl, carboxyl, benzyl, $C_1$-$C_6$alkoxycarbonyl, amino,$C_1$-$C_6$amido, nitro, cyano, unsubstituted or halogenated $C_1$-$C_6$ alkyl, $C_2$-$C_{10}$ alkenyl, $C_1$-$C_6$ alkoxy, $C_1$-$C_6$ alkyl-amine, $C_6$-$C_{10}$ aryl, five-membered or six-membered heteroaryl, -O-($C_6$-$C_{10}$ aryl), and -O-(five-membered or six-membered heteroaryl).

In another preferred embodiment, the compound has the structure shown in formula II:

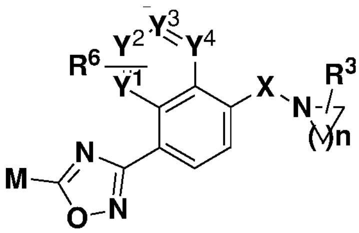

II

wherein,

$Y^1$, $Y^2$, $Y^3$, and $Y^4$ are each independently selected from N or CH, and when $Y^1$, $Y^2$, $Y^3$, or $Y^4$ is CH, the CH may be substituted by $R^6$.

In another preferred embodiment, the compound has the structure shown in formula III:

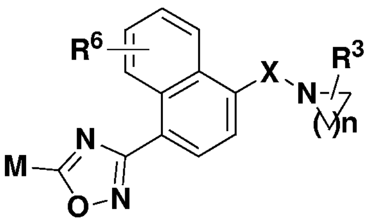

III

Wherein, X is $CHR^2$.

In another preferred embodiment, the M has a structure as shown in the following formula:

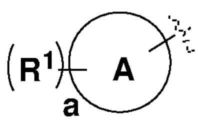

wherein,

A is selected from the group consisting of: substituted or unsubstituted 3-12 membered saturated aliphatic ring, substituted or unsubstituted 3-12 membered unsaturated aliphatic rings, substituted or unsubstituted 3-12 membered aliphatic rings containing 1-8 heteroatoms, substituted or unsubstituted 7-12 membered aromatic fused ring, substituted or unsubstituted C6-C10 aromatic ring (preferably benzene ring), and substituted or unsubstituted 5-12 membered aromatic heterocycle containing 1 to 4 heteroatoms selected from the group consisting of oxygen, sulfur and nitrogen;

each $R^1$ is independently selected from the group consisting of: hydrogen, deuterium, tritium, halogen, cyano, amino, hydroxyl, nitro, aldehyde group, substituted or unsubstituted C1 to C6 alkyl (including trifluoromethyl), C1 to C3 alkyl containing 1-7 fluorine atoms, substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl (5- to 7-membered heteroaryl), substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^4$, $-OCOR^4$, and $SO_2R^4$;

a is 1, 2, 3, 4, 5, 6, 7, 8, 9, 10 or 11.

In another preferred embodiment, ring A is selected from the group consisting of: substituted or unsubstituted benzene ring, substituted or unsubstituted 5-7 membered saturated aliphatic ring, substituted or unsubstituted 5- to 10-membered aromatic heterocycle containing 1 to 4 heteroatoms selected from oxygen, sulfur and nitrogen, substituted or unsubstituted partially unsaturated 5- to 10- membered heterocycle containing 1 to 4 heteroatoms selected from oxygen, sulfur and nitrogen;

each $R^1$ is independently selected from the group consisting of: hydrogen, deuterium, tritium, halogen, cyano, amino, hydroxyl, nitro, aldehyde group, substituted or unsubstituted C1 to C6 alkyl (including trifluoromethyl), C1 to C3 alkyl containing 1-7 fluorine atoms, substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl (5- to 7-membered heteroaryl), substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^4$, $-OCOR^4$, and $SO_2R^4$;

a is 1, 2, 3, 4 or 5.

In another preferred embodiment, the compound is a compound of various Examples of the present invention.

The second aspect of the present invention provides a pharmaceutical composition comprising one or more of the compounds of formula I according to the first aspect of the present invention, a pharmaceutically acceptable salt thereof, a racemate, an R-isomers, an S-isomers and a mixture thereof, and one or more pharmaceutically acceptable carriers, excipients, adjuvants, ingredients and/or diluents.

The third aspect of the present invention provides a use of the compound of formula I according to the first aspect of the present invention, a pharmaceutically acceptable salt thereof, a racemate, an R-isomer, an S-isomer or a mixture thereof, in the the preparation of a pharmaceutical composition for the treatment or prevention of diseases associated with S1P agonists.

In another preferred embodiment, the disease is selected from the group consisting of non-alcoholic fatty liver disease, liver fibrosis, diabetes, hyperlipidemia, multiple sclerosis (includingrelapsing multiple sclerosis, relapsing-remitting multiple sclerosis, active secondary progressive multiple sclerosis), psoriasis, ulcerative colitis, lupus erythematosus, Crohn's disease, immune disorders, wet age-related macular degeneration, atopic dermatitis, inflammatory bowel disease,and clinical isolated syndrome.

The fourth aspect of the present invention provides A method for preparing the compound of formula I according to the first aspect of the present invention, a pharmaceutically acceptable salt thereof, a racemate, an R-isomer, an S-isomer or a mixture thereof, including the steps:

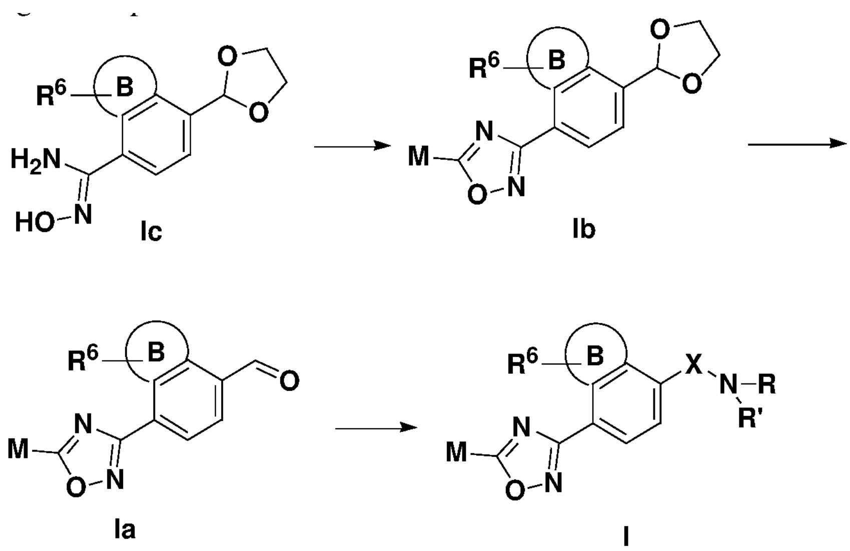

Ic Ib Ia I

(1) in DMF, reacting the compound of formula Ic with M-COOH, HOBT, EDCI and potassium carbonate to give the compound of formula Ib;

(2) in acetone/dilute hydrochloric acid, reacting the compound of formula Ib to obtain the compound of formula Ia;

(3) in a methanol/dichloromethane mixture, reacting the compound of formula Ia with the hydrochlorideof

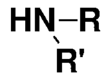

, DIPEA, acetic acid and sodium cyanoborohydride to give the compound of formula I; wherein X is CH.

It should be understood that within the scope of the present invention, the above-mentioned technical features of the present invention and the technical features specifically described in the following (such as embodiments) can be combined with each other to form a new or preferred technical solution. Limited to space, it will not be repeated here.

## DESCRIPTION OF THE DRAWINGS

Figure 1: A1 alleviated obesity symptoms in the GAN diet-induced NASH mouse model, wherein, (A) body weight; (B) food intake; (C) body fat content; (D) fat/lean ratio; (E) subcutaneous adipose (sWAT), epididymal adipose (eWAT) and brown adipose weight (BAT); (F) body temperature; (G) oxygen consumption; (H) carbon dioxide production; and (I) energy expenditure.When comparing LD group with Model group,# P < 0.05,### P < 0.005; When A1 group is compared with Model group, * P < 0.05,**P < 0.05,*** P < 0.005.

Figure 2: A1 alleviated insulin resistance symptoms in the GAN diet-induced NASH mouse model, wherein (A) fasting blood glucose; (B) fasting insulin; (C) HOMA-IR index; (D) GTT; (E) area under the GTT curve; (F) ITT; (G) area under the ITT curve.When comparing LD group with Model group,# P < 0.05,## P < 0.01,### P < 0.005; When comparing group A1 with model group, * P < 0.05,**P < 0.05,*** P < 0.005.

Figure 3: A1 improved serological and hepatic biochemical indexes in the GAN diet-induced NASH mouse model, wherein, (A) liver weight; (B) liver weight/body weight; (C) serum ALT; (D) serum AST; (E) serum triglycerides; (F) serum T-CHO; (G) serum HDL-C; (H) serum LDL-C; (I) liver triglycerides; (1) total liver cholesterol.When comparing LD group with Model group,## P < 0.01,### P < 0.005; When comparing A1 group with Model group, * P < 0.05,**P < 0.05,*** P < 0.005.

Figure 4: A1 improved hepatic lipid metabolism disorders, inflammation and fibrosis in a GAN diet-induced NASH mouse model, wherein, (A) mRNA expression of genes related to fatty acid ab initio synthesis, transport and β-oxidative metabolism; (B) mRNA expression of genes related to hepatic inflammation; (C) mRNA expression of genes related to hepatic fibrosis. *P < 0.05,**P < 0.05,*** P < 0.005.

Figure 5: Histopathological examination of A1 improved GAN diet-induced NASH mouse model, wherein (A) hepatic H&E staining, Oil Red O staining and Sirius scarlet staining; (B) NAS score; (C) quantitative results of hepatic Oil

Red O staining; (D) quantitative results of hepatic Sirius scarlet staining; *P < 0.05, **P < 0.05, ***P < 0.005.

Figure 6:compound A1 reduced ALT level in serum in $CCl_4$ induced liver fibrosis model,wherein, (A) body weight;(B) liver weight; (C) spleen weight; (D) serum ALT;(E) liver index; (f) spleen index; *P < 0.05,**P < 0.05,***P < 0.005;

Figure 7:High-dose A1 downregulates mRNA expression of liver fibrosis-related genes *(Tgfb, Col1a1)* at the gene level; wherein, (A) relative mRNA expression of *Tgfb*; (B) relative mRNA expression of *Col1a1*; *P < 0.05, **P < 0.05, ***P < 0.005.

Figure 8: Pathological section analysis of high-dose A1 alleviating hydroxyproline content in liver; wherein, (A) hepatic hydroxyproline concentration; (B) area fraction of Sirius red staining; (C) pathological section; *P < 0.05, **P < 0.05, ***P < 0.005.

Figure 9: Compound A1 reduced serum ALT and AST levels in a methionine-choline deficient combined with high-fat model feed diet (MCD-HFD) induced NASH model, while significantly alleviating TG accumulation in the liver; wherein, (A) body weight; (B) liver index; (C) spleen weight; (D) serum ALT; (E) serum AST; (F) liver triglycerides; *P < 0.05, **P < 0.05, ***P < 0.005.

Figure 10: A1 alleviated hepatic lipid accumulation as well as inflammatory infiltration at high/medium/low doses; where (A) relative gene expression; (B) H&E-stained liver pathology sections; *P < 0.05, **P < 0.05, ***P < 0.005.

Figure 11: High/medium dose A1 alleviated hydroxyproline content in liver and downregulates expression levels of fibrosis-related genes (*Asma, Tgfb, Col1a1*) at the gene level; wherein, (A) hepatic hydroxyproline content; (B) relative expression of *Asma, Tgfb, Col1a1* genes; *P < 0.05, **P < 0.05, ***P < 0.005.

Figure 12: High/medium/low doses of A1 alleviated the disease process in the dextran sodium sulfate (DSS)-induced IBD model; wherein, (A) weight change; (B) stool consistency index; (C) rectal bleeding index; (D) disease activity index; *P < 0.05, **P < 0.05, ***P < 0.005.

Figure 13: The effect of high/medium/low doses of A1 in the dextran sodium sulfate (DSS)-induced IBD model; wherein, (A) lesioned colon site; (B) colon length; (C) rectal weight; (D) colon pathology section; *P < 0.05, **P < 0.05, ***P < 0.005.

## DETAILED DESCRIPTION OF THE EMBODIMENT

The present inventors have found anoxadiazolecompound represented by the general formula I, a pharmaceutically acceptable salt thereof, a racemate, an R-isomer, an S-isomer, or a mixture thereof after a long and intensive study.The compound is an S1P receptor agonist, therefore, it can be used to prepare and treat indications related to S1P receptor agonists (such as non-alcoholic fatty liver, liver fibrosis, diabetes, hyperlipidemia, multiple sclerosis (includingrelapsing multiple sclerosis, Relapsing-remitting multiple sclerosis, active secondary progressive multiple sclerosis), psoriasis, ulcerative colitis, lupus erythematosus, Crohn's disease, immune disorders, wet age-related macular degeneration, atopic dermatitis, inflammatory bowel disease,Clinical isolated syndrome, etc.) composition.The present invention has been completed on this basis.

### Terms

In the present invention, the halogen is F, Cl, Br, or I.

In the present invention, unless specifically indicated, the terms used have a general meaning well known to those skilled in the art.

In the present invention, the term "C1-C6 alkyl" refers to a linear or branched alkyl having 1 to 6 carbon atoms, including, without limitation, methyl, ethyl, propyl, isopropyl, butyl, isobutyl, sec-butyl, tert-butyl, pentyl, and hexyl; preferably ethyl, propyl, isopropyl, butyl, isobutyl, sec-butyl, and tert-butyl.

In the present invention, the term "C1-C6 alkoxy" refers to a linear or branched alkoxy having 1 to 6 carbon atoms, including, without limitation, methoxy, ethoxy, propoxy, isopropoxy, butoxy, and the like.

In the present invention, the term "C2-C6 alkenyl" refers to a linear or branched alkenyl having 2 to 6 carbon atoms and containing one double bond, including, without limitation, vinyl, propenyl, butenyl, isobutyl, pentenyl, hexenyl, and the like.

In the present invention, the term "C2-C6 alkynyl" refers to a linear or branched alkynyl having 2 to 6 carbon atoms and containing a triple bond, including, without limitation, ethynyl, propynyl, butynyl, isobutynyl, pentynyl, hexynyl, and the like.

In the present invention, the term "C3-C10 cycloalkyl" refers to a cyclic alkyl having 3 to 10 carbon atoms on the ring, including, without limitation, cyclopropyl, cyclobutyl, cyclopentyl, cyclohexyl, cycloheptyl, cyclooctyl, and cyclodecyl. The terms "C3-C8 cycloalkyl", "C3-C7 cycloalkyl", and "C3-C6 cycloalkyl" have similar meanings.

In the present invention, the term "C3-C10 cycloalkenyl" refers to a cyclic alkenyl having 3 to 10 carbon atoms on the ring, including, without limitation, cyclopropenyl, cyclobutenyl, cyclopentyl, cyclohexenyl, cycloheptene, cyclooctenyl, and cyclodecylenyl, and the like. The term "C3-C7 cycloalkenyl" has a similar meaning.

In the present invention, the term "aromatic ring" or "aryl" has the same meaning, and preferably "aryl" is "C6-C12 aryl" or "C6-C10 aryl". The term "C6-C12 aryl" refers to an aromatic ring group having 6 to 12 carbon atoms and does not contain heteroatoms on the ring, such as phenyl, naphthyl, etc. The term "C6-C10 aryl" has a similar meaning.

In the present invention, the term "aromatic heterocycle" or "heteroaryl" has the same meaning and refers to a heteroaromatic group containing one or more heteroatoms. The heteroatoms referred herein include oxygen, sulfur and nitrogen. For example, furyl, thienyl, pyridyl, pyrazolyl, pyrrolyl, N-alkyl pyrrolyl, pyrimidine, pyrazine, imidazolyl, tetrazolyl, etc. The heteroaryl ring may be fused to an aryl, heterocyclyl or cycloalkyl ring, wherein the ring connected to the parent structure is a heteroaryl ring. The heteroaryl may be optionally substituted or unsubstituted.

In the present invention, the term "3-12-membered heterocyclyl" refers to a saturated or unsaturated 3-12-membered cyclic group containing 1 to 3 heteroatoms selected from oxygen, sulfur, and nitrogen on the ring, such as dioxacyclopentyl. The term "3-7-membered heterocyclyl" has a similar meaning.

In the present invention, the term "substituted" means that one or more hydrogen atoms on a specific group are substituted by a specific substituent. The specific substituents are the substituents described in the foregoing, or the substituents that appear in each embodiment. Unless otherwise specified, a substituted group may have a substituent selected from a specific group at any of the substituted sites of the group, and the substituents may be the same or different in each position. Cyclic substituents, such as heterocycloalkyl, can be attached to another ring, such as cycloalkyl, to form a spiro bicyclic system, for example, two rings have a common carbon atom. Those skilled in the art will understand that the combinations of substituents contemplated by the present invention are those that are stable or chemically achievable. The substituents are, for example, but not limited to, C1-8 alkyl, C2-8 alkenyl, C2-8 alkynyl, C3-8 cycloalkyl, 3-to 12-membered heterocyclyl, aryl, heteroaryl, halogen, hydroxyl, carboxyl (-COOH), C1-8 aldehyde group, C2-10 acyl, C2-10 ester, amino, alkoxy, C1-10 sulfonyl, and the like.

**Oxadiazolecompounds as S1P receptor agonists**

Based on the purpose of the present invention, the present invention provides an oxadiazole compound with the following ormula I, a racemate, a R-isomer, an S-isomer a pharmaceutically acceptable salt thereof, or a mixture thereof:

formula I

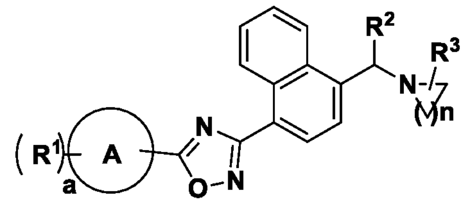

I

wherein,

A is selected from the group consisting of: substituted or unsubstituted 3-12 membered saturated aliphatic ring, substituted or unsubstituted 3-12 membered unsaturated aliphatic rings, substituted or unsubstituted 3-12 membered aliphatic rings containing 1-8 heteroatoms, substituted or unsubstituted 7-12 membered aromatic fused ring, substituted or unsubstituted C6-C10 aromatic ring (preferably benzene ring), and substituted or unsubstituted 5-12 membered aromatic heterocycle containing 1 to 4 heteroatoms selected from the group consisting of oxygen, sulfur and nitrogen; wherein the substituted naphthalene ring, substituted benzene ring or substituted aromatic heterocycle, each ring includes 1 to 7 substituents;

Each $R^1$ is independently selected from the group consisting of: hydrogen, deuterium, tritium, halogen, cyano, amino, hydroxyl, nitro, aldehyde group, substituted or unsubstituted C1 to C6 alkyl (containing trifluoromethyl), C1 to C3 alkyl containing 1-7 fluorine atoms, substituted or unsubstituted C1 to C6 alkoxy (including cyclic and chain alkoxy), substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl 5- to 7-membered heteroaryl, substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, -OSO2R4, -OCOR4, and SO2R4;

a is 1, 2, 3, 4, 5, 6, 7, 8, 9, 10 or 11;(naphthyl, cyclohexyl, etc. may be multi-substituted)

$R^2$ is selected from the group consisting of: hydrogen, deuterium, tritium, halogen, cyano, amino, hydroxyl, nitro,

aldehyde group, substituted or unsubstituted guanyl, substituted or unsubstituted guanidino, substituted or unsubstituted C1 to C6 alkyl (containing trifluoromethyl), C1 to C3 alkyl substituted by 1-7 fluorine atoms, substituted or unsubstituted C1 to C6 alkoxy, substituted or unsubstituted C6 to C10 aryl, substituted or unsubstituted 5-7 -membered heterocycle, substituted or unsubstituted C1 to C6 alkyl phenyl, substituted or unsubstituted C1 to C6 alkyl 5-7 -membered heteroaryl, substituted or unsubstituted C3 to C12 cycloalkyl, substituted or unsubstituted C2 to C10 acyl, substituted or unsubstituted C2 to C10 ester, substituted or unsubstituted C1 to C6 amide, $-SO_2R_5$, and $- COR_5$;

n is 1, 2, 3, 4, 5, 6, or 7;

$R^3$ is carboxyl, sulfonic acid group, phosphate, substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^5$, $-OCOR^5$, $-C(O)R_5$,$-C(O)OR^5$,$-SO_2R_5$, and $-PO(OR_5)_2$;

In a more preferred embodiment of the present invention, the compound of the formula I of the present invention is preferably the following specific compound:

| No. | Name | Structure |
|---|---|---|
| A1 | 1-((4-(5-(3-cyano-4-isopropoxyphenyl)-1,2,4-oxadiazol-3-yl) naphthalen-1-yl) methyl)azetidine-3-carboxylic acid hydrochloric acid | 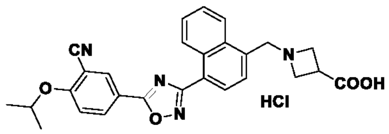 |
| A2 | 1-((4-(5-(3-cyano-4-isopropoxyphenyl)-1,2,4-oxadiazol-3-yl) naphthalen-1-yl) methyl)pyrrolidin-3-carboxylic acid hydrochloric acid | 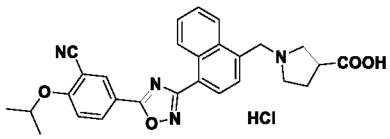 |
| A3 | ((4-(5-(3-cyano-4-isopropoxyphenyl)-1,2,4-oxadiazol-3-yl) naphthalen-1-yl) methyl) proline hydrochloric acid | 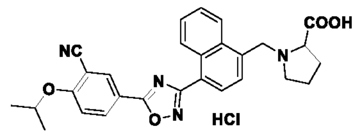 |
| A4 | (cis)-3-(((4-(5-(3-cyano-4-isopropyloxyphenyl)-1,2,4-oxadiazol-3-yl) naphthalen-1-yl)methyl)amino) cyclobutane-1-carboxylic acid hydrochloric acid | 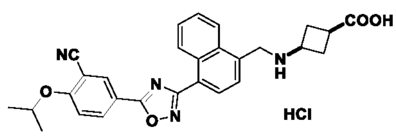 |
| A5 | N-((4-(5-(3-cyano-4-isopropoxyphenyl)-1,2,4-oxadiazol-3-yl) naphthalen-1-yl) methyl)-N-methylglycine hydrochloride | 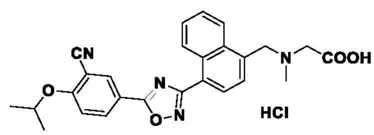 |
| A6 | ((4-(5-(3-cyano-4-isopropoxyphenyl)-1,2,4-oxadiazol-3-yl) naphthalen-1-yl) methyl) alanine hydrochloric acid | 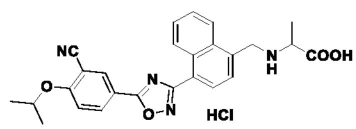 |
| A7 | ((4-(5-(3-cyano-4-isopropoxyphenyl)-1,2,4-oxadiazol-3-yl) naphthalen-1-yl) methyl) glycine hydrochloride | 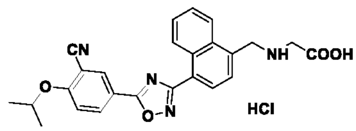 |
| A8 | 3-(((4-(5-(3-cyano-4-isopropoxyphenyl)-1,2,4-oxadiazol-3-yl) naphthalen-1-yl)methyl)amino) propionic acid hydrochloric acid | 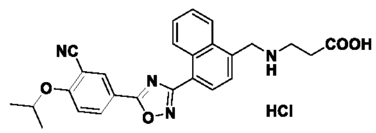 |

(continued)

| No. | Name | Structure |
|---|---|---|
| A9 | 2-(((4-(5-(3-cyano-4-isopropoxyphenyl)-1,2,4-oxadiazol-3-yl) naphthalen-1-yl)methyl)amino) malonic acid hydrochloric acid | 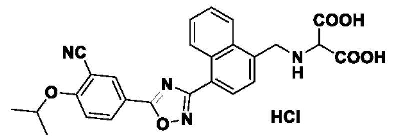 |
| A10 | 1-((4-(5-(3-chloro-4-isopropoxyphenyl)-1,2,4-oxadiazol-3-yl) naphthalen-1-yl) methyl) azetidine-3-carboxylic acid hydrochloric acid | 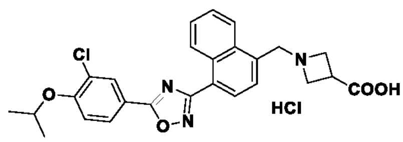 |
| A11 | 1-((4-(5-(3-bromo-4-isopropoxyphenyl)-1,2,4-oxadiazol-3-yl) naphthalen-1-yl) methyl) azetidine-3-carboxylic acid hydrochloric acid | 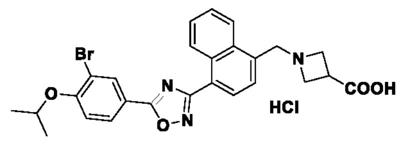 |
| A12 | 1-((4-(5-(4-isopropoxy-3-(trifluoromethyl)phenyl)-1,2,4-oxadiazol-3-yl)naphthalen-1-yl) methyl)azetidine-3-carboxylic acid hydrochloric acid | 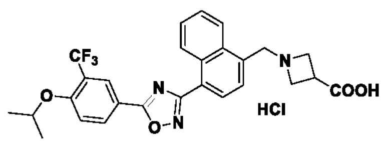 |
| A13 | 1-((4-(5-(4-isopropoxy-3-methoxyphenyl)-1,2,4-oxadiazol-3-yl) naphthalen-1-yl)methyl)azetidine-3-carboxylic acid hydrochloric acid | 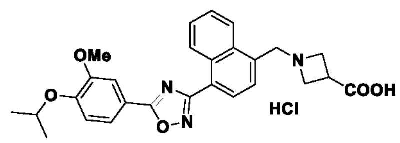 |
| A14 | 1-((4-(5-(4-isopropoxyphenyl)-1,2,4-oxadiazol-3-yl)naphthalen-1-yl) methyl)azetidine-3-carboxylic acid hydrochloric acid | 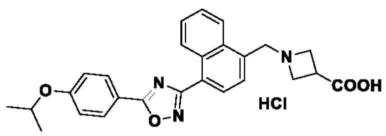 |
| A15 | 1-((4-(5-(4-(tert-butoxy)phenyl)-1,2,4-oxadiazol-3-yl) naphthalen-1-yl) methyl)azetidine-3-carboxylic acid hydrochloric acid | 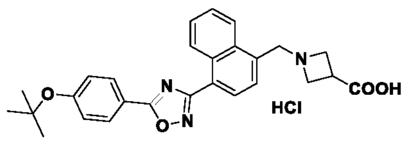 |
| A16 | 1-((4-(5-(4-methoxyphenyl)-1,2,4-oxadiazol-3-yl)naphthalen-1-yl) methyl)azetidine-3-carboxylic acid hydrochloric acid | 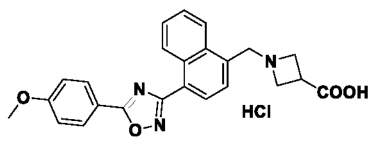 |
| A17 | 1-((4-(5-(4-propoxyphenyl)-1,2,4-oxadiazol-3-yl)naphthalen-1-yl) methyl)azetidine-3-carboxylic acid hydrochloric acid | 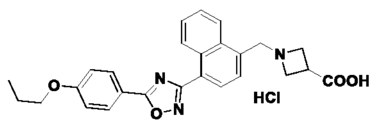 |
| A18 | 1-((4-(5-(4-(benzyloxy)phenyl)-1,2,4-oxadiazol-3-yl) naphthalen-1-yl) methyl)azetidine-3-carboxylic acid hydrochloric acid | 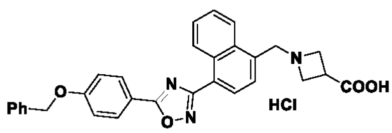 |

(continued)

| No. | Name | Structure |
| --- | --- | --- |
| A19 | 1-((4-(5-(4-(trifluoromethoxy)phenyl)-1,2,4-oxadiazol-3-yl) naphthalen-1-yl) methyl)azetidine-3-carboxylic acid hydrochloric acid | 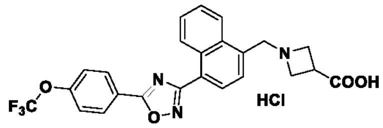 |
| A20 | 1-((4-(5-(4-isopropylphenyl)-1,2,4-oxadiazol-3-yl)naphthalen-1-yl) methyl)azetidine-3-carboxylic acid hydrochloric acid | 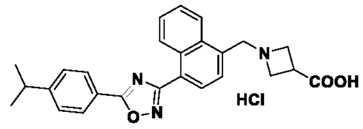 |
| A21 | 1-((4-(5-(4-propylphenyl)-1,2,4-oxadiazol-3-yl)naphthalen-1-yl) methyl)azetidine-3-carboxylic acid hydrochloric acid | 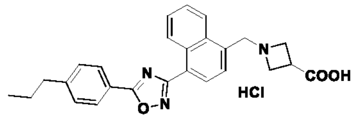 |
| A22 | 1-((4-(5-(4-cyanophenyl)-1,2,4-oxadiazol-3-yl)naphthalen-1-yl) methyl)azetidine-3-carboxylic acid hydrochloric acid | 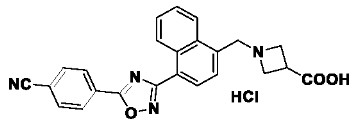 |
| A23 | 1-((4-(5-(4-fluorophenyl)-1,2,4-oxadiazol-3-yl)naphthalen-1-yl) methyl)azetidine-3-carboxylic acid hydrochloric acid | 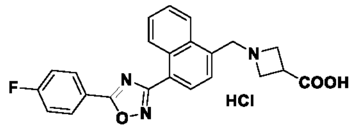 |
| A24 | 1-((4-(5-(3-cyanophenyl)-1,2,4-oxadiazol-3-yl)naphthalen-1-yl) methyl)azetidine-3-carboxylic acid hydrochloric acid | 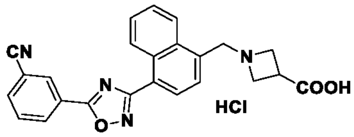 |
| A25 | 1-((4-(5-(4-fluorophenyl)-1,2,4-oxadiazol-3-yl)naphthalen-1-yl) methyl) azetidine-3-carboxylic acid hydrochloric acid | 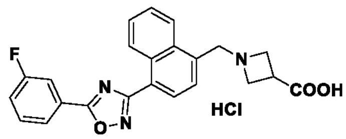 |
| A26 | 1-((4-(5-phenyl-1,2,4-oxadiazol-3-yl) naphthalen-1-yl)methyl) azetidine-3-carboxylic acid hydrochloric acid | 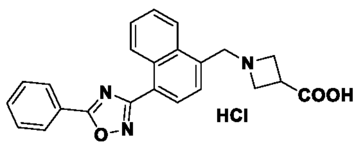 |
| A27 | 1-((4-(5-(pyridin-4-yl)-1,2,4-oxadiazol-3-yl)naphthalen-1-yl) methyl) azetidine-3-carboxylic acid hydrochloric acid | 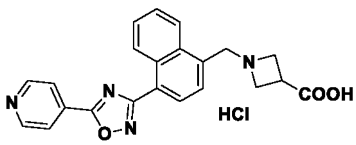 |
| A28 | 1-((4-(5-(pyridin-3-yl)-1,2,4-oxadiazol-3-yl)naphthalen-1-yl) methyl) azetidine-3-carboxylic acid hydrochloric acid | 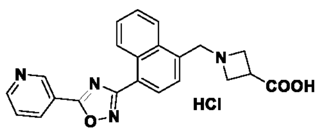 |

(continued)

| No. | Name | Structure |
|---|---|---|
| A29 | 1-((4-(5-(pyridin-2-yl)-1,2,4-oxadiazol-3-yl)naphthalen-1-yl) methyl) azetidine-3-carboxylic acid hydrochloric acid | 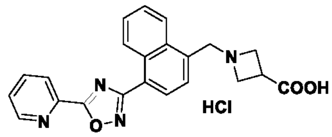 |
| A30 | 1-((4-(5-(5-methylisothiazol-3-yl)-1,2, 4-oxadiazol-3-yl) naphthalen-1-yl) methyl)azetidine-3-carboxylic acid hydrochloric acid | 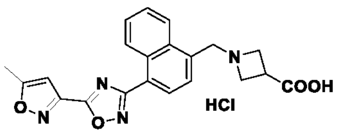 |
| A31 | 1-((4-(5-(5-(benzo[*d*][1,3]dioxazol-5-yl)-1,2,4-oxadiazol-3-yl) naphthalen-1-yl)methyl)azetidine-3-carboxylic acid hydrochloric acid | 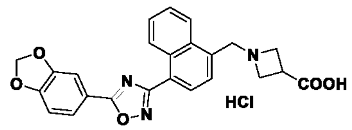 |
| A32 | 1-((4-(5-(2,3-dihydrobenzo [*b*][1,4] dioxin-6-yl)-1,2,4-oxadiazol-3-yl) naphthalen-1-yl)methyl) azetidine-3-carboxylic acid hydrochloric acid | 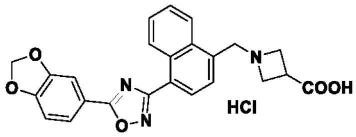 |
| A33 | 1-((4-(5-(quinolin-3-yl)-1,2,4-oxadiazol-3-yl)naphthalen-1-yl) methyl)azetidine-3-carboxylic acid hydrochloric acid | 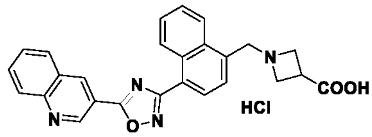 |
| A34 | 1-((4-(5-(quinoxaline-2-yl)-1,2,4-oxadiazol-3-yl)naphthalen-1-yl) methyl)azetidine-3-carboxylic acid hydrochloric acid | 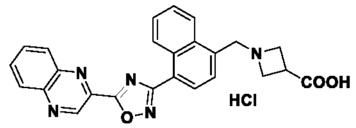 |
| A35 | 1-((4-(5-(1*H*-indole-5-yl)-1,2,4-oxadiazol-3-yl)naphthalen-1-yl) methyl) azetidine-3-carboxylic acid hydrochloric acid | 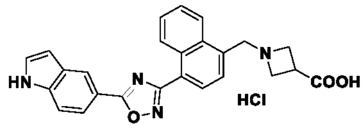 |
| A36 | 1-((4-(5-(1*H*-indole-2-yl)-1,2,4-oxadiazol-3-yl)naphthalen-1-yl) methyl)azetidine-3-carboxylic acid hydrochloric acid | 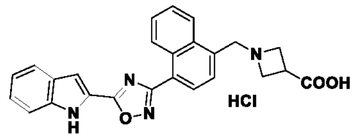 |
| A37 | 1-((4-(5-(1-methyl-1*H*-indole-2-yl)-1,2,4-oxadiazol-3-yl) naphthalen-1-yl) methyl)azetidine-3-carboxylic acid hydrochloric acid | 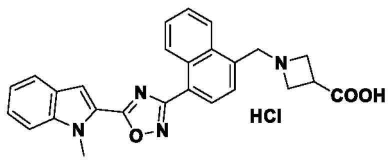 |
| A38 | (*E*)-1-((4-(5-styryl-1,2,4-oxadiazol-3-yl)naphthalen-1-yl)methyl) azetidine-3-carboxylic acid hydrochloric acid | 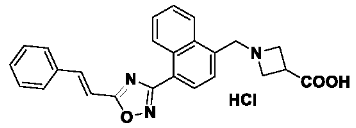 |

(continued)

| No. | Name | Structure |
|---|---|---|
| A39 | 1-((4-(5-cyclohexyl-1,2,4-oxadiazol-3-yl)naphthalen-1-yl) methyl)azetidine-3-carboxylic acid hydrochloric acid | 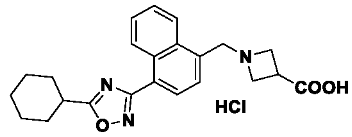 |
| A40 | 1-((4-(5-(3-cyano-4-isopropoxyphenyl)-1,2,4-oxadiazol-3-yl) naphthalen-1-yl) methyl) piperidine-4-carboxylic acid hydrochloric acid | 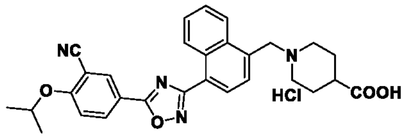 |
| A41 | 1-((4-(5-(3-cyano-4-isopropoxyphenyl)-1,2,4-oxadiazol-3-yl) naphthalen-1-yl) methyl) piperidine-3-carboxylic acid hydrochloric acid | 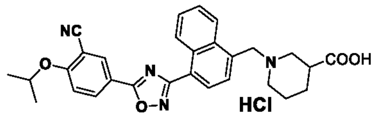 |
| A42 | 1-((4-(5-(3-cyano-4-isopropoxyphenyl)-1,2,4-oxadiazol-3-yl) naphthalen-1-yl) methyl)piperidine-3,4-carboxylic acid hydrochloric acid | 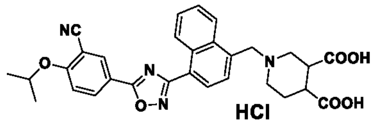 |
| A43 | 1-((4-(5-(3-cyano-4-isopropoxyphenyl)-1,2,4-oxadiazol-3-yl) naphthalen-1-yl) methyl) pyrrolidin-3-sulfonic acid | 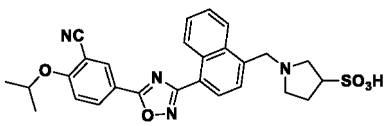 |
| A44 | Methyl 1-((4-(5-(3-cyano-4-isopropoxyphenyl)-1,2,4-oxadiazol-3-yl)naphthalen-1-yl)methyl)azelaic acid -3-carboxylate | 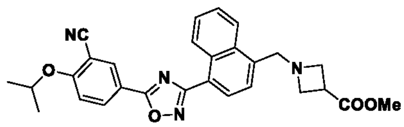 |
| A45 | Ethyl 1-((4-(5-(3-cyano-4-isopropoxyphenyl)-1,2,4-oxadiazol-3-yl) naphthalen-1-yl)methyl)azelaic acid -3-carboxylate | 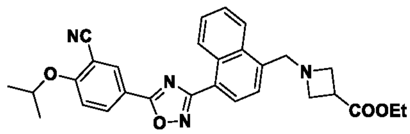 |
| A46 | Isopropyl 1-((4-(5-(3-cyano-4-isopropoxyphenyl)-1,2,4-oxadiazol-3-yl) naphthalen-1-yl)methyl)azelaic acid-3-carboxylate | 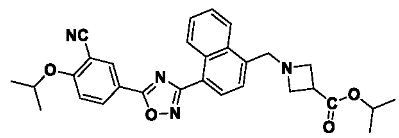 |
| A47 | 47 1-((4-(5-(3-cyano-4-isopropoxyphenyl)-1,2,4-oxadiazol-3-yl) naphthalen-1-yl)methyl)azelaic acid -3-carboxamide | 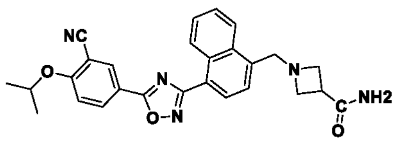 |
| A48 | 1-((4-(5-(3-cyano-4-isopropoxyphenyl)-1,2,4-oxadiazol-3-yl) naphthalen-1-yl) methyl)-N-methyl azetidine-3-formamide | 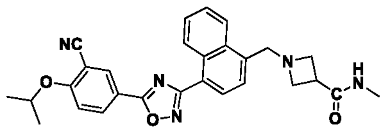 |
| A49 | 1-((4-(5-(3-cyano-4-isopropoxyphenyl)-1,2,4-oxadiazol-3-yl) naphthalen-1-yl) methyl)-N,N-dimethylazetidine-3-formamide | 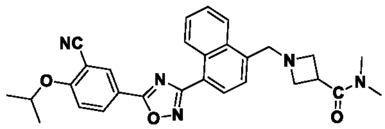 |

(continued)

| No. | Name | Structure |
|---|---|---|
| A50 | -(3-(3-(3-(1H-tetrazol-5-yl)azelaic acid -1-yl)methyl)naphthalen-1-yl)-1,2,4-oxadiazol-5-yl)-2-isopropoxybenzonitrile | 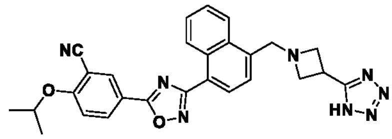 |
| A51 | 3-(4-(3-(1H-tetrazol-1-yl)azelaic acid-1-yl)methyl)naphthalen-1-yl)-5-(4-isopropoxy-3-(trifluoromethyl)phenyl)-1,2, 4-oxadiazole | 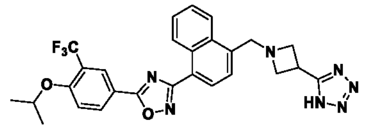 |
| A52 | 3-(4-(3-(1H-tetrazol-1-yl)azelaic acid -1-yl)methyl)naphthalen-1-yl)-5-(1H-indole -5-yl)-1,2,4-oxadiazole | 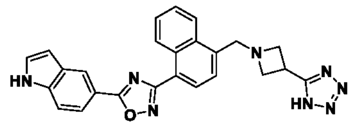 |
| A53 | 3-(4-(3-(1H-tetrazol-1-yl)azelaic acid - 1-yl)methyl)naphthalen-1-yl)-5-(1H-indole-6-yl)-1,2,4-oxadiazole | 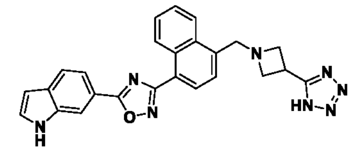 |
| A54 | 3-(4-(3-(1H-tetrazol-1-yl)azelaic acid - 1-yl)methyl)naphthalen-1-yl)-5-(1H-indole-2-yl)-1,2,4-oxadiazole | 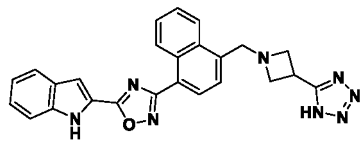 |
| A55 | Diethyl (1-(4-(5-(3-cyano-4-isopropoxyphenyl)-1,2,4-oxadiazol-3-yl) naphthalen-1-yl)methyl)azelaic acid-3-yl)phosphonate | 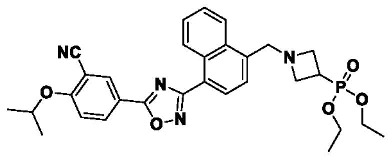 |
| A56 | 1-((5-(5-(3-cyano-4-isopropoxyphenyl)-1,2,4-oxadiazol-3-yl) isoquinoline-8-yl) methyl)azelaic acid-3-carboxylic acid hydrochloric acid | 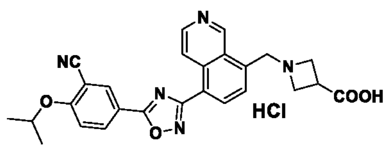 |
| A57 | 1-((5-(5-(3-cyano-4-isopropoxyphenyl)-1,2,4-oxadiazol-3-yl) isoquinoline-8-yl) methyl)azelaic acid-3-carboxylic acid hydrochloric acid | 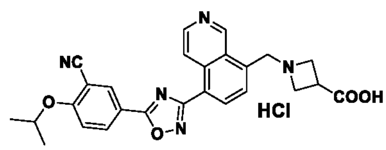 |
| A58 | 1-((8-(5-(3-cyano-4-isopropoxyphenyl)-1,2,4-oxadiazol-3-yl) quinoxaline-5-yl) methyl)azelaic acid-3-carboxylic acid hydrochloric acid | 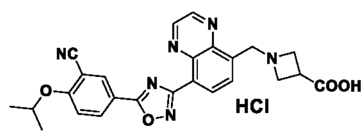 |
| A59 | 1-((4-(5-(thiophen-2-yl)-1,2, 4-oxadiazol-3-yl)naphthalen-1-yl) methyl)azelaic acid-3-carboxylic acid hydrochloric acid | 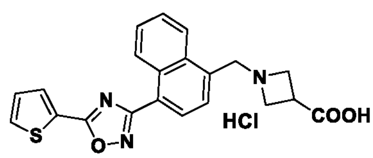 |

(continued)

| No. | Name | Structure |
|---|---|---|
| A60 | 1-((4-(5-(4-methylthiophen-2-yl)-1,2,4-oxadiazol-3-yl) naphthalen-1-yl) methyl)azelaic acid-3-carboxylic acid hydrochloric acid | 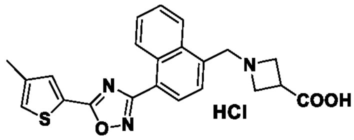 |
| A61 | 1-((4-(5-(5-methoxy-4-methylthiophen-2-yl)-1,2,4-oxadiazol-3-yl)naphthalen-1-yl)methyl)azelaic acid-3-carboxylic acid hydrochloric acid | 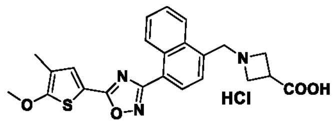 |
| A62 | 1-((4-(5-(furan-2-yl)-1,2,4-oxadiazol-3-yl) naphthalen-1-yl) methyl)azelaic acid -3-carboxylic acid hydrochloric acid | 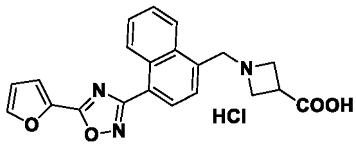 |
| A63 | 1-((4-(5-(4-(trifluoromethyl)furan-2-yl)-1,2,4-oxadiazol-3-yl) naphthalen-1-yl)methyl)azelaic acid-3-carboxylic acid hydrochloric acid | 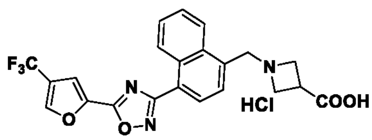 |
| A64 | 1-((4-(5-(4-cyanofuran-2-yl)-1,2,4-oxadiazol-3-yl)naphthalen-1-yl) methyl)azelaic acid-3-carboxylic acid hydrochloric acid | 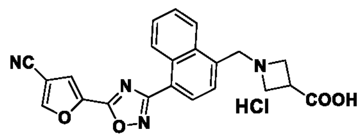 |
| A65 | 1-((4-(5-(4-isopropoxyfuran-2-yl)-1,2, 4-oxadiazol-3-yl) naphthalen-1-yl) methyl)azelaic acid-3-carboxylic acid hydrochloric acid | 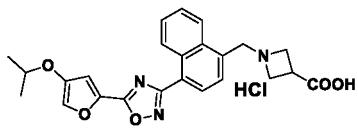 |
| A66 | 1-((4-(5-([1,1'-biphenyl]-4-yl)-1,2,4-oxadiazol-3-yl)naphthalen-1-yl) methyl)azelaic acid-3-carboxylic acid hydrochloric acid | 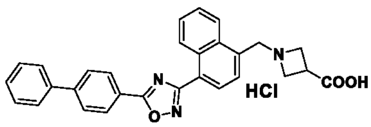 |
| A67 | 1-((4-(5-([1,1'-biphenyl]-4-yl)-1,2,4-oxadiazol-3-yl)naphthalen-1-yl) methyl) azelaic acid -3-carboxylic acid hydrochloric acid | 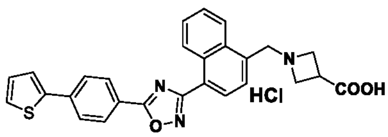 |
| A68 | 1-((4-(4-(4-(1H-pyrrol-2-yl) phenyl)-1,2,4-oxadiazol-3-yl) naphthalen-1-yl) methyl)azelaic acid-3-carboxylic acid hydrochloric acid | 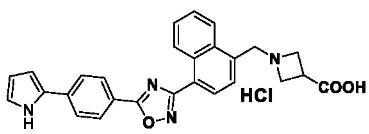 |
| A69 | 1-((4-(5-(4-(furan-2-yl) phenyl)-1,2,4-oxadiazol-3-yl)naphthalen-1-yl) methyl)azelaic acid-3-carboxylic acid hydrochloric acid | 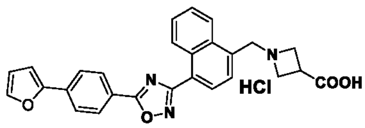 |

(continued)

| No. | Name | Structure |
|---|---|---|
| A70 | 1-((4-(5-(3-Cyano-4-(cyclopentyloxy) phenyl)-1,2,4-oxadiazol-3-yl) naphthalen-1-yl)methyl)azelaic acid-3-carboxylic acid hydrochloric acid | 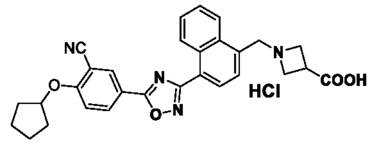 |

The compounds of the present invention have asymmetric centers, chiral axes and chiral planes, and can also be in the form of racemates, R-isomers or S-isomers.Those skilled person in the art can use conventional technical means to obtain the R-isomer and/or S-isomer from the racemate resolution.

The invention provides a pharmaceutically acceptable salt of a general formula I compound, specifically a general formula I compound reacts with an inorganic or organic acid to form a conventional pharmaceutically acceptable salt.For example, conventional medicinal salts can be prepared by reacting a general formula I compound with an inorganic acid or an organic acid, the inorganic acid including hydrochloric acid, hydrobromic acid, sulfuric acid, nitric acid, amino sulfonic acid and phosphoric acid, etc, and the organic acids include citric acid, tartaric acid, lactic acid, pyruvic acid, acetic acid, benzenesulfonic acid, p-toluenesulfonic acid, methanesulfonic acid, naphthalene sulfonic acid, ethanesulfonic acid, naphthalene disulfonic acid, malic acid, malic acid, fumaric acid, succinic acid, propionic acid, oxalic acid, trifluoroacetic acid, stearic acid, hydroxymaleic acid, phenylacetic acid, benzoic acid; or the sodium salt, potassium salt, calcium salt, aluminum salt or ammonium salt formed by the compound of general formula I and inorganic base; or the methylamine salt, ethylamine salt or ethanolamine salt formed by the compound of general formula I and organic base.

The compounds of the present invention can be used for the preparation of pharmaceutical compositions comprising a therapeutically effective amount of one or more selected from the group consisting ofoxadiazolecompounds of formula I, a pharmaceutically acceptable salt thereof, a racemate, an R-isomer, an S-isomers, or a mixture thereof.The pharmaceutical composition can be used to treat non-alcoholic fatty liver, liver fibrosis, diabetes, hyperlipidemia, multiple sclerosis (including relapsing multiple sclerosis, Relapsing-remitting multiple sclerosis, active secondary progressive multiple sclerosis, psoriasis, ulcerative colitis, lupus erythematosus, Crohn's disease, immune disorders, wet age-related macular degeneration, atopic dermatitis, inflammatory bowel disease, clinical isolated syndromeand other diseases.

The compounds of the present invention can also be used for the preparation of S1P receptor agonists comprising one or more selected from the group consisting ofoxadiazolecompounds represented by Formula I above, a pharmaceutically acceptable salt thereof, a racemate, an R-isomer, an S-isomers, or a mixture thereof.

**Preparation of Oxadiazole Compounds**

Another aspect of the present invention provides a process for the preparation of a compound represented by the general formula I, which is carried out as follows.

The compound of formula (I) can be prepared by the method shown in the following scheme

## Scheme:

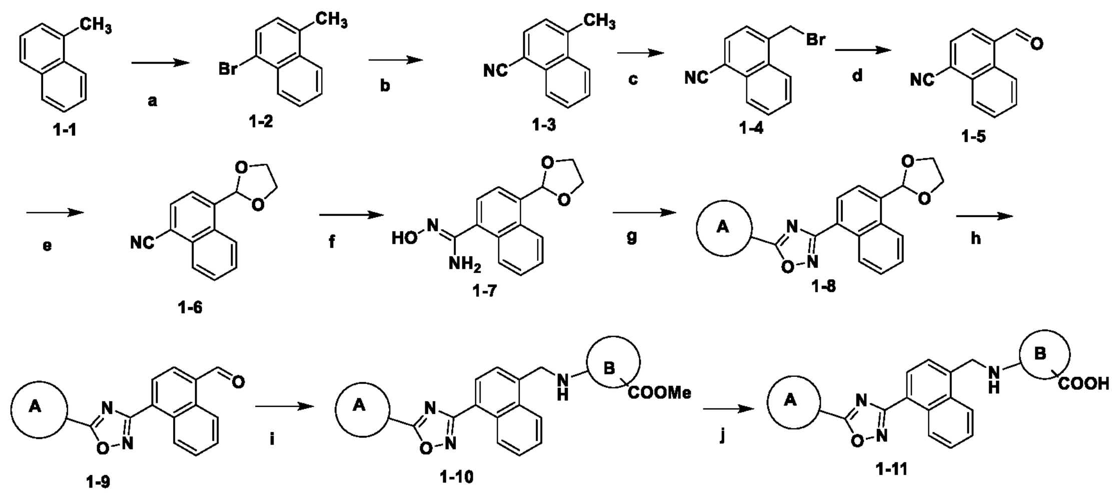


1-1 1-2 1-3 1-4 1-5 1-6 1-7 1-8 1-9 1-10 1-11

**Step a**:Dissolve 1-1(1eq) in an appropriate amount of acetonitrile, add NBS(1.1eq) in batches, and stir at 40 °C for 4 hours;

**Step B**:Dissolve 1-2(1eq)1-2in an appropriate amount of dimethylformamide, add cuprous cyanide (3eq), and stir at 130 °C for reaction overnight;

**Step c**:Dissolve 1-3(1eq) in an appropriate amount of carbon tetrachloride, add NBS(1.2eq) and BPO(0.1eq), and heat and reflow overnight;

**Step d**:Dissolve 1-4(1eq)in an appropriate amount of 50% acetic acid aqueous solution, add hexamethylenetetramine (2eq) in batches under stirring, heat reflux for 6 hours, add 3 mL concentrated hydrochloric acid dropwise, and continue to heat reflux for half an hour;

**Step e**:Dissolve the intermediate 1-5(1eq)in an appropriate amount of toluene, sequentially add ethylene glycol (3eq) and p-toluenesulfonic acid (0.1eq), install the water separator, and heat and reflow for 4 hours;

**Step f**:Dissolve the intermediate 1-6(1eq)4-(1,3-dioxolan-2-yl)-1-naphthenonitrile, hydroxylamine hydrochloride (1.05eq) and triethylamine (1.05eq) in an appropriate amount of ethanol, and heat and reflux overnight;

**Step g**:The intermediate 1-7(1eq) was dissolved in an appropriate amount of DMF, benzoic acid (1.1eq), HOBT(1eq), EDCI(1eq) and potassium carbonate (1.5eq) were sequentially added, and the reaction was heated at 90 °C overnight;

**Step h**:dissolve the intermediate 1-8 in an appropriate amount of acetone/dilute hydrochloric acid (1:1), and heat and stir at 45 °C for 4 hours;

**Step I**:Dissolve the intermediate 1-9 (1eq)in an appropriate amount of methanol/dichloromethane (1:1) mixed solution, add 1.1eq of the corresponding amine hydrochloride, DIPEA(1.1eq), acetic acid (2eq) and sodium cyanoborohydride (1eq) in sequence, and stir overnight at room temperature;

**Step k**:Dissolve the intermediate 1-10(1eq) in an appropriate amount of methanol/water mixed solution, add lithium hydroxide (10eq), and stir at room temperature overnight.

**Pharmaceutical composition and preparation thereof**

Another aspect of the present invention provides a pharmaceutical composition comprising a therapeutically effective amount of one or more of the compounds of formula I, pharmaceutically acceptable salts, enantiomers, diastereomers or racemates thereof, and optionally, one or more pharmaceutically acceptable carriers, excipients, adjuvants, adjuvants and/or diluents thereof.The auxiliary materials are, for example, odorants, flavors, sweeteners, etc.

The pharmaceutical composition provided by the present invention preferably contains an active ingredient in a weight ratio of 1 to 99%, and the preferred proportion is that the compound of formula I as the active ingredient accounts for 65wt% to 99wt% of the total weight, and the rest is a pharmaceutically acceptable carrier, diluent or solution or salt solution.

The compounds and pharmaceutical compositions provided by the present invention may be in various forms, such as tablets, capsules, powders, syrups, solutions, suspensions, aerosols, and the like, and may be present in a suitable solid or liquid carrier or diluent and in a suitable sterilizing appliance for injection or instillation.

Various dosage forms of the pharmaceutical composition of the present invention can be prepared according to the conventional preparation methods in the field of pharmacy.The unit measure of its formulation formulation contains 1 mg-700 mg of the compound of formula I, preferably 25 mg-300 mg of the compound of formula I.

The compounds and pharmaceutical compositions of the present invention can be used clinically in mammals,

including humans and animals, and can be administered through oral, nasal, skin, lung, gastrointestinal tract, etc.The best choice is oral.The most preferred daily dose is 50-1400 mg/kg body weight, taken once, or 25-700 mg/kg body weight in divided doses.Regardless of the method of administration, the optimal dose for the individual should be based on the specific treatment.Usually it starts with a small dose and gradually increases the dose until the most suitable dose is found.

The present invention also provides a S1P agonist comprising one or more selected from the group consisting of the compounds of Formula I above, pharmaceutically acceptable salts, racemates, R-isomers, S-isomers or mixtures thereof, and optionally one or more pharmaceutically acceptable carriers, excipients, adjuvants, excipients and/or diluents.

The compounds and compositions of the present invention are used for the treatment and prevention of non-alcoholic fatty liver, liver fibrosis, diabetes, hyperlipidemia, multiple sclerosis (including relapsing multiple sclerosis, Relapsing-remitting multiple sclerosis, active secondary progressive multiple sclerosis), psoriasis, ulcerative colitis, lupus erythematosus, Crohn's disease, immune disorders, wet age-related macular degeneration, atopic dermatitis, inflammatory bowel disease, Clinical isolation syndrome and other diseases, the diseases include, but are not limited to, various types of diabetes, hyperlipidemia, non-alcoholic fatty liver, liver fibrosis, multiple sclerosis and the like.

Therefore, another aspect of the present invention provides the compounds of general formula I, pharmaceutically acceptable salts, racemates, R-isomers, S-isomers or mixtures thereof for the treatment of non-alcoholic fatty liver, liver fibrosis, diabetes, hyperlipidemia, multiple sclerosis (including relapsing multiple sclerosis, relapsing-remitting multiple sclerosis, active secondary progressive multiple sclerosis) associated with S1P agonists, psoriasis, ulcerative colitis, lupus erythematosus, Crohn's disease, immune disorders, wet age-related macular degeneration, atopic dermatitis, inflammatory bowel disease, clinical isolated syndrome and other diseases, for example, the use of drugs for various types of diabetes, hyperlipidemia, non-alcoholic fatty liver, liver fibrosis, multiple sclerosis and other diseases.

Another aspect of the present invention provides a treatment for non-alcoholic fatty liver, liver fibrosis, diabetes, hyperlipidemia, multiple sclerosis (including relapsing multiple sclerosis, relapsing-remitting multiple sclerosis, Active secondary progressive multiple sclerosis), psoriasis, ulcerative colitis, lupus erythematosus, Crohn's disease, immune disorders, wet age-related macular degeneration, atopic dermatitis, inflammatory bowel disease, clinical isolated syndrome and other diseases-related metabolic diseases and autoimmune diseases, for example, a method of various types of diabetes, hyperlipidemia, non-alcoholic fatty liver disease, liver fibrosis, multiple sclerosis, and the like, comprising administering to a patient in need of such treatment one or more selected from the group consisting of a compound of Formula I, pharmaceutically acceptable salts thereof, racemates, R-isomers, S-isomers, or mixtures thereof.

The present invention is further described below in conjunction with specific embodiments.It is to be understood that these examples are intended to illustrate the invention only and not to limit the scope of the invention.The following embodiments do not indicate the specific conditions of the experimental method, usually according to the conventional conditions, or according to the conditions recommended by the manufacturer.Percentages and servings are calculated by weight unless otherwise stated.

**Example 1 Synthesis of 1-((4-(5-(3-cyano-4-isopropoxyphenyl)-1,2,4-oxadiazol-3-yl)naphthalen-1-yl)methyl)azetidine-3-carboxylic acid hydrochloric acid (A1)**

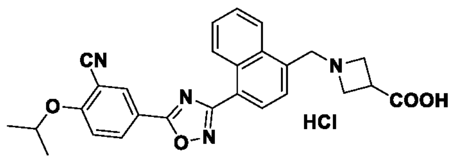

**Synthesis of 1-bromo-4-methylnaphthalene**

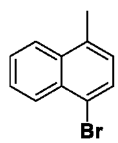

1-methyl naphthalene (28.44g, 200.00 mmol) was dissolved in 300 mL acetonitrile, NBS(39.16g, 220.00 mmol) was added in batches, and stirred at 40 °C for 4 hours. After the reaction was completed, concentrated under reduced pressure, and separated by column chromatography to obtain the target compound (43.20g), yield 98%.$^1$H NMR (500 MHz, $CDCl_3$) δ 8.24 - 8.17 (m, 1H), 7.94 - 7.86 (m, 1H), 7.63 (d, *J* =8.4 Hz, 1H), 7.57 - 7.47 (m, 2H), 7.20 - 7.14 (m, 1H), 2.70 (d, *J* =1.1 Hz, 3H).

**Synthesis of 4-methyl-1-naphthonitrile**

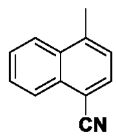

The intermediate1-bromo-4-methylnaphthalene(44.00g, 199.01 mmol) was dissolved in 400 mL dimethylformamide, cuprous cyanide (53.47g, 597.02 mmol) was added, and stirred at 130 °C to react overnight.After the reaction was completed as shown by TLC, the mixture was cooled to room temperature, 300 mL dichloromethane was added, and stirred at room temperature for 1 hour, filtered under reduced pressure.The filtrate was washed with water for three times, dried over anhydrous sodium sulfate, concentrated under reduced pressure, and separated by column chromatography to obtain the target compound (18.21g), yield 55%.$^1$H NMR (500 MHz, $CDCl_3$) δ 8.21 - 8.15 (m, 1H), 7.98 - 7.89 (m, 1H), 7.78 (d, *J* =8.4 Hz, 1H), 7.64 - 7.54 (m, 2H), 7.43 (dq, *J* = 8.3, 1.1 Hz, 1H), 2.69 (d, *J* =1.1 Hz, 3H).

**Synthesis of 4-(bromomethyl)-1-naphthonitrile**

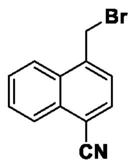

The intermediate4-methyl-1-naphthonitrile(16.00g, 95.69 mmol) was dissolved in 200 mL of carbon tetrachloride, NBS(20.44g, 114.82 mmol) and BPO(2.16g, 9.57 mmol) were added, and heated and refluxed overnight.After the reaction was completed as shown by TLC, it was cooled to room temperature, and the filtrate was filtered under reduced pressure. The filtrate was washed with saturated sodium bicarbonate solution, water and saturated brine successively, dried over anhydrous sodium sulfate, concentrated under reduced pressure, and separated by column chromatography to obtain the target compound (13.00g), yield 55%.$^1$H NMR (500 MHz, $CDCl_3$) δ 8.23 - 8.17 (m, 1H), 8.09 - 8.03 (m, 1H), 7.82 (d, J =8.4 Hz, 1H), 7.62 - 7.53 (m, 2H), 7.48 (d, J =8.4 Hz, 1H), 4.55 (s, 2H).

**Synthesis of 4-formyl-1-naphthonitrile**

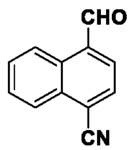

The intermediate4-(bromomethyl)-1-naphthonitrile(13.00g, 52.82 mmol) was dissolved in 50% acetic acid aqueous solution (200 mL), hexamethylenetetramine (14.81g, 105.65 mmol) was added in batches under stirring, heated and refluxed for 6 hours, then 3 mL of concentrated hydrochloric acid was added dropwise, and heated and refluxed for half an hour.After the reaction was completed, it was cooledto room temperature, concentrated under reduced pressure to remove excess acetic acid, and extracted with ethyl acetate for three times.The combined organic phase was dried

over anhydrous sodium sulfate, concentrated under reduced pressure, separated by column chromatography to obtain the target compound (5.5g), yield 57%.$^1$H NMR (500 MHz, $CDCl_3$) δ 9.99 (s, 1H), 8.28 - 8.22 (m, 1H), 8.12 (dd, J = 8.4, 1.4 Hz, 1H), 7.97 - 7.89 (m, 2H), 7.67 (td, *J* = 7.8, 1.4 Hz, 1H), 7.61 (td, *J* = 8.0, 1.3 Hz, 1H).

**Synthesis of 4-(1, 3-dioxolane-2-yl)-1-naphthonitrile**

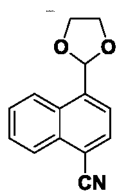

The intermediate4-formyl-1-naphthonitrile(3.00g, 16.56 mmol) was dissolved in 50 mL of toluene, 2.8 mL of ethylene glycol and p-toluenesulfonic acid (0.29g, 1.66 mmol) were added sequentially, and the water separator was installed and heated and refluxed for 4 hours.After the reaction was completed, it was cooled to room temperature, concentrated under reduced pressure, and separated by column chromatography to obtain the target compound (3.51g), yield 91%.$^1$H NMR (500 MHz, $CDCl_3$) δ 8.25 - 8.19 (m, 1H), 8.03 - 7.97 (m, 1H), 7.84 (d, *J* =8.4 Hz, 1H), 7.77 (d, *J* =8.4 Hz, 1H), 7.65 - 7.55 (m, 2H), 6.09 (s, 1H), 4.16 - 4.02 (m, 4H).

**Synthesis of (*Z*)-4-(1, 3-dioxane -2-yl)-*N'*-hydroxy-1-naphthamidine**

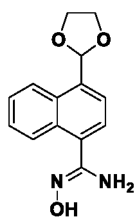

The intermediates4-(1, 3-dioxolan-2-yl)-1-naphthonitrile(3.55g, 15.76 mmol), hydroxylamine hydrochloride (1.15g, 16.55 mmol) and triethylamine (2.29 mL, 16.55 mmol) were dissolved in 40 mL of ethanol, heated and refluxed overnight, after the reaction was completed, it was cooled to room temperature, concentrated under reduced pressure, and directly used for the next reaction without purification.ESI-MS m/z:259.1 $[M+H]^+$.

**Synthesis of 5-(5-(4-(1, 3-dioxpentacycl-2-yl) naphthalen-1-yl)-1,2, 4-oxadiazol-3-yl)-2-isopropoxybenzonitrile**

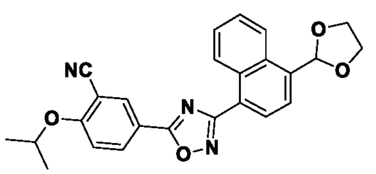

The intermediate(Z)-4-(1,3-dioxane-2-yl)-N'-hydroxy-1-naphthamidine obtained in the previous step was dissolved in 50 mL DMF, 1.1 equivalent of benzoic acid, 1 equivalent of HOBT, 1 equivalent of EDCI and 1.5 equivalent of potassium carbonate were sequentially added, and heated at 90 °C to reactovernight.After the reaction was completed, jt was cooled to room temperature, 100 mL distilled water was added and extracted with ethyl acetate.The organic phase was dried with anhydrous sodium sulfate, concentrated under reduced pressure, separated by column chromatography to obtain the target compound.$^1$H NMR (500 MHz, $CDCl_3$) δ 8.02 - 7.90 (m, 4H), 7.89 - 7.80 (m, 6H), 7.68 (dd, *J* = 8.4, 1.8 Hz, 2H), 7.52 - 7.43 (m, 5H), 7.42 (s, 1H), 5.97 (s, 2H), 4.54 (hept, *J* =6.2 Hz, 2H), 4.17 - 4.03 (m, 8H), 1.36 (d, *J*

=6.2 Hz, 11H). ESI-MS *m/z*:428.1 [M+H]+. ,

**Synthesis of 5-(5-(4-formylnaphthalen-1-yl)-1,2,4-oxadiazol-3-yl)-2-isopropoxybenzonitrile**

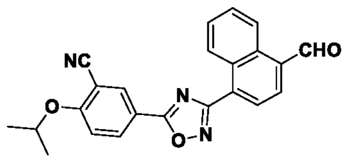

The intermediate5-(4-(4-(1, 3-dioxolane-2-yl) naphthalen-1-yl)-1,2,4-oxadiazol-3-yl)-2-isopropoxybenzonitrile(2.07g, 4.84 mmol) was dissolved in 20 mL of acetone, 20 mL of dilute hydrochloric acid was added, and heated and stirred at 45 °C for 4 hours. After the reaction was completed, 40 mL of distilled water was added, the filter residue was washed with distilled water for several times, and dried to obtain the target compound (1.77g), yield 95%.$^1$H NMR (500 MHz, $CDCl_3$) δ 8.13 (d, *J* =8.4 Hz, 1H), 8.04 - 7.94 (m, 3H), 7.88 (d, *J* =1.9 Hz, 1H), 7.69 (dd, *J* = 8.4, 2.0 Hz, 1H), 7.60 - 7.49 (m, 2H), 7.42 (d, *J* =8.4 Hz, 1H), 4.57 (hept, *J* =6.2 Hz, 1H), 1.35 (d, *J* =6.2 Hz, 6H). ESI-MS *m/z*:384.1 [M+H]+.

**Synthesis of methyl 1-((4-(3-(3-cyano-4-isopropoxyphenyl)-1,2, 4-oxadiazol-5-yl) naphthalen-1-yl) methyl) azetidine-3-carboxylate**

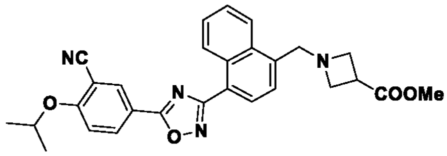

The intermediate 5-(5-(4-formylnaphthalen-1-yl)-1,2, 4-oxadiazol-3-yl)-2-isopropoxybenzonitrile(0.40g, 1.04 mmol) was dissolved in 20 mL of methanol/dichloromethane (1:1) mixed solution, 1.1 equivalent of the corresponding amine hydrochloride, 1.1 equivalent of DIPEA, 2 equivalent of acetic acid and 1 equivalent of sodium cyanoborohydride were sequentially added and stirred overnight at room temperature. After the reaction, an appropriate amount of saturated ammonium chloride solution wasadded, extracted with dichloromethane, the organic phase wasdried with anhydrous sodium sulfate, concentrated under reduced pressure, and separated by column chromatography to obtain the target compound.$^1$H NMR (500 MHz, $CDCl_3$) δ 7.96 - 7.85 (m, 3H), 7.73 - 7.64 (m, 2H), 7.51 (d, *J* =8.4 Hz, 1H), 7.48 - 7.40 (m, 3H), 4.56 (hept, *J* =6.2 Hz, 1H), 3.75 (s, 2H), 3.66 (s, 3H), 3.49 (dd, *J* = 9.0, 5.8 Hz, 2H), 3.19 (dd, *J* = 9.0, 5.8 Hz, 2H), 3.03 (p, *J* =5.8 Hz, 1H), 1.35 (d, *J* =6.2 Hz, 6H). ESI-MS *m/z*:483.1 [M+H]+.

**Synthesis of 1-((4-(5-(3-cyano-4-isopropoxyphenyl)-1,2, 4-oxadiazol-3-yl) naphthalen-1-yl) methyl) azetidine-3-carboxylic acid hydrochloric acid**

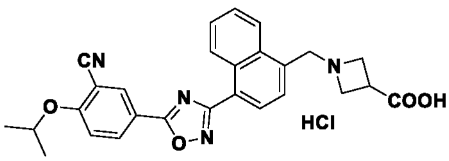

The intermediate methyl 1-(4-(3-(3-cyano -4-isopropoxyphenyl)-1,2, 4-oxadiazol-5-yl) naphthalen-1-yl) methyl) azetidine-3-carboxylate (200 mg, 0.41 mmol) was dissolved in 10 mL of methanol/water mixed solution, and lithium hydroxide (99 mg, 4.14 mmol)was add, stirred overnight at room temperature, concentrated under reduced pressure, 10 mL of distilled water was added, the pH was adjustedto 3 with 1 M hydrochloric acid, white solids were precipitated, and filtered., the filter residue was washed with distilled water several times, and dried to obtain the target compound

A1(152 mg), yield 73%.1H NMR (400 MHz, MeOD) δ 9.11 - 8.99 (m, 1H), 8.57 - 8.47 (m, 2H), 8.44 - 8.30 (m, 2H), 7.90 - 7.76 (m, 3H), 7.50 (d, J = 9.0 Hz, 1H), 5.10 (s, 2H), 5.04 - 4.96 (m, 1H), 4.53 - 4.42 (m, 4H), 3.82 - 3.73 (m, 1H), 1.52 - 1.45 (m, 6H). 13C NMR (125 MHz, DMSO-d6) δ 173.2, 168.4, 162.6, 134.7, 133.9, 131.4, 130.2, 128.7, 128.1, 127.5, 126.3, 124.6, 115.9, 115.3, 115.0, 102.5, 72.6, 55.4, 54.0, 32.3, 21.5. ESI-MS m/z:469.1 [M+H]+.

**Example 2 Synthesis of 1-(4-(5-(3-cyano-4-isopropoxyphenyl)-1,2, 4-oxadiazol-3-yl) naphthalen-1-yl) methyl) pyrrolidine -3-carboxylic acid hydrochloric acid (A2)**

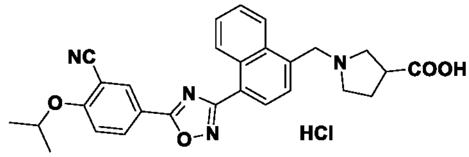

The synthesis method of compound A**2** was similar to that of compound A**1**, with a yield of 77%.$^1$H NMR (400 MHz, MeOD) δ 8.07 (dd, *J* = 8.4, 2.0 Hz, 1H), 7.97 - 7.88 (m, 2H), 7.87 - 7.81 (m, 1H), 7.70 (d, *J* =8.4 Hz, 1H), 7.59 (d, *J* =8.4 Hz, 1H), 7.50 - 7.39 (m, 2H), 7.32 (d, *J* =8.4 Hz, 1H), 4.60 - 4.50 (m, 1H), 4.00 (s, 2H), 3.45 (dd, *J* = 10.2, 5.8 Hz, 1H), 3.04 - 2.92 (m, 2H), 2.86 - 2.77 (m, 1H), 2.57 - 2.48 (m, 1H), 2.09 - 1.99 (m, 1H), 1.90 - 1.80 (m, 1H), 1.35 (d, *J* =6.2 Hz, 6H). ESI-MS *m/z*:483.1 $[M+H]^+$.

**Example 3Synthesis of ((4-(5-(3-cyano-4-isopropyloxyphenyl)-1,2, 4-oxadiazol-3-yl) naphthalen-1-yl) methyl) proline hydrochloric acid) (A3)**

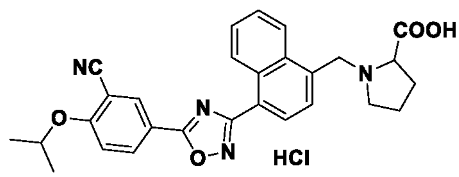

The synthesis method of compound A**3** was similar to that of compound A**1**, with a yield of 65%.$^1$H NMR (400 MHz, MeOD) δ 9.03 (d, *J* =8.4 Hz, 1H), 8.67 (d, *J* =8.4 Hz, 1H), 8.52 - 8.44 (m, 2H), 8.37 (d, *J* =7.5 Hz, 1H), 7.91 (d, *J* =7.5 Hz, 1H), 7.87 - 7.72 (m, 2H), 7.47 (d, *J* =8.9 Hz, 1H), 5.34 (d, *J* =13.2 Hz, 1H), 5.05 - 4.96 (m, 1H), 4.65 (d, *J* =13.2 Hz, 1H), 4.25 - 4.16 (m, 1H), 3.48 - 3.35 (m, 2H), 2.68 - 2.48 (m, 1H), 2.25 - 2.08 (m, 2H), 2.06 - 1.88 (m, 1H), 1.49 (d, *J* =6.0 Hz, 6H). $^{13}$C NMR (125 MHz, DMSO-*d6*) δ 173.1, 168.6, 162.6, 134.7, 133.9, 132.3, 130.2, 128.8, 127.6, 126.7, 126.0, 125.6, 116.0, 115.3, 114.9, 102.5, 72.6, 65.9, 56.0, 53.2, 28.5, 22.4, 21.5. ESI-MS *m/z*:483.1 $[M+H]^+$.

**Example 4 Synthesis of (cis)-3-(((4-(5-(3-cyano-4-isoproxyphenyl)-1,2, 4-oxadiazol-3-yl) naphthalen-1-yl) methyl) amino) cyclobutane -1-carboxylic acid hydrochloric acid (A4)**

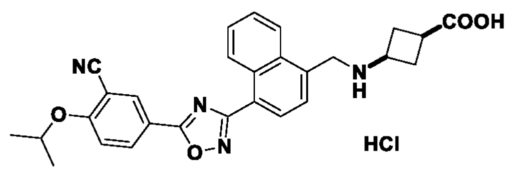

The synthesis method of compound A**4**was similar to that of compound A**1**, with a yield of 71%.$^1$H NMR (400 MHz, MeOD) δ 9.06 (d, *J* =8.2 Hz, 1H), 8.58 - 8.47 (m, 2H), 8.40 (d, *J* =7.4 Hz, 1H), 8.34 - 8.26 (m, 1H), 7.90 - 7.76 (m, 3H), 7.49 (d, *J* =8.9 Hz, 1H), 5.02 - 4.97 (m, 1H), 4.76 (s, 2H), 4.02 - 3.88 (m, 1H), 3.11 - 2.97 (m, 1H), 2.78 - 2.62 (m, 2H), 2.52 - 2.38 (m, 2H), 1.53 - 1.45 (m, 6H). $^{13}$C NMR (125 MHz, DMSO-*d6*) δ 174.9, 173.2, 168.5, 162.6, 134.7, 134.0,

131.5, 130.1, 128.7, 127.9, 127.4, 127.2, 126.3, 124.7, 123.9, 116.0, 115.3, 114.9, 102.5, 72.6, 47.8, 45.5, 30.7, 30.5, 21.5. ESI-MS **m/z:**483.1 $[M+H]^+$.

**Example 5 Synthesis of *N*-((4-(5-(3-cyano-4-isopropyloxyphenyl)-1,2,4-oxadiazol-3-yl) naphthalen-1-yl) methyl)-N-methylglycine hydrochloric acid (A5)**

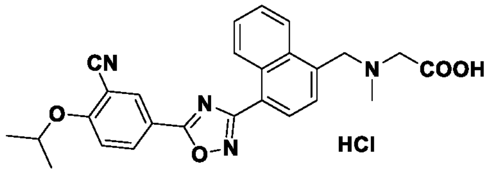

The synthesis method of compound A**5**was similar to that of compound A**1**, with a yield of 63%.$^1$H NMR (400 MHz, MeOD) δ 9.03 (d, *J* =8.4 Hz, 1H), 8.77 (d, *J* =8.3 Hz, 1H), 8.56 - 8.45 (m, 2H), 8.39 (d, *J* =7.5 Hz, 1H), 7.91 (d, *J* =7.5 Hz, 1H), 7.87 - 7.74 (m, 2H), 7.48 (d, *J* =9.0 Hz, 1H), 5.03 - 4.96 (m, 3H), 3.88 (s, 2H), 2.80 (s, 3H), 1.49 (d, *J* =6.0 Hz, 6H). $^{13}$C NMR (150 MHz, DMSO-*d6*) δ 173.5, 169.2, 163.0, 135.2, 134.4, 132.8, 130.7, 129.2, 128.0, 127.5, 126.9, 126.4, 126.2, 123.5, 116.5, 115.8, 115.4, 103.0, 73.0, 58.7, 57.9, 42.1, 22.0. ESI-MS *m*/*z*:457.1 $[M+H]^+$.

**Example 6 Synthesis of ((4-(5-(3-cyano-4-isopropionoxyphenyl)-1,2, 4-oxadiazol-3-yl) naphthalen-1-yl) methyl) alanine hydrochloric acid (A6)**

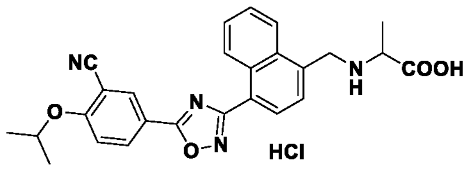

The synthesis method of compound A**6**was similar to that of compound A**1**, with a yield of 70%.$^1$H NMR (400 MHz, MeOD) δ 8.08 (dd, *J* = 8.4, 1.8 Hz, 1H), 8.04 - 7.93 (m, 2H), 7.90 (d, *J* =1.9 Hz, 1H), 7.70 (d, *J* =8.4 Hz, 1H), 7.56 (d, *J* =8.4 Hz, 1H), 7.52 - 7.46 (m, 1H), 7.46 - 7.39 (m, 1H), 7.32 (d, *J* =8.4 Hz, 1H), 4.59 - 4.50 (m, 1H), 4.21 (d, *J* =5.4 Hz, 2H), 3.66 - 3.54 (m, 2H), 1.37 (d, *J* =6.2 Hz, 3H), 1.35 - 1.27 (m, 6H). ESI-MS *m*/*z*:457.1 $[M+H]^+$.

**Example 7 Synthesis of (4-(5-(3-cyano-4-isopropoxyphenyl)-1,2, 4-oxadiazol-3-yl) naphthalen-1-yl) methyl) glycine hydrochloric acid (A7)**

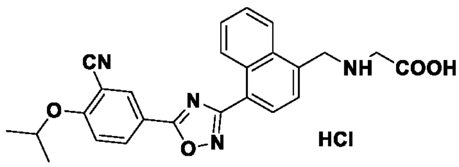

The synthesis method of compound A**7**was similar to that of compound**A1**, with a yield of 79%.$^1$H NMR (400 MHz, MeOD) δ 9.05 (d, *J* =8.1 Hz, 1H), 8.56 (d, *J* =2.3 Hz, 1H), 8.54 - 8.45 (m, 2H), 8.41 (d, *J* =7.4 Hz, 1H), 7.88 (d, *J* =7.6 Hz, 1H), 7.86 - 7.78 (m, 2H), 7.50 (d, *J* =9.0 Hz, 1H), 4.98 (d, *J* =6.9 Hz, 1H), 4.89 (s, 3H), 3.98 (s, 2H), 1.49 (d, *J* =6.0 Hz, 6H). ESI-MS m/z:443.1 $[M+H]^+$.

**Example 8 Synthesis of 1-(((4-(5-(3-cyano-4-isopropoxyphenyl)-1,2, 4-oxadiazol-3-yl) naphthalen-1-yl) methyl) amino) propionic acid hydrochloric acid (A8)**

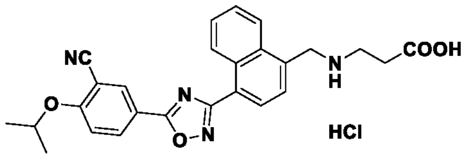

The synthesis method of compound A**8**was similar to that of compound A**1**, with a yield of 66%.$^1$H NMR (400 MHz, MeOD) δ 9.07 - 9.01 (m, 1H), 8.53 - 8.45 (m, 2H), 8.38 (d, *J* =7.5 Hz, 1H), 8.34 - 8.30 (m, 1H), 7.86 (d, *J* =7.5 Hz, 1H), 7.84 - 7.75 (m, 2H), 7.48 (d, *J* =9.0 Hz, 1H), 5.02 - 4.96 (m, 1H), 4.86 (s, 2H), 3.45 (t, *J* =6.5 Hz, 2H), 2.73 (t, *J* =6.5 Hz, 2H), 1.49 (d, *J* =6.0 Hz, 6H). $^{13}$C NMR (125 MHz, DMSO-*d6*) δ 172.5, 172.1, 168.0, 162.0, 134.1, 133.4, 131.0, 129.6, 128.2, 127.2, 126.5, 126.1, 125.7, 124.1, 122.9, 115.4, 114.7, 114.4, 102.0, 72.0, 47.9, 43.3, 31.5, 21.0. ESI-MS **m/z**:457.1 [M+H]$^+$.

**Example 9 Synthesis of 2-(((4-(5-(3-cyano-4-isopropoxyphenyl)-1,2, 4-oxadiazol-3-yl) naphthalen-1-yl) methyl) amino) malic acid hydrochloric acid (A9)**

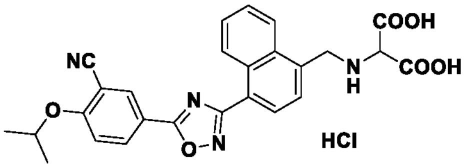

The synthesis method of compound A**9**was similar to that of compound A**1**, with a yield of 58%.$^1$H NMR (400 MHz, MeOD) δ 9.04 (d, *J* =8.4 Hz, 1H), 8.58 - 8.49 (m, 3H), 8.40 (d, *J* =7.4 Hz, 1H), 7.87 (d, *J* =7.5 Hz, 1H), 7.85 - 7.75 (m, 2H), 7.50 (d, *J* =9.0 Hz, 1H), 5.06 - 4.96 (m, 1H), 4.87 (s, 2H), 3.71 (s, 1H), 1.49 (d, *J* =6.0 Hz, 6H). ESI-MS *m/z*:487.1 [M+H]$^+$.

**Example 10 Synthesis of 1-((4-(5-(3-chloro-4-isopropyloxyphenyl)-1,2, 4-oxadiazol-3-yl) naphthalen-1-yl) methyl) azetidine-3-carboxylic acid hydrochloric acid (A10)**

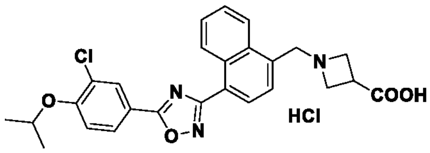

The synthesis method of compound **A10**was similar to that of compound**A1**, with a yield of 65%.$^1$H NMR (400 MHz, MeOD) δ 9.08 - 8.99 (m, 1H), 8.40 - 8.32 (m, 2H), 8.28 (d, *J* =2.1 Hz, 1H), 8.22 - 8.15 (m, 1H), 7.87 - 7.76 (m, 3H), 7.36 (d, *J* =8.8 Hz, 1H), 5.01 (s, 2H), 4.90 - 4.82 (m, 1H), 4.41 - 4.27 (m, 4H), 3.57 (p, *J* =8.4 Hz, 1H), 1.45 (d, *J* =6.0 Hz, 6H). $^{13}$C NMR (125 MHz, DMSO-*d6*) δ 173.7, 173.4, 168.6, 156.8, 131.7, 130.2, 129.5, 128.9, 128.7, 127.6, 126.7, 126.1, 124.9, 123.0, 116.1, 115.4, 71.8, 56.5, 33.3, 21.6. ESI-MS *m/z*:478.1 [M+H]$^+$.

**Example 11 Synthesis of 1-((4-(5-(3-bromo-4-isopropoxyphenyl)-1,2, 4-oxadiazol-3-yl) naphthalen-1-yl) methyl) azetidine-3-carboxylic acid hydrochloric acid (A11)**

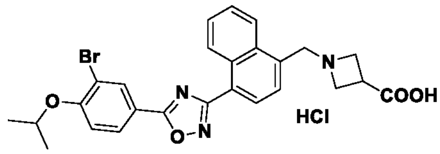

The synthesis method of compound **A11** was similar to that of compound **A1**, with a yield of 72%. $^1$H NMR (400 MHz, MeOD) δ 9.06 - 9.01 (m, 1H), 8.44 (d, *J* =2.1 Hz, 1H), 8.36 (dd, *J* = 10.5, 7.6 Hz, 2H), 8.22 (dd, *J* = 8.7, 2.1 Hz, 1H), 7.87 - 7.77 (m, 3H), 7.31 (d, *J* =8.7 Hz, 1H), 5.05 (s, 2H), 4.90 - 4.80 (m, 1H), 4.49 - 4.33 (m, 4H), 3.74 - 3.62 (m, 1H), 1.45 (d, *J* =6.0 Hz, 6H). $^{13}$C NMR (150 MHz, DMSO-*d6*) δ 173.9, 168.9, 158.3, 133.1, 132.0, 130.7, 129.8, 129.3, 128.4, 127.7, 126.7, 125.2, 117.0, 115.6, 113.1, 72.3, 56.4, 33.2, 22.1. ESI-MS *m*/*z*:522.1 [M+H]$^+$.

**Example 12 Synthesis of 1-(4-(5-(4-isopropoxy-3-(trifluoromethyl) phenyl)-1,2, 4-oxadiazol-3-yl) naphthalen-1-yl) methyl) azetidine-3-carboxylic acid hydrochloric acid (A12)**

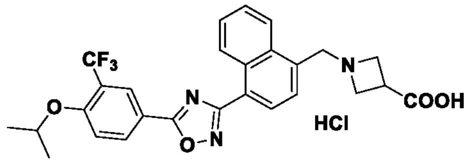

The synthesis method of compound **A12** was similar to that of compound **A1**, with a yield of 81%. $^1$H NMR (400 MHz, MeOD) δ 9.05 (dd, *J* = 7.6, 2.0 Hz, 1H), 8.47 (d, *J* =7.4 Hz, 2H), 8.38 (dd, *J* = 14.9, 8.1 Hz, 2H), 7.89 - 7.77 (m, 3H), 7.49 (d, *J* =9.0 Hz, 1H), 5.06 (s, 2H), 5.00 - 4.95 (m, 1H), 4.48 - 4.35 (m, 4H), 3.79 - 3.61 (m, 1H), 1.45 (d, *J* =6.0 Hz, 6H). $^{13}$C NMR (150 MHz, DMSO-*d6*) δ 174.0, 168.9, 159.7, 134.6, 132.0, 130.7, 129.3, 128.4, 127.7, 127.4, 126.7, 125.2, 124.4, 122.6, 119.2, 119.0, 116.2, 115.5, 72.5, 56.4, 33.1, 22.0. ESI-MS *m*/*z*:512.1 [M+H]$^+$.

**Example 13 Synthesis of 1-(4-(5-(4-isopropoxy-3-methoxyphenyl)-1,2, 4-oxadiazol-3-yl) naphthalen-1-yl) methyl) azetidine-3-carboxylic acid hydrochloric acid (A13)**

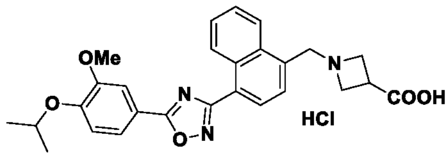

The synthesis method of compound **A13** was similar to that of compound **A1**, with a yield of 64%. $^1$H NMR (400 MHz, MeOD) δ 9.06 - 8.99 (m, 1H), 8.38 (d, *J* =7.8 Hz, 2H), 7.93 - 7.75 (m, 5H), 7.22 (d, *J* =8.5 Hz, 1H), 5.12 (s, 2H), 4.83 - 4.75 (m, 1H), 4.52 - 4.42 (m, 4H), 3.99 (s, 3H), 3.87 - 3.74 (m, 1H), 1.41 (d, *J* =6.0 Hz, 6H). ESI-MS *m*/*z*:474.1 [M+H]$^+$.

**Example 14 Synthesis of 1-((4-(5-(4-isoproxyphenyl)-1,2, 4-oxadiazol-3-yl) naphthalen-1-yl) methyl) azetidine-3-carboxylic acid hydrochloric acid (A14)**

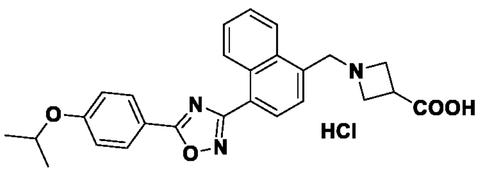

The synthesis method of compound **A14** was similar to that of compound **A1**, with a yield of 66%. $^1$H NMR (400

MHz, MeOD) δ 9.08 - 9.00 (m, 1H), 8.36 (t, *J* =7.2 Hz, 2H), 8.22 (d, *J* =8.7 Hz, 2H), 7.89 - 7.75 (m, 3H), 7.16 (d, *J* =8.8 Hz, 2H), 5.08 (s, 2H), 4.86 - 4.74 (m, 1H), 4.50 - 4.39 (m, 4H), 3.79 - 3.67 (m, 1H), 1.41 (d, *J* =6.0 Hz, 6H). $^{13}$C NMR (125 MHz, DMSO-*d6*) δ 174.1, 167.7, 161.1, 130.9, 129.7, 129.6, 128.1, 127.4, 126.7, 125.8, 124.0, 115.7, 114.6, 69.4, 55.2, 31.9, 21.1. ESI-MS *m*/*z*:444.1 [M+H]$^+$.

**Example 15 Synthesis of 1-((4-(5-(4-(tert-butoxy) phenyl)-1,2, 4-oxadiazol-3-yl) naphthalen-1-yl) methyl) azetidine-3-carboxylic acid hydrochloric acid (A15)**

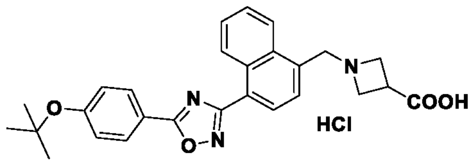

The synthesis method of compound**A15**was similar to that of compound**A1**, with a yield of 77%.$^1$H NMR (400 MHz, MeOD) δ 9.08 - 8.99 (m, 1H), 8.41 - 8.32 (m, 2H), 8.26 - 8.18 (m, 2H), 7.89 - 7.76 (m, 3H), 7.32 - 7.22 (m, 2H), 5.08 (s, 2H), 4.50 - 4.37 (m, 4H), 3.79 - 3.67 (m, 1H), 1.50 (s, 9H). $^{13}$C NMR (125 MHz, DMSO-*d6*) δ 174.6, 168.4, 160.1, 131.5, 130.3, 129.6, 128.6, 128.0, 127.3, 126.3, 124.6, 122.7, 117.1, 79.7, 55.7, 32.5, 28.5. ESI-MS *m*/*z*:458.1 [M+H]$^+$.

**Example 16 Synthesis of 1-((4-(5-(4-methoxyphenyl)-1,2, 4-oxadiazol-3-yl) naphthalen-1-yl) methyl) azetidine-3-carboxylic acid hydrochloric acid (A16)**

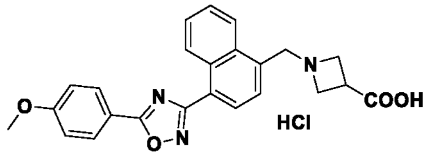

The synthesis method of compound**A16**was similar to that of compound**A1**, with a yield of 72%.$^1$H NMR (400 MHz, MeOD) δ 9.03 (d, *J* =8.3 Hz, 1H), 8.39 - 8.33 (m, 2H), 8.25 (d, *J*=8.7 Hz, 2H), 7.88 - 7.76 (m, 3H), 7.21 (d, *J* =8.7 Hz, 2H), 5.03 (s, 2H), 4.43 - 4.28 (m, 4H), 3.95 (s, 3H), 3.64 - 3.56 (m, 1H). $^{13}$C NMR (125 MHz, DMSO-*d6*) δ 174.1, 167.8, 162.7, 131.0, 129.7, 129.5, 128.1, 127.3, 126.6, 125.8, 124.1, 115.1, 114.5, 55.3, 55.2, 32.1. ESI-MS *m*/*z*:416.1 [M+H]$^+$.

**Example 17 Synthesis of 1-((4-(5-(4-propoxyphenyl)-1,2, 4-oxadiazol-3-yl) naphthalen-1-yl) methyl) azetidine-3-carboxylic acid hydrochloric acid (A17)**

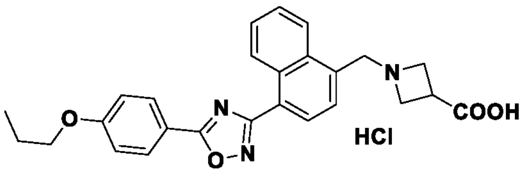

The synthesis method of compound**A17**was similar to that of compound**A1**, with a yield of 68%.$^1$H NMR (400 MHz, MeOD) δ 9.06 - 9.01 (m, 1H), 8.41 - 8.32 (m, 2H), 8.26 - 8.20 (m, 2H), 7.88 - 7.76 (m, 3H), 7.21 - 7.15 (m, 2H), 5.08 (s, 2H), 4.50 - 4.38 (m, 4H), 4.10 (t, *J* =6.4 Hz, 2H), 3.78 - 3.70 (m, 1H), 1.95 - 1.82 (m, 2H), 1.11 (t, *J* =7.4 Hz, 3H). $^{13}$C NMR (125 MHz, DMSO-*d6*) δ 175.1, 168.7, 163.2, 131.9, 130.7, 130.5, 129.0, 128.5, 127.9, 126.8, 125.4, 125.0, 115.9, 115.9, 70.0, 55.9, 32.8, 22.4, 10.8. ESI-MS **m/z**:444.1 [M+H]$^+$.

**Example 18 Synthesis of 1-((4-(5-(4-(benzyloxy) phenyl)-1,2, 4-oxadiazol-3-yl) naphthalen-1-yl) methyl) azetidine-3-carboxylic acid hydrochloric acid (A18)**

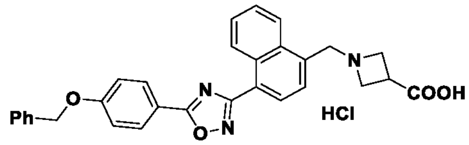

The synthesis method of compound**A18**was similar to that of compound**A1**, with a yield of 65%.$^1$H NMR (400 MHz, MeOD) δ 9.04 (d, *J* =8.2 Hz, 1H), 8.40 - 8.32 (m, 2H), 8.25 (d, *J* =8.6 Hz, 2H), 7.87 - 7.77 (m, 3H), 7.51 (d, *J* =7.5 Hz, 2H), 7.46 - 7.40 (m, 2H), 7.37 (d, *J* =7.1 Hz, 1H), 7.28 (d, *J* =8.7 Hz, 2H), 5.26 (s, 2H), 5.06 (s, 2H), 4.47 - 4.35 (m, 4H), 3.74 - 3.64 (m, 1H). $^{13}$C NMR (125 MHz, DMSO-*d6*) δ 175.1, 168.8, 162.8, 136.8, 131.9, 130.8, 130.6, 129.1, 129.0, 128.6, 128.3, 126.8, 125.0, 116.4, 116.3, 70.2, 56.3, 32.9. ESI-MS *m*/*z*:492.1 [M+H]$^+$.

**Example 19 Synthesis of 1-(4-(5-(4-(trifluoromethoxy) phenyl)-1,2, 4-oxadiazol-3-yl) naphthalen-1-yl) methyl) azetidine-3-carboxylic acid hydrochloric acid (A19)**

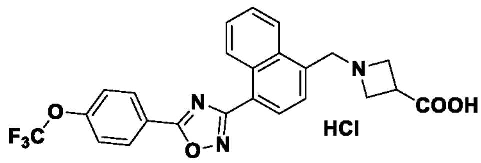

The synthesis method of compound**A19**was similar to that of compound**A1**, with a yield of 67%.$^1$H NMR (400 MHz, MeOD) δ 9.10 - 9.03 (m, 1H), 8.46 - 8.39 (m, 3H), 8.36 (d, *J* =8.1 Hz, 1H), 7.89 - 7.78 (m, 3H), 7.61 (d, *J* =8.4 Hz, 2H), 5.09 (s, 2H), 4.52 - 4.38 (m, 4H), 3.79 - 3.67 (m, 1H). $^{13}$C NMR (125 MHz, DMSO-*d6*) δ 173.2, 168.0, 151.1, 130.9, 130.1, 129.7, 128.2, 127.5, 126.9, 125.8, 124.0, 121.9, 121.3, 55.1, 31.8. ESI-MS *m*/*z*:470.1 [M+H]$^+$.

**Example 20 Synthesis of 1-((4-(5-(4-isopropylphenyl)-1,2, 4-oxadiazol-3-yl) naphthalen-1-yl) methyl) azetidine-3-carboxylic acid hydrochloric acid (A20)**

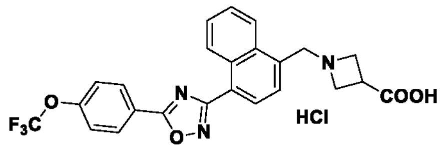

The synthesis method of compound**A20**was similar to that of compound**A1**, with a yield of 61%.$^1$H NMR (400 MHz, MeOD) δ 9.10 - 9.00 (m, 1H), 8.41 - 8.34 (m, 2H), 8.23 (d, *J* =8.1 Hz, 2H), 7.89 - 7.76 (m, 3H), 7.56 (d, *J* =8.0 Hz, 2H), 5.08 (s, 2H), 4.55 - 4.37 (m, 4H), 3.79 - 3.67 (m, 1H), 3.14 - 2.98 (m, 1H), 1.35 (d, *J* =6.9 Hz, 6H). $^{13}$C NMR (150 MHz, DMSO-*d6*) δ 175.3, 168.9, 154.9, 132.0, 130.7, 129.2, 128.7, 128.4, 128.1, 127.8, 126.8, 125.1, 121.5, 56.2, 34.1, 33.0, 24.0. ESI-MS *m*/*z*:428.1 [M+H]$^+$.

**Example 21 Synthesis of 1-(4-(5-(4-propylphenyl)-1,2, 4-oxadiazol-3-yl) naphthalen-1-yl) methyl) azetidine-3-carboxylic acid hydrochloric acid (A21)**

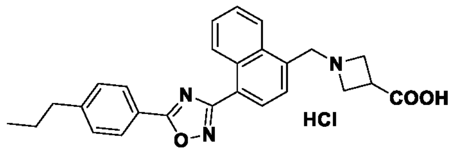

The synthesis method of compound**A21**was similar to that of compound**A1**, with a yield of 69%.$^1$H NMR (400 MHz, MeOD) δ 9.07 - 9.03 (m, 1H), 8.42 - 8.33 (m, 2H), 8.22 (d, *J* =8.0 Hz, 2H), 7.90 - 7.77 (m, 3H), 7.51 (d, *J* =8.0 Hz, 2H), 5.09 (s, 2H), 4.51 - 4.39 (m, 4H), 3.80 - 3.67 (m, 1H), 2.76 (t, *J* =7.6 Hz, 2H), 1.84 - 1.65 (m, 2H), 1.02 (t, *J* =7.4 Hz, 3H). $^{13}$C NMR (150 MHz, DMSO-*d6*) δ 175.3, 168.8, 148.9, 131.9, 130.7, 130.1, 129.2, 128.5, 127.9, 126.8, 125.1, 121.3, 56.1, 37.7, 32.9, 24.2, 14.1. ESI-MS *m*/*z*:428.1 [M+H]$^+$.

**Example 22 Synthesis of 1-((4-(5-(4-cyanophenyl)-1,2, 4-oxadiazol-3-yl) naphthalen-1-yl) methyl) azetidine-3-carboxylic acid hydrochloric acid (A22)**

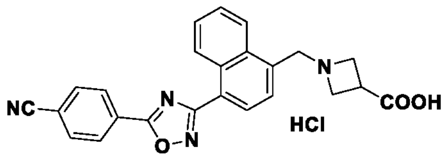

The synthesis method of compound**A22**was similar to that of compound**A1**, with a yield of 65%.$^1$H NMR (500 MHz, MeOD) δ 7.97 - 7.89 (m, 2H), 7.83 - 7.76 (m, 2H), 7.73 - 7.65 (m, 3H), 7.55 - 7.47 (m, 2H), 7.47 - 7.40 (m, 1H), 3.74 (s, 2H), 3.48 (dd, *J* = 9.0, 5.8 Hz, 2H), 3.16 (dd, *J* = 9.0, 5.8 Hz, 2H), 2.98 (p, *J* =5.8 Hz, 1H). ESI-MS *m*/*z*:411.1 [M+H]$^+$.

**Example 23 Synthesis of 1-((4-(5-(4-fluorophenyl)-1,2, 4-oxadiazol-3-yl) naphthalen-1-yl) methyl) azetidine-3-carboxylic acid hydrochloric acid (A23)**

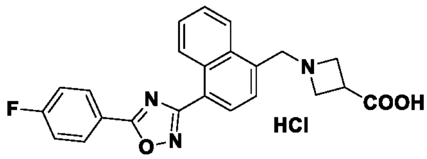

The synthesis method of compound**A23**was similar to that of compound**A1**, with a yield of 66%.$^1$H NMR (400 MHz, MeOD) δ 9.07 - 9.01 (m, 1H), 8.41 - 8.32 (m, 4H), 7.88 - 7.75 (m, 3H), 7.48 - 7.37 (m, 2H), 5.03 (s, 2H), 4.44 - 4.30 (m, 4H), 3.68 - 3.57 (m, 1H). $^{13}$C NMR (125 MHz, DMSO-*d6*) δ 174.3, 169.1, 167.3, 164.5, 132.1, 131.5, 131.4, 130.7, 129.3, 129.1, 128.3, 128.2, 127.7, 127.4, 126.8, 126.6, 125.3, 125.0, 120.6, 117.5, 117.3, 56.8, 48.1, 33.5. ESI-MS *m*/*z*:404.1 [M+H]$^+$.

**Example 24 Synthesis of 1-((4-(5-(3-cyanophenyl)-1,2, 4-oxadiazol-3-yl) naphthalen-1-yl) methyl) azetidine-3-carboxylic acid hydrochloric acid (A24)**

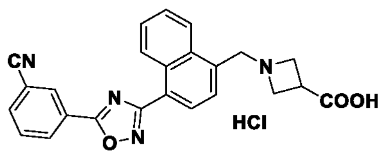

The synthesis method of compound**A24**was similar to that of compound**A1**, with a yield of 74%.$^1$H NMR (500 MHz, MeOD) δ 7.97 - 7.90 (m, 3H), 7.85 (t, *J* =1.9 Hz, 1H), 7.78 (ddd, *J* = 7.9, 1.9, 1.2 Hz, 1H), 7.71 (d, *J* =8.6 Hz, 1H),

7.56 (d, $J$ =7.8 Hz, 1H), 7.54 - 7.40 (m, 3H), 3.74 (s, 2H), 3.48 (dd, $J$ = 9.0, 5.8 Hz, 2H), 3.16 (dd, $J$ = 9.0, 5.8 Hz, 2H), 2.98 (p, $J$ =5.8 Hz, 1H). ESI-MS $m/z$:411.1 $[M+H]^+$.

**Example 25 Synthesis of 1-((4-(5-(3-fluorophenyl)-1,2, 4-oxadiazol-3-yl) naphthalen-1-yl) methyl) azetidine-3-carboxylic acid hydrochloric acid (A25)**

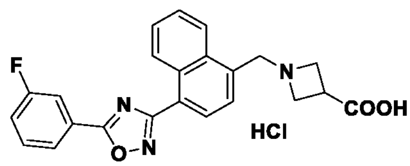

The synthesis method of compound**A25**was similar to that of compound**A1**, with a yield of 78%.$^1$H NMR (400 MHz, MeOD) δ 9.08 - 9.03 (m, 1H), 8.41 (d, $J$ =7.4 Hz, 1H), 8.38 - 8.34 (m, 1H), 8.15 (ddd, $J$ = 7.8, 1.6, 0.9 Hz, 1H), 8.04 (ddd, $J$ = 9.1, 2.6, 1.5 Hz, 1H), 7.88 - 7.78 (m, 3H), 7.72 (td, $J$ = 8.1, 5.5 Hz, 1H), 7.51 (tdd, $J$ = 8.5, 2.7, 0.9 Hz, 1H), 5.06 (s, 2H), 4.50 - 4.33 (m, 4H), 3.67 (p, $J$ =8.5 Hz, 1H). ESI-MS $m/z$:404.1 $[M+H]^+$.

**Example 26 Synthesis of 1-(4-(5-phenyl -1,2, 4-oxadiazol-3-yl) naphthalen-1-yl) methyl) azetidine-3-carboxylic acid hydrochloric acid (A26)**

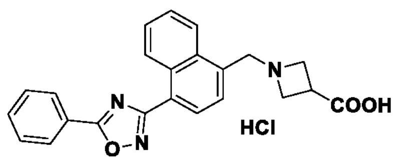

The synthesis method of compound**A26**was similar to that of compound**A1**, with a yield of 71%.$^1$H NMR (400 MHz, MeOD) δ 9.08 - 9.01 (m, 1H), 8.39 (d, $J$ =7.4 Hz, 1H), 8.36 (d, $J$=8.3 Hz, 1H), 8.33 - 8.28 (m, 2H), 7.86 - 7.71 (m, 4H), 7.70 - 7.65 (m, 2H), 5.00 (s, 2H), 4.37 - 4.25 (m, 4H), 3.53 (p, $J$ =8.1 Hz, 1H). $^{13}$C NMR (150 MHz, DMSO-$d6$) δ 175.1, 174.3, 169.1, 133.9, 132.2, 130.6, 130.1, 129.4, 128.5, 128.0, 127.1, 126.6, 126.3, 125.4, 123.9, 57.0, 33.9. ESI-MS $m/z$:386.1 $[M+H]^+$.

**Example 27 Synthesis of 1-((4-(5-(pyridine-4-yl)-1,2, 4-oxadiazol-3-yl) naphthalen-1-yl) methyl) azetidine-3-carboxylic acid hydrochloric acid (A27)**

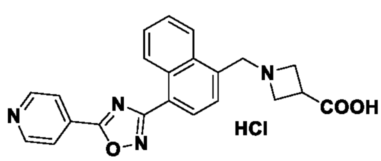

The synthesis method of compound**A27**was similar to that of compound**A1**, with a yield of 66%.$^1$H NMR (400 MHz, MeOD) δ 9.09 - 9.02 (m, 1H), 8.94 - 8.88 (m, 2H), 8.43 (d, $J$ =7.5 Hz, 1H), 8.39 - 8.34 (m, 1H), 8.29 - 8.24 (m, 2H), 7.90 - 7.78 (m, 3H), 5.05 (s, 2H), 4.44 - 4.33 (m, 4H), 3.72 - 3.58 (m, 1H). $^{13}$C NMR (125 MHz, DMSO-$d6$) δ 173.7, 169.3, 151.7, 132.1, 130.9, 130.6, 129.4, 128.3, 127.5, 126.6, 125.3, 121.9, 56.7, 33.4. ESI-MS $m/z$:387.1 $[M+H]^+$.

**Example 28 Synthesis of 1-((4-(5-(pyridine-3-yl)-1,2, 4-oxadiazol-3-yl) naphthalen-1-yl) methyl) azetidine-3-carboxylic acid hydrochloric acid (A28)**

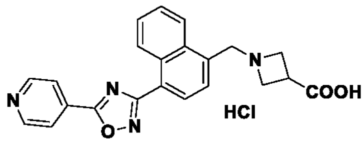

The synthesis method of compound**A28**was similar to that of compound**A1**, with a yield of 68%.$^1$H NMR (400 MHz, MeOD) δ 9.45 (dd, *J* = 2.2, 0.9 Hz, 1H), 9.08 - 9.03 (m, 1H), 8.88 (dd, *J* = 4.9, 1.7 Hz, 1H), 8.70 (ddd, *J* = 8.0, 2.3, 1.6 Hz, 1H), 8.42 (d, *J* =7.5 Hz, 1H), 8.38 - 8.34 (m, 1H), 7.88 - 7.80 (m, 3H), 7.77 - 7.73 (m, 1H), 5.03 (s, 2H), 4.42 - 4.31 (m, 4H), 3.68 - 3.55 (m, 1H). $^{13}$C NMR (150 MHz, DMSO-*d6*) δ 173.5, 169.1, 154.2, 149.1, 136.2, 132.1, 130.6, 129.4, 129.2, 128.2, 127.7, 127.4, 126.8, 126.6, 125.3, 125.0, 120.5, 56.8, 48.1, 33.6. ESI-MS *m*/*z*:387.1 [M+H]$^+$.

**Example 29 Synthesis of 1-((4-(5-(pyridine-2-yl)-1,2, 4-oxadiazol-3-yl) naphthalen-1-yl) methyl) azetidine-3-carboxylic acid hydrochloric acid (A29)**

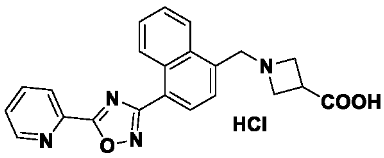

The synthesis method of compound**A29**was similar to that of compound**A1**, with a yield of 69%.$^1$H NMR (400 MHz, MeOD) δ 9.13 - 9.06 (m, 1H), 8.90 - 8.85 (m, 1H), 8.51 - 8.43 (m, 2H), 8.40 - 8.35 (m, 1H), 8.21 - 8.15 (m, 1H), 7.90 - 7.74 (m, 4H), 5.11 (s, 2H), 4.54 - 4.41 (m, 4H), 3.83 - 3.71 (m, 1H). $^{13}$C NMR (150 MHz, DMSO-*d6*) δ 174.3, 169.0, 151.2, 143.3, 138.7, 131.9, 130.7, 129.2, 128.6, 128.1, 128.0, 126.8, 125.2, 125.1, 56.0, 32.8. ESI-MS *m*/*z*:387.1 [M+H]$^+$.

**Example 30 Synthesis of 1-((4-(5-(5-methylisothiazol-3-yl)-1,2, 4-oxadiazol-3-yl) naphthalen-1-yl) methyl) azetidine-3-carboxylic acid hydrochloric acid (A30)**

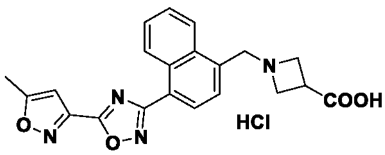

The synthesis method of compound**A30**was similar to that of compound**A1**, with a yield of 71%.$^1$H NMR (400 MHz, MeOD) δ 9.08 - 9.00 (m, 1H), 8.44 - 8.33 (m, 2H), 7.90 - 7.76 (m, 3H), 6.95 (t, *J* =0.9 Hz, 1H), 5.06 (s, 2H), 4.48 - 4.33 (m, 4H), 3.68 (p, *J* =8.4 Hz, 1H), 2.64 (d, *J* =0.9 Hz, 3H). $^{13}$C NMR (125 MHz, DMSO-*d6*) δ 187.4, 172.4, 168.0, 166.7, 149.9, 140.1, 131.0, 129.6, 128.5, 127.5, 126.7, 125.6, 124.2, 101.6, 55.5, 32.2, 11.4. ESI-MS *m*/*z*:391.1 [M+H]$^+$.

**Example 31 Synthesis of 1-((4-(5-(5-(*d*][1,3] dioxazol-5-yl)-1,2, 4-oxadiazol-3-yl) naphthalen-1-yl) methyl) azetidine-3-carboxylic acid hydrochloric acid (A31)**

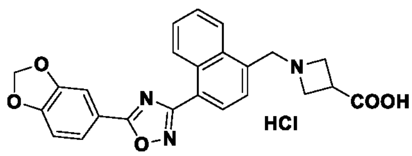

The synthesis method of compound**A31**was similar to that of compound**A1**, with a yield of 71%.$^1$H NMR (500 MHz, MeOD) δ 8.00 - 7.91 (m, 2H), 7.77 (dd, *J* = 8.4, 1.8 Hz, 1H), 7.71 (d, *J* =8.4 Hz, 1H), 7.53 (d, *J* =8.4 Hz, 1H), 7.51

- 7.39 (m, 2H), 7.33 (d, *J* =1.8 Hz, 1H), 6.91 (d, *J* =8.4 Hz, 1H), 6.09 (s, 2H), 3.74 (s, 2H), 3.49 (dd, *J* = 9.0, 5.8 Hz, 2H), 3.16 (dd, *J* = 9.0, 5.8 Hz, 2H), 2.98 (p, *J* =5.8 Hz, 1H). ESI-MS *m*/*z*:430.1 [M+H]$^+$.

**Example 32 Synthesis of 1-((4-(5-(2, 3-dihydrobenzo [*B*][1,4] dioxin-6-yl)-1,2, 4-oxadiazol-3-yl) naphthalen-1-yl) methyl) azetidine-3-carboxylic acid hydrochloric acid (A32)**

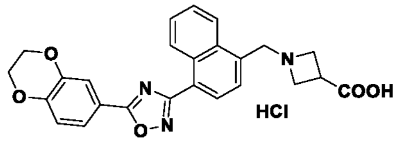

The synthesis method of compound**A32**was similar to that of compound**A1**, with a yield of 64%.$^1$H NMR (400 MHz, MeOD) δ 9.03 (dd, *J* = 7.6, 2.0 Hz, 1H), 8.40 - 8.31 (m, 2H), 7.87 - 7.75 (m, 5H), 7.11 (d, *J* =8.4 Hz, 1H), 5.09 (s, 2H), 4.50 - 4.34 (m, 8H), 3.81 - 3.70 (m, 1H). $^{13}$C NMR (125 MHz, DMSO-*d6*) δ 174.9, 168.7, 148.5, 144.4, 131.9, 130.7, 129.1, 128.5, 127.9, 126.9, 125.0, 122.3, 118.8, 117.1, 116.6, 65.1, 64.6, 56.1, 32.8. ESI-MS *m*/*z*:444.1 [M+H]$^+$.

**Example 33 Synthesis of 1-((4-(5-(quinoline-3-yl)-1,2, 4-oxadiazol-3-yl) naphthalen-1-yl) methyl) azetidine-3-carboxylic acid hydrochloric acid (A33)**

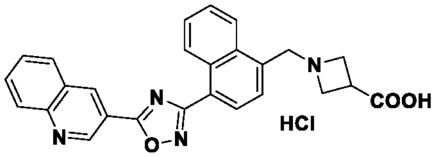

The synthesis method of compound**A33**was similar to that of compound**A1**, with a yield of 74%.$^1$H NMR (400 MHz, MeOD) δ 9.15 - 9.09 (m, 1H), 8.70 - 8.66 (m, 1H), 8.53 - 8.47 (m, 2H), 8.40 - 8.31 (m, 2H), 8.14 - 8.10 (m, 1H), 7.99 - 7.93 (m, 1H), 7.91 - 7.79 (m, 4H), 5.09 (s, 2H), 4.55 - 4.35 (m, 4H), 3.74 (q, *J* =8.5 Hz, 1H). $^{13}$C NMR (125 MHz, DMSO-*d6*) δ 174.2, 169.2, 147.8, 143.4, 138.9, 132.0, 131.7, 130.7, 130.1, 129.6, 129.4, 129.3, 128.8, 128.5, 127.8, 126.8, 125.2, 121.2, 56.4, 33.1. ESI-MS *m*/*z*:437.1 [M+H]$^+$.

**Example 34 Synthesis of 1-((4-(5-(quinoxaline-2-yl)-1,2, 4-oxadiazol-3-yl) naphthalen-1-yl) methyl) azetidine-3-carboxylic acid hydrochloric acid (A34)**

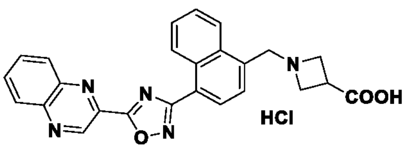

The synthesis method of compound**A34**was similar to that of compound**A1**, with a yield of 69%.$^1$H NMR (400 MHz, MeOD) δ 9.82 (s, 1H), 9.19 - 9.09 (m, 1H), 8.51 (d, *J* =7.3 Hz, 1H), 8.40 - 8.34 (m, 2H), 8.31 - 8.25 (m, 1H), 8.11 - 8.01 (m, 2H), 7.95 - 7.81 (m, 3H), 5.12 (s, 2H), 4.57 - 4.40 (m, 4H), 3.88 - 3.72 (m, 1H). $^{13}$C NMR (125 MHz, DMSO-*d6*) δ 171.7, 168.2, 166.1, 144.0, 142.2, 140.4, 138.0, 132.4, 131.4, 131.1, 131.0, 129.6, 129.6, 129.4, 128.7, 128.5, 128.3, 127.7, 127.6, 126.9, 126.8, 125.8, 125.7, 124.2, 124.1, 55.3, 46.8, 46.7, 32.0. ESI-MS *m*/*z*:438.1 [M+H]$^+$.

**Example 35 Synthesis of 1-(4-(5-(1*H*-indole -5-yl)-1,2, 4-oxadiazol-3-yl) naphthalen-1-yl) methyl) azetidine-3-carboxylic acid hydrochloric acid (A35)**

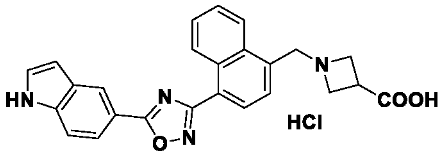

The synthesis method of compound**A35**was similar to that of compound**A1**, with a yield of 62%.$^1$H NMR (400 MHz, MeOD) δ 9.04 (dd, *J* = 7.3, 2.3 Hz, 1H), 8.57 (dd, *J* = 1.7, 0.7 Hz, 1H), 8.40 - 8.31 (m, 2H), 8.04 (dd, *J* = 8.6, 1.7 Hz, 1H), 7.88 - 7.76 (m, 3H), 7.66 - 7.59 (m, 1H), 7.44 (d, *J* =3.2 Hz, 1H), 6.70 (dd, *J* = 3.2, 0.9 Hz, 1H), 5.03 (s, 2H), 4.46 - 4.24 (m, 4H), 3.60 (p, *J* =8.4 Hz, 1H). $^{13}$C NMR (125 MHz, DMSO-*d6*) δ 176.6, 168.9, 138.9, 132.1, 130.7, 129.1, 128.3, 128.0, 127.3, 126.7, 125.2, 121.8, 121.1, 114.6, 113.0, 103.2, 56.8, 33.6. ESI-MS *m*/*z*:425.1 [M+H]$^+$.

**Example 36 Synthesis of 1-(4-(5-(1*H*-indole -2-yl)-1,2, 4-oxadiazol-3-yl) naphthalen-1-yl) methyl) azetidine-3-carboxylic acid hydrochloric acid (A36)**

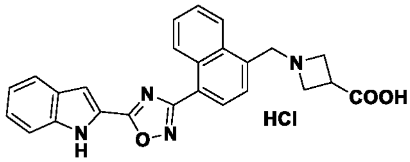

The synthesis method of compound**A6**was similar to that of compound**A1**, with a yield of 55%.$^1$H NMR (400 MHz, MeOD) δ 9.09 - 9.03 (m, 1H), 8.41 (d, *J* =7.4 Hz, 1H), 8.37 (dd, *J* = 6.9, 2.7 Hz, 1H), 8.02 (d, *J* =1.0 Hz, 1H), 7.92 - 7.80 (m, 4H), 7.78 - 7.73 (m, 1H), 7.63 - 7.56 (m, 1H), 7.49 - 7.41 (m, 1H), 5.03 (s, 2H), 4.44 - 4.28 (m, 4H), 3.64 - 3.54 (m, 1H). $^{13}$C NMR (125 MHz, DMSO-*d6*) δ 168.0, 166.5, 154.9, 139.8, 131.1, 129.6, 128.5, 127.7, 127.1, 126.4, 126.3, 125.5, 124.3, 124.0, 122.7, 113.1, 111.7, 55.9, 32.7. ESI-MS *m*/*z*:425.1 [M+H]$^+$.

**Example 37 Synthesis of 1-((4-(5-(1-methyl -1*H*-indole -2-yl)-1,2, 4-oxadiazol-3-yl) naphthalen-1-yl) methyl) azetidine-3-carboxylic acid hydrochloric acid (A37)**

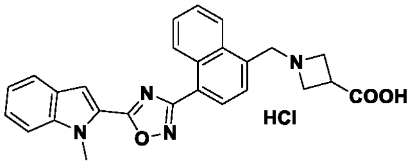

The synthesis method of compound**A37**was similar to that of compound**A1**, with a yield of 61 %.$^1$H NMR (400 MHz, MeOD) δ 9.12 - 9.06 (m, 1H), 8.43 (d, *J* =7.4 Hz, 1H), 8.36 (dd, *J* = 6.8, 2.4 Hz, 1H), 7.88 - 7.80 (m, 3H), 7.77 (d, *J* =8.1 Hz, 1H), 7.61 (d, *J* =8.7 Hz, 2H), 7.47 - 7.40 (m, 1H), 7.25 - 7.19 (m, 1H), 5.04 (s, 2H), 4.43 - 4.35 (m, 4H), 4.33 (s, 3H), 3.64 (p, *J* =8.4 Hz, 1H). $^{13}$C NMR (125 MHz, DMSO-*d6*) δ 168.6, 167.6, 139.0, 131.1, 129.7, 128.4, 127.3, 126.5, 125.6, 124.8, 124.2, 122.0, 121.6, 120.4, 110.6, 108.3, 55.6, 32.3, 31.5. ESI-MS *m*/*z*:439.1 [M+H]$^+$.

**Example 38 Synthesis of (*E*)-1-((4-(5-styryl-1, 2,4-oxadiazol-3-yl) naphthalen-1-yl) methyl) azetidine-3-carboxylic acid hydrochloric acid (A38)**

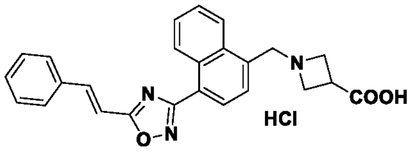

The synthesis method of compound**A38**was similar to that of compound**A1**, with a yield of 66%.$^1$H NMR (400 MHz, MeOD) δ 9.04 - 8.96 (m, 1H), 8.40 - 8.27 (m, 2H), 8.04 (d, *J* =16.5 Hz, 1H), 7.86 - 7.73 (m, 5H), 7.53 - 7.44 (m, 3H), 7.36 (d, *J* =16.4 Hz, 1H), 5.03 (s, 2H), 4.45 - 4.31 (m, 3H), 3.64 (p, *J* =8.5 Hz, 1H). $^{13}$C NMR (125 MHz, DMSO-*d6*) δ 175.1, 173.6, 168.8, 167.4, 143.5, 134.7, 132.1, 131.2, 130.6, 129.5, 129.1, 128.9, 128.1, 127.3, 126.8, 126.6, 125.2, 110.7, 56.7, 48.1, 33.6. ESI-MS **m/z**:412.1 [M+H]$^+$.

**Example 39 Synthesis of 1-(4-(5-cyclohexyl -1,2, 4-oxadiazol-3-yl) naphthalen-1-yl) methyl) azetidine-3-carboxylic acid hydrochloric acid (A39)**

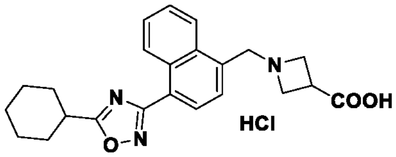

The synthesis method of compound**A39**was similar to that of compound**A1**, with a yield of 81%.$^1$H NMR (400 MHz, MeOD) δ 8.98 - 8.92 (m, 1H), 8.34 (dd, *J* = 8.0, 1.5 Hz, 1H), 8.27 (d, *J* =7.4 Hz, 1H), 7.86 - 7.73 (m, 3H), 5.06 (s, 2H), 4.51 - 4.33 (m, 4H), 3.72 (p, *J* =8.5 Hz, 1H), 3.26 - 3.07 (m, 1H), 2.28 - 2.16 (m, 2H), 1.96 - 1.85 (m, 2H), 1.85 - 1.71 (m, 3H), 1.61 - 1.47 (m, 2H), 1.49 - 1.35 (m, 1H). $^{13}$C NMR (125 MHz, DMSO-*d6*) δ 182.7, 168.0, 131.9, 130.6, 129.0, 128.4, 127.7, 126.7, 125.0, 56.2, 35.7, 32.9, 30.2, 25.6, 25.2. ESI-MS *m*/*z*:392.1 [M+H]$^+$.

**Example 40 Synthesis of 1-((4-(5-(3-cyano-4-isopropyloxyphenyl)-1,2, 4-oxadiazol-3-yl) naphthalen-1-yl) methyl) piperidin-4-carboxylic acid hydrochloric acid (A40)**

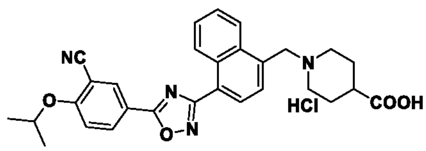

The synthesis method of compound**A40**was similar to that of compound **A1**.$^1$H NMR (500 MHz, Chloroform-*d*) δ 8.24 (dd, *J* = 7.3, 1.6 Hz, 1H), 7.64 (d, *J* =7.5 Hz, 2H), 7.56 - 7.43 (m, 3H), 7.35 (d, *J* =2.0 Hz, 1H), 7.16 (dd, *J* = 7.5, 2.0 Hz, 1H), 4.43 (m, 1H), 3.39 (s, 2H), 3.16 (dt, *J* = 12.4, 7.0 Hz, 2H), 2.88 (p, *J* =7.0 Hz, 1H), 2.33 (dt, *J* = 12.5, 7.1 Hz, 2H), 1.98 (dq, *J* = 14.1, 7.1 Hz, 2H), 1.72 - 1.61 (m, 2H), 1.36 (d, *J* =6.8 Hz, 6H). $^{13}$C NMR (125 MHz, DMSO-*d6*) δ 178.14 , 172.69 , 166.15 , 159.05 , 134.27 , 132.00 , 131.29 , 131.05 , 129.57, 128.91 , 127.75, 127.03 , 125.31 , 124.41 , 115.46 , 113.72 , 105.66 , 72.86 , 58.88 , 50.85 , 40.41 , 28.26 , 21.93. ESI-MS *m*/*z*:497.2 [M+H]$^+$.

**Example 41 Synthesis of 1-((4-(5-(3-cyano-4-isopropyloxyphenyl)-1,2, 4-oxadiazol-3-yl) naphthalen-1-yl) methyl) piperidin-3-carboxylic acid hydrochloric acid (A41)**

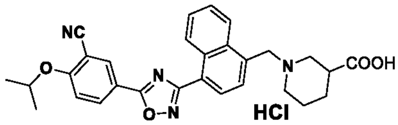

The synthesis method of compound**A41**was similar to that of compound **A1**.$^1$H NMR (500 MHz, Chloroform-*d*) δ 8.25 (dd, *J* = 7.4, 1.6 Hz, 1H), 7.98 (dd, *J* = 7.4, 1.5 Hz, 1H), 7.64 (dd, *J* = 7.5, 1.1 Hz, 2H), 7.56 - 7.43 (m, 3H), 7.34 (d, *J* =2.0 Hz, 1H), 7.16 (dd, *J* = 7.5, 2.0 Hz, 1H), 4.49 - 4.41 (m, 1H), 4.38 (t, *J* =12.8 Hz, 1H), 3.78 (d, *J* =12.3 Hz, 1H), 3.15 (dq, *J* = 12.3, 7.0 Hz, 2H), 2.65 (dd, *J* = 12.4, 7.0 Hz, 1H), 2.53 (p, *J* =6.9 Hz, 1H), 2.27 (ddt, *J* = 29.0, 12.4, 7.0 Hz, 2H), 1.75 - 1.63 (m, 1H), 1.63 - 1.46 (m, 2H), 1.36 (dd, *J* = 24.9, 6.8 Hz, 6H). $^{13}$C NMR ((125 MHz, DMSO-*d6)* δ 174.94 ,

172.69 , 166.15 , 159.05 , 134.27 , 132.00 , 131.29 , 131.05 , 129.62 , 129.52 , 128.91 , 127.81 , 127.68 , 127.03 , 125.31 , 124.41 , 115.46 , 113.72 , 105.66 , 72.86 , 58.90 , 55.40 , 49.52 , 41.86 , 25.14 , 22.81 , 21.93. ESI-MS *m/z*:497.2 $[M+H]^+$.

**Example 42 Synthesis of 1-((4-(5-(3-cyano-4-isopropyloxyphenyl)-1,2, 4-oxadiazol-3-yl) naphthalen-1-yl) methyl) piperidine-3, 4-carboxylic acid hydrochloric acid (A42)**

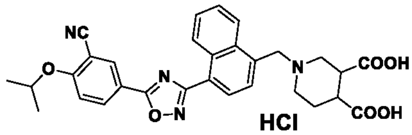

The synthesis method of compound**A42**was similar to that of compound**A1**.$^1$H NMR (500 MHz, Chloroform-*d*) δ 8.26 (dd, *J* = 7.5, 1.5 Hz, 1H), 7.87 (dd, *J* = 7.4, 1.5 Hz, 1H), 7.65 (dd, *J* = 7.5, 1.3 Hz, 2H), 7.59 - 7.49 (m, 2H), 7.46 (td, *J* = 7.5, 1.7 Hz, 1H), 7.34 (d, *J* =2.2 Hz, 1H), 7.16 (dd, *J* = 7.5, 2.0 Hz, 1H), 4.44 (dq, *J* = 13.6, 6.8 Hz, 1H), 4.38 (d, *J* =12.4 Hz, 1H), 3.76 (d, *J* =12.2 Hz, 1H), 3.47 - 3.37 (m, 1H), 3.21 - 3.11 (m, 1H), 2.75 - 2.65 (m, 2H), 2.54 - 2.44 (m, 1H), 2.35 - 2.22 (m, 2H), 1.82 - 1.70 (m, 1H), 1.36 (dd, *J* = 25.1, 6.8 Hz, 6H). $^{13}$C NMR (125 MHz, DMSO-*d6)* δ 178.27 , 176.57 , 172.69 , 166.15 , 159.05 , 134.27 , 132.00 , 131.29 , 131.05 , 129.57 (d, *J* =12.4 Hz), 128.91 , 127.75 (d, *J* =16.2 Hz), 127.03 , 125.31 , 124.41 , 115.46 , 113.72 , 105.66 , 72.86 , 58.90 , 53.78 , 53.20 , 42.81 , 27.48 , 21.93. ESI-MS *m/z*:541.2 [M H].

**Example 43 Synthesis of 1-((4-(5-(3-cyano-4-isopropyloxyphenyl)-1,2, 4-oxadiazol-3-yl) naphthalen-1-yl) methyl) pyrrolidin-3-sulfonic acid(A43)**

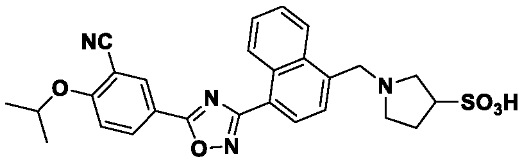

The synthesis method of compound**A43**was similar to that of compound **A1**.$^1$H NMR (500 MHz, Chloroform-*d*) δ 8.25 (dd, *J* = 7.4, 1.6 Hz, 1H), 7.99 (dd, *J* = 7.3, 1.6 Hz, 1H), 7.79 (s, 1H), 7.64 (dd, *J* = 7.5, 2.8 Hz, 2H), 7.56 - 7.43 (m, 3H), 7.34 (d, *J* =2.0 Hz, 1H), 7.15 (dd, *J* = 7.5, 2.0 Hz, 1H), 4.49 - 4.35 (m, 2H), 3.76 (d, *J* =12.4 Hz, 1H), 3.40 (dd, *J* = 9.5, 7.0 Hz, 1H), 3.10 (ddt, *J* = 16.9, 14.0, 7.0 Hz, 2H), 2.67 (dd, *J* = 9.5, 7.0 Hz, 1H), 2.46 (dq, *J* = 12.8, 7.0 Hz, 1H), 2.35 (dt, *J* = 9.5, 7.1 Hz, 1H), 2.15 (dq, *J* = 12.9, 7.0 Hz, 1H), 1.36 (dd, *J* = 25.1, 6.8 Hz, 6H). $^{13}$C NMR (125 MHz, DMSO-*d6)* δ 172.69 , 166.15 , 159.05 , 134.27 , 132.00 , 131.29 , 131.05 , 129.57 (d, *J* =12.4 Hz), 128.91 , 127.75 (d, *J* =16.2 Hz), 127.03 , 125.31 , 124.41 , 115.46 , 113.72 , 105.66 , 72.86 , 67.47 , 59.06 , 56.90 , 52.53 , 28.58 , 21.93. ESI-MS *m/z*:519.2 [M H].

**Example 44 Synthesis of methyl 1-((4-(5-(3-cyano-4-isopropoxyphenyl)-1,2, 4-oxadiazol-3-yl) naphthalen-1-yl) methyl) azelaic acid -3-carboxylater(A44)**

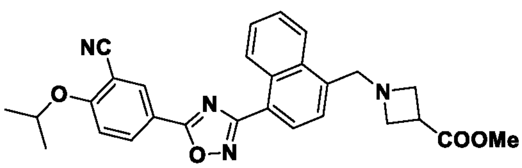

The synthesis method of compound**A44**was similar to that of compound**A1**.$^1$H NMR (500 MHz, Chloroform-d)

δ 8.25 (dd, *J* = 7.4, 1.6 Hz, 1H), 8.00 (dd, *J* = 7.4, 1.6 Hz, 1H), 7.64 (dd, *J* = 7.5, 4.4 Hz, 2H), 7.56 - 7.43 (m, 3H), 7.34 (d, *J* =2.0 Hz, 1H), 7.15 (dd, *J* = 7.5, 1.8 Hz, 1H), 4.43 (h, *J* =6.8 Hz, 1H), 3.81 (dd, *J* = 11.1, 6.9 Hz, 2H), 3.67 (s, 3H), 3.39 (s, 2H), 3.23 (dd, *J* = 11.1, 6.9 Hz, 2H), 3.14 (p, *J* =6.8 Hz, 1H), 1.36 (d, *J* =6.8 Hz, 6H). $^{13}$C NMR (125 MHz, DMSO-*d6*) δ 173.55 , 172.69 , 166.15 , 159.05 , 134.27 , 132.00 , 131.29 , 131.05 , 129.57 (d, *J* =12.4 Hz), 128.91 , 127.75 (d, *J* =16.2 Hz), 127.03 , 125.31 , 124.41 , 115.46 , 113.72 , 105.66 , 72.86 , 57.90 , 51.81 , 43.42 , 42.29 , 21.93 ..ESI-MS *m*/*z*:483.2 [M H].

**Example 45 Synthesis ofEthyl 1-((4-(5-(3-cyano-4-isopropoxyphenyl)-1,2, 4-oxadiazol-3-yl) naphthalen-1-yl) methyl) azelaic acid -3-carboxylate(A45)**

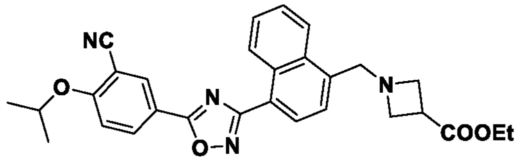

The synthesis method of compound**A45**was similar to that of compound**A1**.$^1$H NMR (500 MHz, Chloroform-*d*) δ 8.25 (dd, *J* = 7.3, 1.6 Hz, 1H), 7.98 (dd, *J* = 7.4, 1.6 Hz, 1H), 7.64 (dd, *J* = 7.5, 4.4 Hz, 2H), 7.53 (dd, *J* = 7.4, 1.6 Hz, 1H), 7.52 - 7.43 (m, 2H), 7.34 (d, *J* =2.0 Hz, 1H), 7.15 (dd, *J* = 7.5, 2.0 Hz, 1H), 4.43 (hept, *J* =6.8 Hz, 1H), 4.17 (q, *J* =5.9 Hz, 2H), 3.82 (dd, *J* = 10.8, 6.7 Hz, 2H), 3.39 (s, 2H), 3.26 - 3.11 (m, 3H), 1.36 (d, *J* =6.8 Hz, 6H), 1.23 (t, *J* =5.9 Hz, 3H). $^{13}$C NMR (125 MHz, DMSO-*d6*) δ 172.69, 172.53 , 166.15, 159.05, 134.27 , 132.00, 131.29, 131.05 , 129.57 (d, *J* =12.4 Hz), 128.91 , 127.75 (d, *J* =16.2 Hz), 127.03 , 125.31 , 124.41 , 115.46 , 113.72 , 105.66 , 72.86 , 60.82 , 57.90 , 43.42 , 42.31 , 21.93 , 14.26. ESI-MS *m*/*z*:497.2 [M H].

**Example 46 Synthesis ofisopropyl 1-((4-(5-(3-cyano-4-isopropoxyphenyl)-1,2, 4-oxadiazol-3-yl) naphthalen-1-yl) methyl) azelaic acid -3-carboxylater(A46)**

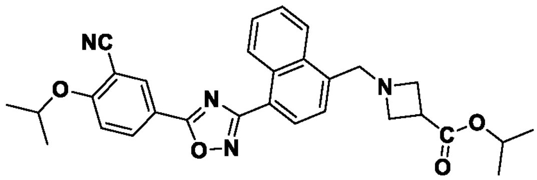

The synthesis method of compound**A46** was similar to that of compound**A1**.$^1$H NMR (500 MHz, Chloroform-d) δ 8.25 (dd, *J* = 7.5, 1.6 Hz, 1H), 7.99 (dd, *J* = 7.4, 1.5 Hz, 1H), 7.65 (dd, *J* = 9.6, 7.4 Hz, 2H), 7.56 - 7.43 (m, 3H), 7.32 (d, J =2.0 Hz, 1H), 7.15 (dd, *J* = 7.5, 2.0 Hz, 1H), 5.04 (hept, *J* =6.8 Hz, 1H), 4.43 (hept, *J* =6.9 Hz, 1H), 3.82 (dd, *J* = 10.8, 6.7 Hz, 2H), 3.39 (s, 2H), 3.27 - 3.12 (m, 3H), 1.36 (d, *J* =6.8 Hz, 6H), 1.23 (d, *J* =6.9 Hz, 6H). $^{13}$C NMR (125 MHz, DMSO-*d6*) δ 172.69 , 170.80 , 166.15 , 159.05 , 134.27 , 132.00 , 131.29 , 131.05 , 129.62 , 129.52, 128.91 , 127.81 , 127.68 , 127.03 , 125.31 , 124.41 , 115.46, 113.72, 105.66, 72.86 , 67.81 , 57.90 , 43.42 , 42.42 , 21.93 , 21.90. ESI-MS *m*/*z*:511.2 [M H].

**Example 47 Synthesis of 1-(4-(5-(3-cyano-4-isopropoxyphenyl)-1,2, 4-oxadiazol-3-yl) naphthalen-1-yl) methyl) azelaic acid-3-carboxamide(A47)**

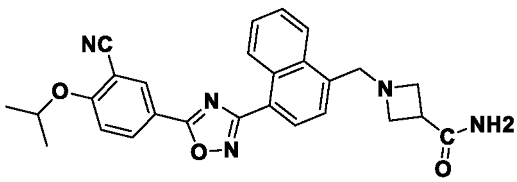

The synthesis method of compound**A47** was similar to that of compound**A1**.$^1$H NMR (500 MHz, Chloroform-*d*) δ 8.25 (dd, *J* = 7.4, 1.5 Hz, 1H), 8.01 (dd, *J* = 7.4, 1.6 Hz, 1H), 7.73 (d, *J* =7.5 Hz, 1H), 7.63 (d, *J* =7.5 Hz, 1H), 7.56 - 7.43 (m, 3H), 7.23 (d, *J* =2.0 Hz, 1H), 7.09 (dd, *J* = 7.5, 2.0 Hz, 1H), 5.68 (s, 2H), 4.43 (hept, *J* =6.9 Hz, 1H), 3.77 (dd, *J* = 11.1, 6.9 Hz, 2H), 3.48 - 3.39 (m, 2H), 3.24 (dd, *J* = 11.1, 7.0 Hz, 2H), 1.36 (d, *J* =6.8 Hz, 6H). $^{13}$C NMR (125 MHz, DMSO-*d6*) δ 172.67 (d, *J* =4.7 Hz), 166.15 , 159.05 , 134.27 , 132.00 , 131.29 , 131.05 , 129.57 (d, *J* =12.4 Hz), 128.91 , 127.75 (d, *J* =16.2 Hz), 127.03 , 125.31 , 124.41 , 115.46 , 113.72 , 105.66 , 72.86 , 57.90 , 47.45 , 41.10 , 21.93. ESI-MS m/z:468.2 [M H].

**Example 48 Synthesis of 1-(4-(5-(3-cyano-4-isopropoxyphenyl)-1,2, 4-oxadiazol-3-yl) naphthalen-1-yl) methyl)-N-methylazetidine-3-formamide(A48)**

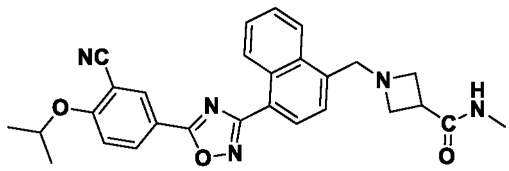

The synthesis method of compound**A48** was similar to that of compound**A1**.$^1$H NMR (500 MHz, Chloroform-*d*) δ 8.25 (dd, *J* = 7.4, 1.5 Hz, 1H), 8.01 (dd, *J* = 7.4, 1.6 Hz, 1H), 7.64 (dd, *J* = 7.5, 5.5 Hz, 2H), 7.52 (td, *J* = 7.7, 7.2, 1.8 Hz, 2H), 7.46 (td, *J* = 7.5, 1.6 Hz, 1H), 7.34 (d, *J* =2.0 Hz, 1H), 7.15 (dd, *J* = 7.5, 2.0 Hz, 1H), 5.98 (s, 1H), 4.43 (hept, *J* =6.8 Hz, 1H), 3.76 (dd, *J* = 11.1, 7.0 Hz, 2H), 3.44 (p, *J* =7.0 Hz, 2H), 3.39 (s, 1H), 3.24 (dd, *J* = 11.1, 7.0 Hz, 2H), 2.80 (s, 3H), 1.36 (d, *J* =6.8 Hz, 6H). $^{13}$C NMR (125 MHz, DMSO-*d6*) δ 172.69 , 172.45 , 166.15 , 159.05 , 134.27 , 132.00 , 131.29 , 131.05 , 129.62 , 129.52 , 128.91 , 127.81 , 127.68 , 127.03 , 125.31 , 124.41 , 115.46 , 113.72 , 105.66 , 72.86 , 57.90 , 46.88 , 41.89 , 26.32 , 21.93 ..ESI-MS *m*/*z*:482.2 [M H].

**Example 49 Synthesis of 1-(4-(5-(3-cyano-4-isopropoxyphenyl)-1,2, 4-oxadiazol-3-yl) naphthalen-1-yl) methyl)-N, N-dimethylazetidine-3-formamide(A49)**

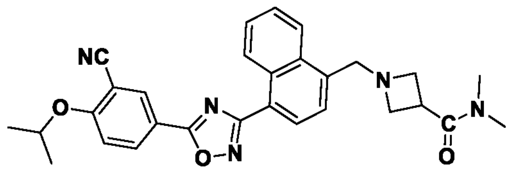

The synthesis method of compound**A49** was similar to that of compound**A1**.$^1$H NMR (500 MHz, Chloroform-*d*) δ 8.25 (dd, *J* = 7.4, 1.5 Hz, 1H), 7.99 (dd, *J* = 7.4, 1.5 Hz, 1H), 7.65 (d, *J* =7.5 Hz, 2H), 7.56 - 7.49 (m, 2H), 7.46 (td, *J* = 7.4, 1.6 Hz, 1H), 7.34 (d, *J* =2.0 Hz, 1H), 7.16 (dd, *J* = 7.5, 2.0 Hz, 1H), 4.44 (hept, *J* =6.8 Hz, 1H), 3.80 (dd, *J* = 11.1, 7.0 Hz, 2H), 3.61 (p, *J* =6.9 Hz, 1H), 3.39 (s, 2H), 3.22 (dd, *J* = 11.1, 6.9 Hz, 2H), 3.04 (s, 6H), 1.36 (d, *J* =6.8 Hz, 6H). $^{13}$C NMR (125 MHz, DMSO-*d6*) δ 173.60, 172.69, 166.15, 159.05, 134.27 , 132.00, 131.29, 131.05 , 129.57 (d, *J* =12.4 Hz), 128.91 , 127.75 (d, *J* =16.2 Hz), 127.03 , 125.31 , 124.41 , 115.46, 113.72, 105.66, 72.86 , 57.90 , 46.35 , 42.85 , 35.58 , 21.93. ESI-MS *m*/*z*:496.2 [M H].

**Example 50**Synthesis **of5-(3-((3-(1H-tetrazol-1-yl) azelaic acid -1-yl) methyl) naphthalen-1-yl)-1,2, 4-oxadiazol-5-yl)-2-isopropoxybenzonitrile (A50)**

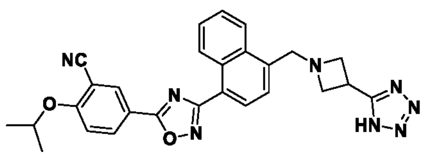

The synthesis method of compound**A50** was similar to that of compound**A1**.$^1$H NMR (500 MHz, Chloroform-*d*) δ 8.26 (dd, *J* = 7.5, 1.6 Hz, 1H), 8.02 (dd, *J* = 7.4, 1.6 Hz, 1H), 7.65 (dd, *J* = 7.4, 1.9 Hz, 2H), 7.56 - 7.49 (m, 2H), 7.47 (td, *J* = 7.4, 1.7 Hz, 1H), 7.35 (d, *J* =2.0 Hz, 1H), 7.16 (dd, *J* = 7.4, 1.9 Hz, 1H), 4.43 (h, *J* =6.8 Hz, 1H), 3.93 (dd, *J* = 11.2, 7.0 Hz, 2H), 3.78 (p, *J* =7.0 Hz, 1H), 3.39 (s, 2H), 3.20 (dd, *J* = 11.1, 6.9 Hz, 2H), 1.36 (d, *J* =6.8 Hz, 6H). $^{13}$C NMR (125 MHz, DMSO-*d6*) δ 172.69 , 166.15 , 159.05 , 144.67 , 134.27 , 132.00 , 131.29 , 131.05 , 129.62 , 129.52 , 128.91 , 127.81 , 127.68 , 127.03 , 125.31 , 124.41 , 115.46 , 113.72 , 105.66 , 72.86 , 57.90 , 46.90 , 37.35 , 21.93 . ESI-MS *m*/*z*:493.2 [M H].

**Example 51 Synthesis of 3-(3-(3-(1H-tetrazol-5-yl) azelaic acid -1-yl) methyl) naphthalen-1-yl)-5-(4-isopropoxy-3-(trifluoromethyl) phenyl)-1,2, 4-oxadiazole(A51)**

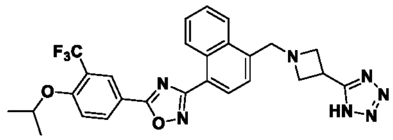

The synthesis method of compound**A51** was similar to that of compound**A1**.$^1$H NMR (500 MHz, Chloroform-*d*) δ 8.27 (dd, *J* = 7.3, 1.6 Hz, 1H), 8.02 (dd, *J* = 7.4, 1.6 Hz, 1H), 7.65 (d, *J* =7.5 Hz, 1H), 7.56 - 7.43 (m, 4H), 7.24 (dd, *J* = 2.0, 1.0 Hz, 1H), 6.98 (dd, *J* = 7.5, 2.0 Hz, 1H), 4.43 (h, *J* =6.9 Hz, 1H), 3.93 (dd, *J* = 11.2, 7.0 Hz, 2H), 3.78 (p, *J* =7.0 Hz, 1H), 3.39 (s, 2H), 3.20 (dd, *J* = 11.2, 6.9 Hz, 2H), 1.35 (d, *J* =6.9 Hz, 6H). $^{13}$C NMR (125 MHz, DMSO-*d6*) δ 172.69 , 166.15 , 154.72 , 144.67 , 132.69 , 132.00 , 131.05 , 129.62 , 129.52 , 128.91 , 127.81 , 127.68 , 127.03 , 126.67 , 126.64 , 125.87 , 125.40 , 125.31 , 124.41 , 123.72 , 121.65 , 121.40 , 115.12 , 115.10 , 115.09 , 72.86 , 57.90 , 46.90 , 37.35 , 21.93 . ESI-MS *m*/*z*:536.2 [M H].

**Example 52 Synthesis of 3-(4-((3-(1H-tetrazol-5-yl) azelaic acid -1-yl) methyl) naphthalen-1-yl)-5-(1H-indole -5-yl)-1,2, 4-oxadiazole (A52)**

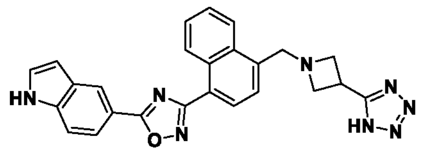

3-

The synthesis method of compound**A52** was similar to that of compound**A1**.$^1$H NMR (500 MHz, Chloroform-*d*) δ 9.83 (s, 1H), 8.31 (dd, *J* = 7.4, 1.6 Hz, 1H), 8.23 (s, 1H), 7.99 (dd, *J* = 7.4, 1.6 Hz, 1H), 7.66 (d, *J* =7.5 Hz, 1H), 7.60 (t, *J* =1.6 Hz, 1H), 7.57 - 7.48 (m, 3H), 7.46 (dd, *J* = 7.5, 1.5 Hz, 1H), 7.23 - 7.15 (m, 2H), 6.66 (dd, *J* = 7.4, 1.5 Hz, 1H), 5.47 (p, *J* =7.0 Hz, 1H), 4.02 (dd, *J* = 11.2, 7.0 Hz, 2H), 3.39 (s, 2H), 3.27 (dd, *J* = 11.2, 7.0 Hz, 2H). $^{13}$C NMR (125 MHz, DMSO-*d6)* δ 172.69 , 166.15 , 142.71 , 139.91 , 132.00 , 131.05 , 129.57 (d, *J* =12.4 Hz), 128.91 , 127.99 - 127.58 (m), 127.00 (d, *J* =7.6 Hz), 125.80, 125.31 , 124.78 , 124.41 , 122.29 , 112.56 , 102.91 , 57.90 , 53.88 , 50.83 ..ESI-MS *m*/*z*:449.2 [M H].

**Example 53 Synthesis of 3-(4-((3-(1H-tetrazol-1-yl) azelaic acid -1-yl) methyl) naphthalen-1-yl)-5-(1H-indole -6-yl)-1,2, 4-oxadiazole (A53)**

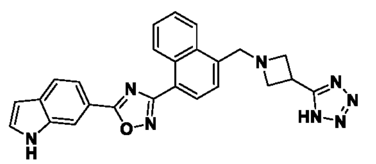

The synthesis method of compound**A53** was similar to that of compound**A1**.$^1$H NMR (500 MHz, Chloroform-*d*) δ 9.83 (s, 1H), 8.30 (dd, *J* = 7.4, 1.6 Hz, 1H), 8.03 - 7.98 (m, 2H), 7.80 (dd, *J* = 7.4, 1.5 Hz, 1H), 7.66 (d, *J* =7.5 Hz, 1H), 7.57 - 7.49 (m, 2H), 7.47 (td, *J* = 7.5, 1.7 Hz, 1H), 7.34 - 7.26 (m, 2H), 7.18 (d, *J* =7.5 Hz, 1H), 6.56 (dd, *J* = 7.5, 1.6 Hz, 1H), 5.48 (p, *J* =7.0 Hz, 1H), 4.02 (dd, *J* = 11.2, 7.0 Hz, 2H), 3.39 (s, 2H), 3.28 (dd, *J* = 11.2, 7.0 Hz, 2H). $^{13}$C NMR (125 MHz, DMSO-*d6*) δ 172.69 , 166.15 , 142.71 , 135.24 , 132.00 , 131.05 , 129.57 (d, *J* =12.4 Hz), 128.91 , 128.65 , 127.74 (d, *J* =15.3 Hz), 127.03 , 125.31 , 125.04 , 124.41 , 121.84 (d, *J* =7.7 Hz), 112.61 , 102.70 , 57.90 , 53.88 , 50.83. ESI-MS *m*/*z*:449.2 [M H].

**Example 54 Synthesis of 3-(4-(3-(1H-tetrazol-1-yl) azelaic acid -1-yl) methyl) naphthalen-1-yl)-5-(1H-indole-2-yl)-1,2, 4-oxadiazole (A54)**

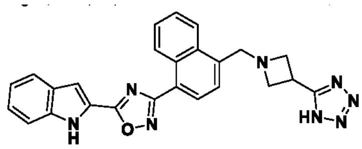

The synthesis method of compound A54 was similar to that of compound A1.$^1$H NMR (500 MHz, Chloroform-*d*) δ 9.83 (s, 1H), 9.34 (s, 1H), 8.30 (dd, *J* = 7.4, 1.6 Hz, 1H), 8.01 (dd, *J* = 7.4, 1.6 Hz, 1H), 7.74 (dt, *J* = 7.5, 1.6 Hz, 1H), 7.67 (dd, *J* = 7.4, 2.2 Hz, 2H), 7.58 - 7.50 (m, 2H), 7.47 (td, *J* = 7.4, 1.6 Hz, 1H), 7.19 - 7.08 (m, 2H), 7.00 (td, *J* = 7.4, 1.5 Hz, 1H), 5.48 (p, J =7.1 Hz, 1H), 4.02 (dd, *J* = 11.2, 7.0 Hz, 2H), 3.39 (s, 2H), 3.28 (dd, *J* = 11.2, 7.0 Hz, 2H). $^{13}$C NMR (125 MHz, DMSO-*d6*) δ 167.59 , 166.52 , 142.71 , 136.46 , 132.00 , 131.05 , 129.57 (d, *J* =12.4 Hz), 128.91 , 128.46 , 127.75 (d, *J* =16.2 Hz), 127.03 , 125.31 , 124.41 , 122.50 , 121.37 (d, *J* =13.3 Hz), 116.68 , 111.94 , 107.96 , 57.90 , 53.88 , 50.83. ESI-MS *m*/*z*:449.2 [M H].

**Example 55 Synthesis of diethyl (1-(4-(5-(3-cyano-4-isopropoxyphenyl)-1,2, 4-oxadiazol-3-yl) naphthalen-1-yl) methyl) azelaic acid -3-yl) phosphonate(A55)**

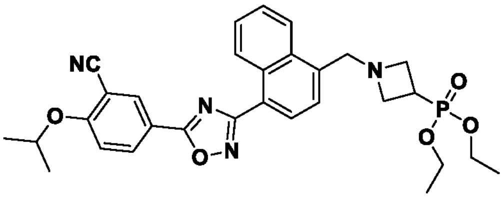

The synthesis method of compound**A55**was similar to that of compound**A1**.$^1$H NMR (500 MHz, Chloroform-*d*) δ 8.25 (dd, *J* = 7.4, 1.5 Hz, 1H), 7.89 (dd, *J* = 7.4, 1.5 Hz, 1H), 7.74 (dd, *J* = 9.3, 7.5 Hz, 2H), 7.64 (d, *J* =7.5 Hz, 1H), 7.52 (td, *J* = 7.4, 1.7 Hz, 1H), 7.46 (td, *J* = 7.5, 1.6 Hz, 1H), 7.23 (d, *J* =2.1 Hz, 1H), 7.09 (dd, *J* = 7.5, 2.0 Hz, 1H), 4.43 (hept, *J* =6.8 Hz, 1H), 4.24 - 4.10 (m, 6H), 3.39 (s, 2H), 3.19 (dd, *J* = 11.2, 7.0 Hz, 2H), 2.52 (dp, *J* = 11.4, 7.0 Hz, 1H), 1.39 - 1.28 (m, 12H). $^{13}$C NMR (125 MHz, DMSO-*d6*) δ 172.69, 166.15 , 159.05 , 134.27 , 132.00, 131.29, 131.05 , 129.57 (d, *J* =12.4 Hz), 128.91 , 127.75 (d, *J* =16.2 Hz), 127.03 , 125.31 , 124.41 , 115.46, 113.72, 105.66, 72.86 , 62.14 (d, *J* =5.7 Hz), 57.90 (d, *J* =3.8 Hz), 47.33 (d, *J* =6.7 Hz), 21.93 , 16.52 (d, *J*=5.7 Hz). ESI-MS *m*/*z*:561.2 [M H].

**Example 56 Synthesis of 1-(5-(5-(3-cyano-4-isopropoxyphenyl)-1,2, 4-oxadiazol-3-yl) quinolin-8-yl) methyl) azelaic acid -3-carboxylic acid hydrochloric acid (A56)**

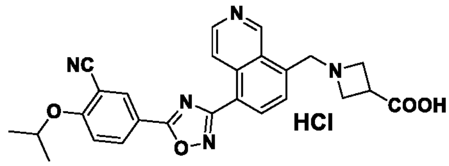

The synthesis method of compound**A56**was similar to that of compound**A1**.$^1$H NMR (500 MHz, Chloroform-*d*) δ 9.00 (dd, *J* = 7.4, 1.6 Hz, 1H), 8.32 (dd, *J* = 7.4, 1.5 Hz, 1H), 7.72 (d, *J* =7.5 Hz, 1H), 7.66 - 7.60 (m, 2H), 7.56 (t, *J* =7.5 Hz, 1H), 7.46 (dd, *J* = 7.5, 2.0 Hz, 1H), 7.22 (d, *J* =7.4 Hz, 1H), 4.43 (hept, *J*=6.8 Hz, 1H), 3.78 (dd, *J* = 11.1, 6.9 Hz, 2H), 3.69 (s, 2H), 3.25 (dd, *J* = 11.1, 6.9 Hz, 2H), 3.14 (p, *J* =6.9 Hz, 1H), 1.36 (d, *J* =6.8 Hz, 6H). $^{13}$C NMR (125 MHz, DMSO-*d6*) δ 177.47 , 172.69 , 166.15 , 159.05 , 149.66 , 137.44 , 134.27 , 133.09 , 132.93 , 132.29 , 131.29 , 129.17 , 128.00 , 127.54 , 123.60 , 122.49 , 115.46 , 113.72 , 105.66 , 72.86 , 57.07 , 42.88 , 21.93. ESI-MS *m*/*z*:470.2 [M H].

**Example 57 Synthesis of 1-(5-(5-(3-cyano-4-isopropoxyphenyl)-1,2, 4-oxadiazol-3-yl) isoquinoline-8-yl) methyl) azelaic acid -3-carboxylic acid hydrochloric acid (A57)**

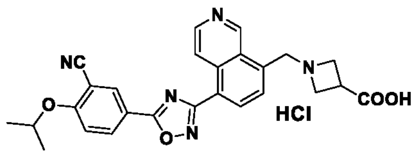

The synthesis method of compound**A57**was similar to that of compound**A1**.$^1$H NMR (500 MHz, Chloroform-*d*) δ 9.27 (s, 1H), 8.61 (d, *J* =7.5 Hz, 1H), 7.93 (d, *J* =7.5 Hz, 1H), 7.76 (d, *J* =7.5 Hz, 1H), 7.62 (d, *J* =2.1 Hz, 1H), 7.54 (d, *J* =7.5 Hz, 1H), 7.46 (dd, *J* = 7.5, 2.0 Hz, 1H), 7.22 (d, *J* =7.5 Hz, 1H), 4.42 (h, *J* =6.9 Hz, 1H), 3.86 - 3.76 (m, 2H), 3.23 - 3.10 (m, 3H), 2.95 (s, 2H), 1.36 (d, *J* =6.8 Hz, 6H). $^{13}$C NMR (125 MHz,DMSO-*d6*) δ 177.47 , 172.69 , 166.15 , 159.05 , 147.57 , 146.61 , 134.27 , 131.73 , 131.41 , 131.29 , 130.22 , 130.09 , 128.35 , 123.60 , 118.14 , 115.46, 113.72, 105.66, 72.86 , 57.90 , 42.88 , 42.82 , 21.93. ESI-MS *m*/*z*:470.2 [M H].

**Example 58 Synthesis of 1-(8-(5-(3-cyano-4-isopropoxyphenyl)-1,2, 4-oxadiazol-3-yl) quinoxaline-5-yl) methyl) azelaic acid -3-carboxylic acid hydrochloric acid (A58)**

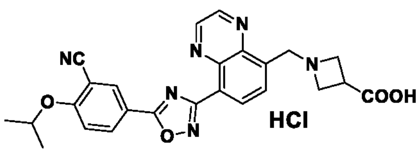

The synthesis method of compound**A58**was similar to that of compound**A1**.$^1$H NMR (500 MHz, Chloroform-*d*) δ 9.06 - 8.98 (m, 2H), 7.85 (d, *J*=7.5 Hz, 1H), 7.78 (d, *J*=7.5 Hz, 1H), 7.62 (d, *J* =2.0 Hz, 1H), 7.44 (dd, *J* = 7.5, 2.0 Hz, 1H), 7.16 (d, *J* =7.4 Hz, 1H), 4.43 (hept, *J* =6.8 Hz, 1H), 3.80 (dd, *J* = 10.8, 6.6 Hz, 2H), 3.69 (s, 2H), 3.26 - 3.12 (m, 3H), 1.36 (d, *J* =6.8 Hz, 6H). $^{13}$C NMR (125 MHz, DMSO-*d6*) δ 177.47 , 172.69 , 168.74 , 159.05 , 139.75 , 139.30, 137.09, 134.27 , 131.29 , 131.22 , 128.47 , 128.04 , 124.57 , 123.60 , 115.46 , 113.72 , 105.66 , 72.86 , 57.07 , 42.88 , 42.82 , 21.93. ESI-MS *m*/*z*:471.2 [M H]

**Example 59 Synthesis of 1-((4-(5-(thiophen-2-yl)-1,2, 4-oxadiazol-3-yl) naphthalen-1-yl) methyl) azelaic acid -3-carboxylic acid hydrochloric acid (A59)**

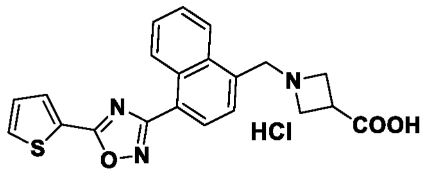

The synthesis method of compound**A59**was similar to that of compound**A1**.$^1$H NMR (500 MHz, Chloroform-*d*) δ 8.25 (dd, *J* = 7.3, 1.6 Hz, 1H), 7.97 (dd, *J* = 7.4, 1.6 Hz, 1H), 7.71 (dd, *J* = 7.5, 1.5 Hz, 1H), 7.64 (d, *J* =7.5 Hz, 1H), 7.57 - 7.41 (m, 4H), 7.26 (t, *J* =7.5 Hz, 1H), 3.87 - 3.76 (m, 2H), 3.39 (s, 2H), 3.23 - 3.10 (m, 3H). $^{13}$C NMR (125 MHz, DMSO-*d6*) δ 177.47 , 166.52 , 166.11, 133.15, 132.00, 131.57, 131.05, 129.62, 129.52, 128.95, 128.91 127.81 127.73, 127.68 , 127.03 , 125.31 , 124.41 , 57.90 , 42.88 , 42.82 . ESI-MS *m*/*z*:392.1 [M H].

**Example 60 1-(4-(5-(4-methylthiophen-2-yl)-1,2, 4-oxadiazol-3-yl) naphthalen-1-yl) methyl) azelaic acid-3-carboxylic acid hydrochloric acid synthesis (A60)**

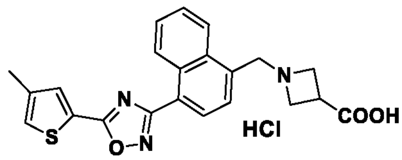

The synthesis method of compound**A60**was similar to that of compound**A1**.$^1$H NMR (500 MHz, Chloroform-*d*) δ 8.25 (dd, *J* = 7.4, 1.5 Hz, 1H), 7.98 (dd, *J* = 7.4, 1.6 Hz, 1H), 7.64 (d, *J* =7.5 Hz, 1H), 7.55 - 7.41 (m, 4H), 7.17 (d, *J* =1.5 Hz, 1H), 3.87 - 3.76 (m, 2H), 3.39 (s, 2H), 3.23 - 3.10 (m, 3H), 2.29 (s, 3H). $^{13}$C NMR (125 MHz, DMSO-*d6*) δ 177.47 , 166.52 , 166.13 , 138.12 , 133.54 , 132.00 , 131.05 , 129.79 , 129.62 , 129.52 , 128.91 , 127.81 , 127.68 , 127.03 , 125.31 , 124.41 , 115.40 , 57.90 , 42.88 , 42.82 , 15.39 . ESI-MS *m*/*z*:406.1 [M H].

**Example 61 Synthesis of 1-(4-(5-(5-methoxy-4-methylthiophen-2-yl)-1,2, 4-oxadiazol-3-yl) naphthalen-1-yl) methyl) azelaic acid -3-carboxylic acid hydrochloric acid (A61)**

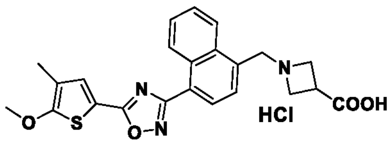

The synthesis method of compound**A61**was similar to that of compound **A1**.$^1$H NMR (500 MHz, Chloroform-*d*) δ 8.25 (dd, *J* = 7.4, 1.5 Hz, 1H), 7.95 (dd, *J* = 7.4, 1.6 Hz, 1H), 7.64 (d, *J* =7.5 Hz, 1H), 7.55 - 7.47 (m, 2H), 7.45 (td, *J* = 7.4, 1.6 Hz, 1H), 7.23 (s, 1H), 3.87 - 3.77 (m, 5H), 3.39 (s, 2H), 3.20 (d, *J* =6.9 Hz, 1H), 3.21 - 3.10 (m, 2H), 2.21 (s, 3H). $^{13}$C NMR (125 MHz, DMSO-*d6*) δ 177.47 , 166.52 , 166.20 , 164.76 , 136.28 , 132.00 , 131.05 , 129.62 , 129.52, 128.91 , 127.81 , 127.68 , 127.03 , 125.31 , 124.41 , 105.79 , 100.40 , 58.07 , 57.90 , 42.88 , 42.82 , 12.45 . ESI-MS *m*/*z*:436.1 [M H].

**Example 62 Synthesis of 1-(4-(5-(furan -2-yl)-1,2, 4-oxadiazol-3-yl) naphthalen-1-yl) methyl) azelaic acid -3-carboxylic acid hydrochloric acid (A62)**

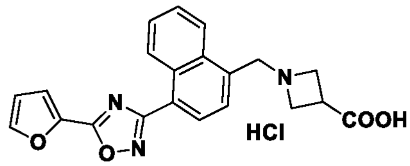

The synthesis method of compound**A62**was similar to that of compound**A1**.$^1$H NMR (500 MHz, Chloroform-*d*) δ 8.24 (dd, *J* = 7.3, 1.5 Hz, 1H), 7.98 (dd, *J* = 7.3, 1.6 Hz, 1H), 7.66 - 7.59 (m, 2H), 7.54 - 7.41 (m, 3H), 6.93 (dd, *J* = 7.5, 1.5 Hz, 1H), 6.54 (t, *J* =7.5 Hz, 1H), 3.87 - 3.76 (m, 2H), 3.39 (s, 2H), 3.23 - 3.10 (m, 3H). $^{13}$C NMR (125 MHz, DMSO-*d6*) δ 177.47 , 167.71 , 166.52 , 147.75 , 141.74 , 132.00 , 131.05 , 129.62 , 129.52 , 128.91 , 127.81 , 127.68 , 127.03 , 125.31, 124.41, 117.03 , 112.90 , 57.90 , 42.88 , 42.82 . ESI-MS *m*/*z*:376.1 [M H].

**Example 63 Synthesis of(4-(5-(4-(trifluoromethyl) furan-2-yl)-1,2, 4-oxadiazol-3-yl) naphthalen-1-yl) methyl) azelaic acid-3-carboxylic acid hydrochloric acid (A63)**

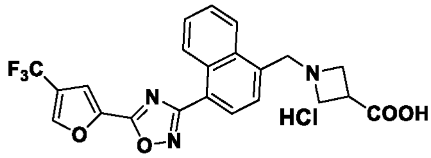

The synthesis method of compound**A63**was similar to that of compound**A1**.$^1$H NMR (500 MHz, Chloroform-*d*) δ 8.23 (dd, *J* = 7.3, 1.6 Hz, 1H), 7.94 (dd, *J* = 7.4, 1.5 Hz, 1H), 7.71 (d, *J* =1.5 Hz, 1H), 7.63 (d, *J* =7.4 Hz, 1H), 7.54 - 7.46 (m, 2H), 7.45 (td, *J* = 7.5, 1.6 Hz, 1H), 6.98 (d, *J* =1.5 Hz, 1H), 3.86 - 3.75 (m, 2H), 3.39 (s, 2H), 3.23 - 3.10 (m, 3H). $^{13}$C NMR (125 MHz, DMSO-*d6)* δ 177.47 , 167.83 , 166.52 , 140.16 , 140.14, 137.23 , 137.20 , 132.00 , 131.05 , 129.62 , 129.52 , 128.91 , 127.81 , 127.68 , 127.03 , 125.31 , 125.14 , 124.89 , 124.41 , 122.84 , 122.81 , 119.53 , 117.39 , 57.90 , 42.88 , 42.82 . ESI-MS *m*/*z*:444.1 [M H].

**Example 64 Synthesis of 1-(4-(5-(4-cyanofuran-2-yl)-1,2, 4-oxadiazol-3-yl) naphthalen-1-yl) methyl) azelaic acid-3-carboxylic acid hydrochloric acid (A64)**

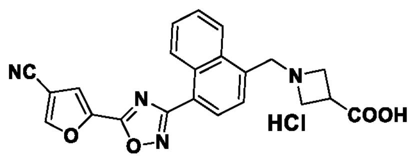

The synthesis method of compound **A64** was similar to that of compound **A1**.$^1$H NMR (500 MHz, Chloroform-*d*) δ 8.25 - 8.17 (m, 2H), 7.95 (dd, *J* = 7.4, 1.6 Hz, 1H), 7.63 (d, *J* =7.5 Hz, 1H), 7.51 (td, *J* = 7.5, 2.0 Hz, 2H), 7.45 (td, *J* = 7.4, 1.7 Hz, 1H), 7.07 (d, *J* =1.7 Hz, 1H), 3.87 - 3.75 (m, 2H), 3.39 (s, 2H), 3.23 - 3.10 (m, 3H). $^{13}$C NMR (125 MHz, DMSO-*d6*) δ 177.47 , 167.83 , 166.52 , 147.02 , 142.97 , 132.00 , 131.05 , 129.62 , 129.52 , 128.91 , 127.81 , 127.68 , 127.03 , 125.31 , 124.49 , 124.41 , 113.54 , 96.69 , 57.90 , 42.88 , 42.82 . ESI-MS *m*/*z*:401.1 [M H].

**Example 65 Synthesis of 1-(4-(5-(4-isopropoxyfuran-2-yl)-1,2, 4-oxadiazol-3-yl) naphthalen-1-yl) methyl) azelaic acid-3-carboxylic acid hydrochloric acid (A65)**

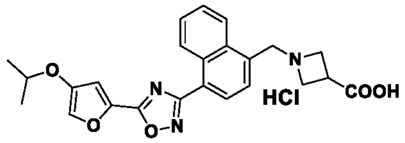

The synthesis method of compound **A65** was similar to that of compound **A1**.$^1$H NMR (500 MHz, Chloroform-*d*) δ 8.24 (dd, *J* = 7.4, 1.5 Hz, 1H), 7.92 (dd, *J* = 7.3, 1.6 Hz, 1H), 7.63 (d, *J* =7.5 Hz, 1H), 7.50 (td, *J* = 7.4, 1.5 Hz, 2H), 7.44 (td, *J* = 7.4, 1.7 Hz, 1H), 6.73 (d, *J* =1.5 Hz, 1H), 6.37 (d, *J*=1.5 Hz, 1H), 4.45 (hept, *J* =6.9 Hz, 1H), 3.86 - 3.74 (m, 2H), 3.39 (s, 2H), 3.23 - 3.10 (m, 3H), 1.34 (d, *J* =6.9 Hz, 6H). $^{13}$C NMR (125 MHz, DMSO-*d6*) δ 177.47 , 167.83 , 166.52 , 152.80 , 137.02 , 132.00 , 131.05 , 129.62 , 129.52 , 128.91 , 127.81 , 127.68 , 127.03 , 125.31 , 124.41 , 119.72, 112.38 , 70.83 , 57.90 , 42.88 , 42.82 , 22.03 . ESI-MS *m*/*z*:434.2 [M H].

**Example 66 Synthesis of 1-((4-(5-([1,1 '-biphenyl]-4-yl)-1,2, 4-oxadiazol-3-yl) naphthalen-1-yl) methyl) azelaic acid-3-carboxylic acid hydrochloric acid (A66)**

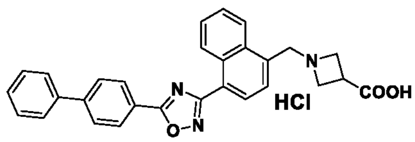

The synthesis method of compound **A66** was similar to that of compound **A1**.$^1$H NMR (500 MHz, Chloroform-*d*) δ 8.29 (dd, *J* = 7.4, 1.6 Hz, 1H), 7.98 (dd, *J* = 7.4, 1.6 Hz, 1H), 7.65 (d, *J* =7.5 Hz, 1H), 7.62 - 7.56 (m, 2H), 7.57 (s, 4H), 7.57 - 7.47 (m, 2H), 7.50 - 7.40 (m, 3H), 7.33 (ddt, *J* = 7.4, 5.9, 2.0 Hz, 1H), 3.86 - 3.75 (m, 2H), 3.39 (s, 2H), 3.23 - 3.10 (m, 3H).$^{13}$C NMR (125 MHz, DMSO-*d6*) δ 177.47 , 173.12, 166.15 , 144.42 , 139.22 , 132.00 , 131.05 , 129.62 , 129.52 , 128.91 , 128.86 , 127.81 , 127.71 , 127.68 , 127.03 , 126.99 , 126.35 , 125.67 , 125.31 , 124.41 , 57.90 , 42.88 , 42.82 . ESI-MS *m*/*z*:462.2 [M H].

**Example 67 Synthesis of 1-(4-(5-(4-(thiophen-2-yl) phenyl)-1,2, 4-oxadiazol-3-yl) naphthalen-1-yl) methyl) azelaic acid-3-carboxylic acid hydrochloric acid (A67)**

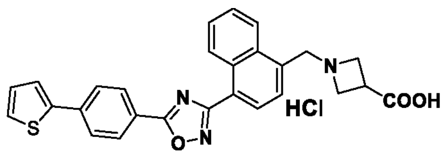

The synthesis method of compound **A67** was similar to that of compound **A1**.$^1$H NMR (500 MHz, Chloroform-*d*) δ 8.29 (dd, *J* = 7.5, 1.5 Hz, 1H), 7.97 (dd, *J* = 7.4, 1.5 Hz, 1H), 7.66 (dd, *J* = 7.5, 2.6 Hz, 3H), 7.61 - 7.54 (m, 2H), 7.53 (td, *J* = 7.5, 1.6 Hz, 3H), 7.46 (td, *J* = 7.4, 1.5 Hz, 1H), 7.36 (dd, *J* = 7.5, 1.7 Hz, 1H), 7.17 (t, *J* =7.5 Hz, 1H), 3.93 (dd, *J* = 10.9, 6.6 Hz, 2H), 3.39 (s, 2H), 3.44 - 3.30 (m, 3H). $^{13}$C NMR (125 MHz, DMSO-*d6*) δ 177.47 , 173.12, 166.15 , 143.09 , 137.84 , 132.00 , 131.05 , 129.62 , 129.52 , 128.91 , 127.81 , 127.68 , 127.03 , 126.75 , 126.64 , 125.91 , 125.42 , 125.31 , 124.62 , 124.41 , 57.90 , 42.88 . ESI-MS *m*/*z*:468.1 [M H].

**Example 68 Synthesis of 1-(4-(5-(4-(1H-pyrrole-2-yl) phenyl)-1,2, 4-oxadiazol-3-yl) naphthalen-1-yl) methyl) azelaic acid-3-carboxylic acid hydrochloric acid (A68)**

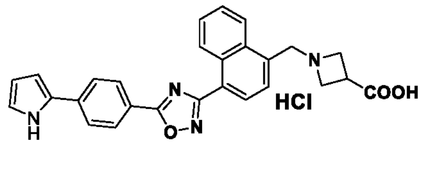

The synthesis method of compound **A68** was similar to that of compound **A1**.$^1$H NMR (500 MHz, Chloroform-*d*) δ 8.64 (s, 1H), 8.29 (dd, *J* = 7.4, 1.5 Hz, 1H), 8.00 (dd, *J* = 7.4, 1.6 Hz, 1H), 7.73 - 7.67 (m, 2H), 7.65 (d, *J* =7.5 Hz, 1H), 7.60 - 7.54 (m, 2H), 7.56 - 7.43 (m, 3H), 6.90 (dd, *J* = 7.4, 1.6 Hz, 1H), 6.55 (dd, *J* = 7.4, 1.6 Hz, 1H), 6.33 (t, *J* =7.5 Hz, 1H), 3.89 - 3.78 (m, 2H), 3.39 (s, 2H), 3.24 - 3.12 (m, 3H). $^{13}$C NMR (125 MHz, DMSO-*d6*) δ 177.47 , 173.12, 166.15 , 132.92 , 132.00 , 130.34 , 129.62 , 129.52 , 128.91 , 127.81 , 127.68 , 127.03 , 126.97 , 125.83 , 125.31 , 124.41 , 124.18 , 120.05 , 111.35 , 107.64 , 57.90 , 42.88 , 42.82 . ESI-MS *m*/*z*:451.2 [M H].

**Example 69 Synthesis of 1-(4-(5-(4-(furan-2-yl) phenyl)-1,2, 4-oxadiazol-3-yl) naphthalen-1-yl) methyl) azelaic acid-3-carboxylic acid hydrochloric acid (A69)**

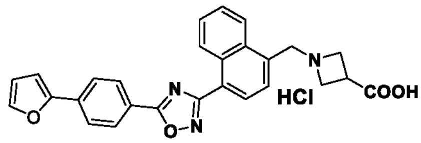

The synthesis method of compound **A69** was similar to that of compound **A1**.$^1$H NMR (500 MHz, Chloroform-*d*) δ 8.29 (dd, *J* = 7.4, 1.5 Hz, 1H), 7.98 (dd, *J* = 7.4, 1.7 Hz, 1H), 7.77 (dd, *J* = 7.5, 1.5 Hz, 1H), 7.66 (dd, *J* = 8.8, 7.3 Hz, 3H), 7.61 - 7.55 (m, 2H), 7.56 - 7.49 (m, 2H), 7.46 (td, *J* = 7.4, 1.6 Hz, 1H), 6.89 (dd, *J* = 7.5, 1.5 Hz, 1H), 6.61 (t, *J* =7.5 Hz, 1H), 3.92 (dd, *J* = 11.0, 6.8 Hz, 2H), 3.39 (s, 2H), 3.38 (dd, *J* = 11.1, 6.9 Hz, 2H), 3.31 (p, *J* =6.8 Hz, 1H). $^{13}$C NMR (125 MHz, DMSO-*d6*) δ 177.47 , 173.12, 166.15 , 152.87 , 142.41 , 134.11 , 132.00 , 131.05 , 129.62 , 129.52 , 128.91 , 127.81 , 127.68 , 127.03 , 126.79 , 125.37 , 125.31 , 125.23 , 124.41 , 109.76 , 107.76 , 57.90 , 42.88 . ESI-MS *m*/*z*:452.2 $[M+H]^+$.

**Example 70 Synthesis of 1-(4-(5-(3-cyano-4-(cyclopentoxy) phenyl)-1,2, 4-oxadiazol-3-yl) naphthalen-1-yl) methyl) azelaic acid-3-carboxylic acid hydrochloric acid (A70)**

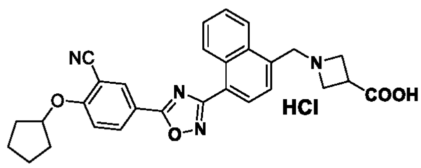

The synthesis method of compound **A70** was similar to that of compound **A1**.$^1$H NMR (500 MHz, Chloroform-*d*) δ 8.26 (dd, *J* = 7.5, 1.6 Hz, 1H), 8.00 (dd, *J* = 7.4, 1.6 Hz, 1H), 7.71 (d, *J* =7.5 Hz, 1H), 7.64 (d, *J* =7.5 Hz, 1H), 7.53 (td, *J* = 7.4, 1.5 Hz, 1H), 7.52 - 7.43 (m, 2H), 7.22 (d, *J* =2.1 Hz, 1H), 7.08 (dd, *J* = 7.5, 2.0 Hz, 1H), 4.70 - 4.61 (m, 1H), 3.87 - 3.78 (m, 2H), 3.39 (s, 2H), 3.23 - 3.10 (m, 3H), 1.96 - 1.79 (m, 6H), 1.60 (tdd, *J* = 11.0, 5.2, 1.9 Hz, 2H). $^{13}$C NMR (125 MHz, DMSO-*d6*) δ 177.47 , 172.69 , 166.15 , 159.08 , 134.08 , 132.00 , 131.11 , 131.05 , 129.62 , 129.52 , 128.91 , 127.81 , 127.68 , 127.03 , 125.31 , 124.41 , 123.28 , 115.46 , 114.09 , 105.53 , 81.35 , 57.90 , 42.88 , 32.83 , 23.82 . ESI-MS *m*/*z*:495.2 $[M+H]^+$.

**Example of pharmacological activity experiments**

**Experimental Example 1: Compound agonistic activity test on S1P receptors**

**Instrument:**FLIPR™ TETRA (Molecular Device)

**Experimental materials:** CHO-K1/Gα15/EDG1 cells constructed by GenScript autonomously.
**Sample processing:** Before the experiment, the working solution with the corresponding concentration was prepared by diluting with GPCR dilution buffer.
**Experimental method:** CHO-K1/Gα15/EDG1 cells stably expressing EDG1 receptor were cultured in a 10cm culture dish in a 37°C/5% $CO_2$incubator, and digested when the confluence rate reached 80%-85%. The cells in collected cell suspension were inoculated into 384 microtiter plates at appropriate density, and then placed in a 37°C/5% $CO_2$ incubator and continue to incubate for at least 18 hours before using for experiments. After 18-20 hours, the cell culture plat was taken out and added with dye working solution, and then the plate was placed in a 37°C/5% $CO_2$ incubator for 1 hour, and finally equilibrated at room temperature for 15 minutes.Positive agonist working solution was added, and RFU values were detected and recorded.The 384 microplates, cell plates and tip box with positive agonist working solution were placed into FLIPRTM TETRA and the agonist mode assay program was run with an overall assay time of 120 seconds. The positive agonist working fluid was added to the cell culture plate at the 21st second. 384 microplate, cell plate and gun head box containing the positive antagonist working fluid were put into the FLIPRTM TETRA. The antagonistic mode detection program, the overall detection time of the instrument was 120 seconds, and positive antagonist working solution was added to the cell culture plate at the 21st second.
**Data processing:**Raw data were obtained via ScreenWorks (version 3.1) and saved as *FMD files on the Kingsray computer network system.Data were collected and analyzed using Excel and GraphPad Prism 6 software programs.For each well, the average fluorescence intensity value from 1 to 20 seconds was used as the baseline, and the maximum fluorescence intensity value from 21 to 120 seconds minus the minimum fluorescence intensity value from 21 to 120 seconds was the relative fluorescence intensity value (ΔRFU), from which the percentage activation or inhibition can be calculated based on the following equation.

$$\%\text{Activation Rate} = (\Delta\text{RFUCompound} - \Delta\text{RFUBackground}) / (\Delta\text{RFUAgonist control} - \Delta\text{RFUBackground}) \times 100\%$$

The data was analyzed using GraphPad Prism 6 with a four-parameter equation to calculate the $EC_{50}$ and $IC_{50}$ values.The four-parameter equation was as follows: y = Bottom + (Top-Bottom) / (1+10^((LogEC50/IC50-X) * HillSlope)), wherein X is the Log value of the concentration and Y was the inhibition rate.

**Experimental results:**

| Compound | $S1PR_1$ $EC_{50}$ | $S1PR_3$ $EC_{50}$ |
|---|---|---|
| Siponmod | 547.0 nM | > 10 μM |
| Ozanimod | 222.0 nM | > 10 μM |
| FTY720-P | 39.3 nM | 4.52μM |
| A1 | 48.0 nM | > 10 μM |
| A10 | 698.9 nM | > 10 μM |
| A11 | 1366.0 nM | > 10 μM |
| A12 | 358.0 nM | > 10 μM |
| A13 | 1549.0 nM | > 10 μM |
| A14 | 1668.0 nM | > 10 μM |
| A15 | 1231.0 nM | > 10 μM |

(continued)

| Compound | S1PR$_1$ EC$_{50}$ | S1PR$_3$ EC$_{50}$ |
|---|---|---|
| A16 | 1169.0 nM | > 10 μM |
| A17 | > 10 μM | > 10 μM |
| A18 | > 10 μM | > 10 μM |
| A19 | 1125.0 nM | > 10 μM |
| A20 | 1049.0 nM | > 10 μM |
| A21 | 1273.0 nM | > 10 μM |
| A22 | 1127.0 nM | > 10 μM |
| A23 | 1960.0 nM | > 10 μM |
| A24 | 684.8 nM | > 10 μM |
| A25 | 1057.0 nM | > 10 μM |
| A26 | 749.2 nM | > 10 μM |
| A27 | > 10 μM | > 10 μM |
| A28 | 1182.0 nM | > 10 μM |
| A29 | 992.5 nM | > 10 μM |
| A30 | 122.9 nM | > 10 μM |
| A31 | 1300.0 nM | > 10 μM |
| A32 | 617.0 nM | > 10 μM |
| A33 | 932.6 nM | > 10 μM |
| A34 | 739.4 nM | > 10 μM |
| A35 | 1960.0 nM | > 10 μM |
| A36 | 1108.0 nM | > 10 μM |
| A37 | > 10 μM | > 10 μM |
| A38 | 1425.0 nM | > 10 μM |
| A39 | 830.6 nM | > 10 μM |

**Experimental Example 2:Subtype Selectivity Study of Compound A1**

The S1P receptor selectivity was tested for different subtypes using the method described in Experimental Example 1.The results showed that Compound A1 has good selectivity for different subtypes, especially for S1P1 and S1P5 subtypes, as shown in the table below.

| Compound | S1P$_1$ EC$_{50}$ (nM) | S1P$_2$ EC$_{50}$ (nM) | S1P$_3$ EC$_{50}$ (nM) | S1P$_4$ EC$_{50}$ (nM) | S1P$_5$ EC$_{50}$ (nM) |
|---|---|---|---|---|---|
| A1 | 48 | >10000 | >10000 | >10000 | 120 |
| Siponmod | 547 | > 10000 | >1000 | 750 | 0.98 |
| Ozanimod | 222 | > 10000 | 2618 | > 10000 | 10.66 |

**Experimental Example 3:Preliminary Assessment of Pharmacokinetics of Compound A1 in Mice**

Dosing regimen

9 CD-1 mice, male, weighing 20-26g, were randomly divided into 3 groups with 3 mice in each group.The test compounds were administered intragastric, intravenously or intraperitoneally according to the following protocol.

The mice were fasted for 12 h before the test and drank water freely.Food was consumed uniformly 2 h after administration.

Three animals were used at each time point, and the grouping and blood collection time points were shown in the table below.

| Group | Drug name | Mode of administration | Dosage (mg/Kg) | Administration volume (mL/Kg) | Sampling time (h) |
|---|---|---|---|---|---|
| 1 | A1 | intragastric | 20 | 10 | 0.25, 0.5,1, 2.4, 8, 24h |
| 2 | | intravenous | 2 | 5 | 0.05,0.25,0.75, 2.4, 8, 24h |
| 3 | | intraperitoneal | 20 | 5 | 0.05,0.25,0.75, 2.4, 8, 24h |

The intragastric administration solution was prepared with DMSO/0.5% HPMC(5/95, v/v) to the final concentration.Intravenous and intraperitoneal administration solution was formulated with DMSO/PEG300/EtOH/NaCl(5/40/5/50, v/v/v).The sample of the administration solution was left (50 μL of the solution was mixed with 50 μL of DMSO before and after the administration, respectively) for test.

The drug was given according to the above dose, the time of administration was recorded, and 20 μL of blood was taken from the femoral plexus of mice at the above set time points and placed in heparinized tubes.The blood was immediately centrifuged at 11000 rpm for 5 min. 10 μL of plasma was immediately and accurately sucked into a centrifuge tube prefilled with 100 μL of PK-IS solution (prepared with methanol: acetonitrile (1:1,v/v)), well mixed, and freezed at -20°C for test.

We performed pharmacokinetic studies on compound A1.Compound A1 was administered orally at 5071 h*ng/mL, intravenously at 806 h*ng/mL, and intraperitoneally at 7975 h*ng/mL.The main pharmacokinetics data were shown in the table below. Concentration of A1 drug in plasma of mice after intraperitoneal administration of 20 mg/kg A1 (ng/mL)

| Time(h) | 1 | 2 | 3 | Mean | SD |
|---|---|---|---|---|---|
| 0.25 | 1095 | 1070 | 3041 | 1735 | 1131 |
| 0.50 | 1574 | 1388 | 3514 | 2159 | 1177 |
| 1.00 | 1914 | 1843 | 4055 | 2604 | 1257 |
| 2.00 | 571 | 1318 | 1662 | 1184 | 558 |
| 4.00 | 146 | 215 | 65.0 | 142 | 75 |
| 8.00 | 25.8 | 36.4 | 11.5 | 24.6 | 12.5 |
| 24.0 | 4.80 | 3.80 | 3.80 | 4.13 | 0.58 |
| BLQ:Below LLOQ(LLOQ=0.3 ng/mL) | | | | | |

Concentration of A1 drug in plasma of mice after oral administration of 2 mg/kg A1 (ng/mL)

| Time(h) | 4 | 5 | 6 | Mean | SD |
|---|---|---|---|---|---|
| 0.05 | 1786 | 1521 | 2513 | 1940 | 514 |
| 0.25 | 1041 | 572 | 1021 | 878 | 265 |
| 0.75 | 178 | 113 | 216 | 169 | 52 |
| 2.00 | 49.2 | 39.5 | 60.6 | 49.8 | 10.6 |
| 4.00 | 8.60 | 11.5 | 5.20 | 8.43 | 3.15 |
| 8.00 | 8.70 | 2.10 | 1.10 | 3.97 | 4.13 |
| 24.0 | 0.500 | 0.800 | 0.500 | 0.600 | 0.173 |
| BLQ:Below LLOQ(LLOQ=0.3 ng/mL) | | | | | |

The concentration of A1 drug in plasma of mice (ng/mL) after intraperitoneal injection of 20 mg/kg A 1

| Time(h) | 4 | 5 | 6 | Mean | SD |
|---|---|---|---|---|---|
| 0.05 | 1105 | 473 | 715 | 764 | 318 |
| 0.25 | 1168 | 827 | 1507 | 1168 | 340 |
| 0.75 | 1206 | 1534 | 3318 | 2019 | 1136 |
| 2.00 | 516 | 1443 | 4405 | 2122 | 2031 |
| 4.00 | 376 | 201 | 535 | 370 | 167 |
| 8.00 | 273 | 78.2 | 119 | 157 | 102 |
| 24.0 | 86.9 | 26.5 | 35.0 | 49.5 | 32.7 |
| BLQ:Below LLOQ (LLOQ=0.3 ng/mL) | | | | | |

**Experimental Example 4: Preliminary Assessment of the In Vivo Pharmacokinetics of Compound A1 (DC411151) in Rats**

Experimental Design-Rats

| Group | Animal number | Administration compound | Route of administration | Administration dose (mg/kg) | dosing volume (mL/kg) |
|---|---|---|---|---|---|
| 1 | 1-3 | DC411151 | intravenous | 2 | 5 |
| 2 | 4-6 | DC411151 | intragastric | 20 | 10 |

The mice were fasted for 12 h before the test and drank water freely.Food was consumed uniformly 4 h after administration.

**Drug preparation:**

The drug was first dissolved with DMSO and Tween80, then saline was added to a final concentration of 1% DMSO, 2% Tween, 97% saline.

The drug for intravenous administration was prepared with DMSO/HS15/saline (10/10/80, v/v/v).

Sample collection:Rat

Before and 0.25, 0.5, 1.0, 2.0, 4.0, 6.0, 8.0, 24 after administration (5 min sampling point was added in intravenous administration group); 0.2 ml of blood was taken from Posterior ocular venous plexus at the above set time point, placed in EDTA-$K_2$ test tube, centrifuged at 11000 rpm for 5 min, separated plasma, and frozen in -20°C refrigerator.

Sample test:The concentration of DC411151 in plasma was determined by LC-MS/MS method.

Data processing: the pharmacokinetic parameters after drug administration were calculated using a non-atrial model with WinNonlin 7.0 software (Pharsight, USA).

The peak time $T_{max}$ and the peak concentration $C_{max}$ were measured;

Area under the drug-time curve $AUC_{0-t}$ values: calculated using the trapezoidal method; $AUC_{0-\infty} = AUC_{0-t} + C_t/k_e$, Ct being the blood concentration at the last measurable time point and $k_e$ being the elimination rate constant.

$$\text{Elimination half-life } t_{1/2}=0.693 / k_e;$$

$$\text{Mean residence time MRT} = \text{AUMC} / \text{AUC}.$$

$$\text{Clearance rate CL} = D/AUC_{0-\infty};$$

$$\text{Steady-state distribution volume } V_{ss} = CL \times MRT$$

$$\text{Absolute bioavailability } F = (AUC_{intragastric} \times D_{intravenous}) / (AUC_{intravenous} \times D_{gintragastric}) \times 100\%$$

Test results

Pharmacokinetic results in rats

The drug plasma concentrations in rat after a single intravenous injection of 2 mg/kg DC411151 are shown in Table 1, the blood concentration-time curve was shown inFigure 1, and the corresponding pharmacokinetic parameters were shown inTable 2.

After intravenous administration, the plasma clearance rate of DC411151 in rats was 8.66 mL/min/kg, equivalent to 15.7% of the rat liver blood flow (about 55 mL/min/kg), which was a low clearance drug; the steady-state apparent distribution volume Vss was 0.758 L/kg, which was comparable to the total body fluid of rats (about 0.67 L/kg); the mean plasma elimination half-life $t_{1/2}$ was 3.04 h.

The drug plasma concentration was shown in Table 3, the blood concentration-time curve was shown in Figure 2, and the corresponding pharmacokinetic parameters were shown in Table 4 after the intragastric administrationof 20 mg/kg DC411151 in rat.

After the rats were given 20 mg/kg DC411151 by gavage, the peak was reached rapidly with the peak time $T_{max}$ of 0.5-1 h, the peak concentration $C_{max}$ of 2760 ng/mL, and the area $AUC_{0-t}$ under the drug concentration-time curve was 8780 ng-h/mL, calculated as the mean value of $AUC_{0-t}$ after dose correction. The absolute bioavailability of 20 mg/kg of DC411151 given by gavage to rats was 22.8%.

Table 1 Plasma drug concentrations in rats after intravenous administration of 2 mg/kg of DC411151

| | ID | | | | |
|---|---|---|---|---|---|
| | 1 | 2 | 3 | Mean | SD |
| Time (h) | Conc (ng/ml) | | | | |
| 0.083 | 3940 | 3720 | 3390 | 3680 | 277 |
| 0.25 | 2950 | 2650 | 2700 | 2770 | 161 |
| 0.5 | 1570 | 1710 | 1890 | 1720 | 160 |
| 1 | 900 | 1110 | 951 | 987 | 110 |
| 2 | 419 | 563 | 528 | 503 | 75.1 |
| 4 | 128 | 138 | 172 | 146 | 23.1 |
| 6 | 45.8 | 51.4 | 47 | 48.1 | 2.95 |
| 8 | 11.8 | 13.6 | 16.9 | 14.1 | 2.59 |
| 24 | BQL | 1.16 | 1.94 | 1.55 | 0.552 |

Table 2 Pharmacokinetic parameters in rats after intravenous administration of 2 mg/kg of DC411151

| **ID** | $AUC_{0-t}$ (ng·h/mL) | $AUC_{0-\infty}$ (ng·h/mL) | $MRT_{0-\infty}$ (h) | $t_{1/2}$ (h) | CL (mL/min/kg) | $V_{ss}$ (L/kg) |
|---|---|---|---|---|---|---|
| 1 | 3550 | 3570 | 1.2 | 1.15 | 9.34 | 0.672 |
| 2 | 4030 | 4040 | 1.52 | 3.67 | 8.26 | 0.752 |
| 3 | 3960 | 3980 | 1.69 | 4.3 | 8.38 | 0.85 |
| Mean | 3850 | 3860 | 1.47 | 3.04 | 8.66 | 0.758 |
| SD | 261 | 255 | 0.249 | 1.67 | 0.593 | 0.0892 |
| CV% | 6.78 | 6.6 | 17 | 54.8 | 6.84 | 11.8 |

Table 3 Plasma drug concentrations in rats after intragastric administration of 20 mg/kg of DC411151

| | | ID | | | | |
|---|---|---|---|---|---|---|
| | | 4 | 5 | 6 | Mean | SD |
| Route | Time (h) | conc (ng/ml) | | | | |
| PO-20 mg/kg | 0.25 | 1960 | 696 | 1150 | 1270 | 640 |
| | 0.5 | 3130 | 1570 | 2600 | 2430 | 793 |
| | 1 | 2560 | 2070 | 3070 | 2570 | 500 |
| | 2 | 2290 | 1220 | 2150 | 1890 | 582 |
| | 4 | 884 | 635 | 841 | 787 | 133 |
| | 6 | 322 | 350 | 289 | 320 | 30.5 |
| | 8 | 65.1 | 64.0 | 57.0 | 62.0 | 4.39 |
| | 24 | 4.33 | 2.49 | 2.92 | 3.25 | 0.963 |

Table 4 Pharmacokinetic parameters of rats after intragastric administration of 20 mg/kg of DC411151

| **Route** | **ID** | $T_{max}$ (h) | $C_{max}$ (ng/mL) | $AUC_{0-t}$ (ng·h/mL) | $AUC_{0-\infty}$ (ng·h/mL) | $MRT_{0-\infty}$ (h) | $t_{1/2}$ (h) |
|---|---|---|---|---|---|---|---|
| PO-20 mg/kg | 4 | 0.5 | 3130 | 10100 | 10100 | 2.62 | 2.5 |
| | 5 | 1 | 2070 | 6710 | 6720 | 3.00 | 2.51 |
| | 6 | 1 | 3070 | 9590 | 9600 | 2.55 | 2.45 |
| | Mean | 0.833 | 2760 | 8780 | 8790 | 2.72 | 2.49 |
| | SD | 0.289 | 595 | 1810 | 1810 | 0.243 | 0.0325 |
| | CV% | 34.6 | 21.6 | 20.6 | 20.6 | 8.92 | 1.31 |

**Experimental Example 5:Preliminary Evaluation of Pharmacokinetics of Compound A1 in Beagle Dogs**

Test Design-Beagle

| Group | Animal number | Administration compound | Route of administration | Administration dose (mg/kg) | dosing volume (ml/kg) |
|---|---|---|---|---|---|
| 1 | 1 to 3 | DC411151 | intravenous | 0.5 | 1 |
| 2 | 4 to 6 | DC411151 | intragastric | 3 | 2 |

The mice were fasted for 12 h before the test and drank water freely.Food was consumed uniformly 4 h after administration.

Drug preparation:The drug was first dissolved with DMSO and Tween80, then saline was added to a final concentration of 1% DMSO, 2% Tween, and 97% saline.

The drug for intravenous administration was prepared with DMSO/HS 15/saline (10/10/80, v/v/v).

Sample collection:Administration before and 0.25, 0.5, 1.0, 2.0, 3.0, 4.0, 6.0, 8.0, 24 h after administration (5 min sampling point was added in intravenous and subcutaneous administration group); at the above set time point, 1 ml of blood was taken from vein, placed in EDTA-$K_2$ test tube, centrifuged at 3500 rpm for 10 min, separated plasma, and frozen in - 20°C refrigerator.

Sample test:The concentration of DC411151 in plasma was determined by LC-MS/MS method.

Data processing: the pharmacokinetic parameters after drug administration were calculated using a non-atrial model with WinNonlin 7.0 software (Pharsight, USA).

The peak time $T_{max}$ and the peak concentration $C_{max}$ were measured;
Area under the drug-time curve $AUC_{0-t}$ values: calculated using the trapezoidal method; $AUCO_{0-\infty} = AUC_{0-t} + C_t/k_e$, Ct being the blood concentration at the last measurable time point and $k_e$ being the elimination rate constant.

$$\text{Elimination half-life } t_{1/2}=0.693 / k_e;$$

$$\text{Mean residence time MRT} = \text{AUMC} / \text{AUC}.$$

$$\text{Clearance rate CL} = D/AUC_{0-\infty};$$

$$\text{Steady-state distribution volume } V_{ss} = CL \times MRT$$

$$\text{Absolute bioavailability } F = (AUC_{intragastric} \times D_{intravenous})/ (AUC_{intravenous} \times D_{gintragastric}) \times 100\%$$

Test results

The drug plasma concentration after a single intravenous injection of 0.5 mg/kg DC411151 in Beagle dogs was shown in Table 5, the blood concentration-time curve was shown in Figure 3, and the corresponding pharmacokinetic parameters were shown in Table 6.

After intravenous administration of Beagle dogs, the plasma clearance rate CL of DC411151 in dogs was 23.3 mL/min/kg, which was equivalent to 75.2% of the blood flow of dog liver (about 31 mL/min/kg), and the stable apparent distribution volume $V_{ss}$ was 1.28 L/kg, which was higher than the total body fluid volume of dogs (0.6 L/kg); the elimination half-life $t_{1/2}$ was 0.984 h.

The drug plasma concentrations after intragastric administration of 3 mg/kg DC411151 in Beagle dogs were shown in Table 7, the blood concentration-time curve was shown in Figure 4, and the corresponding pharmacokinetic parameters were shown in Table 8.

After the beagle dogs were given 3 mg/kg DC411151 by gavage, the peak time $T_{max}$ was 0.25-2 h, the peak concentration $C_{max}$ was 366 ng/mL, and the area $AUC_{0-t}$ under the drug concentration-time curve was1160 ng h/mL, calculated as the mean value of $AUC_{0-t}$ after dose correction. The absolute bioavailability of 3 mg/kg DC411151 was 54.0%.

Table 5 Plasma drug concentration in Beagle dogs after intravenous administration of 0.5 mg/kg of DC411151

| | ID | | | | |
|---|---|---|---|---|---|
| | 1 | 2 | 3 | Mean | SD |
| Time (h) | conc (ng/ml) | | | | |
| 0.083 | 604 | 554 | 537 | 565 | 34.8 |
| 0.25 | 237 | 219 | 340 | 265 | 65.3 |
| 0.5 | 190 | 138 | 176 | 168 | 26.9 |
| 1 | 98.8 | 76.9 | 97.1 | 90.9 | 12.2 |
| 2 | 27.5 | 27.8 | 35.4 | 30.2 | 4.48 |
| 3 | 20.4 | 12.5 | 20.3 | 17.7 | 4.53 |
| 4 | 9.68 | 7.88 | 10.6 | 9.39 | 1.38 |
| 6 | 2.51 | 1.64 | 2.47 | 2.21 | 0.491 |
| 8 | BQL | 1.05 | 0.496 | 0.773 | 0.392 |

Table 6 Pharmacokinetic parameters after intravenous administration of 0.5 mg/kg of DC411151 in Beagle dogs

| ID | $AUC_{0-t}$ (ng·h/mL) | $AUC_{0-\infty}$ (ng·h/mL) | $MRT_{0-\infty}$ (h) | $t_{1/2}$ (h) | CL (mL/min/kg) | $V_{ss}$ (L/kg) |
|---|---|---|---|---|---|---|
| 1 | 375 | 379 | 0.910 | 0.997 | 22.0 | 1.20 |
| 2 | 317 | 319 | 0.892 | 1.05 | 26.1 | 1.40 |
| 3 | 382 | 382 | 0.952 | 0.905 | 21.8 | 1.24 |
| Mean | 358 | 360 | 0.918 | 0.984 | 23.3 | 1.28 |
| SD | 35.5 | 35.7 | 0.0309 | 0.0728 | 2.45 | 0.104 |
| CV% | 9.92 | 9.92 | 3.36 | 7.39 | 10.5 | 8.09 |

Table 7 Plasma drug concentration after intragastric administration of 3 mg/kg DC411151 in Beagle dogs

| | | ID | | | | |
|---|---|---|---|---|---|---|
| | | 4 | 5 | 6 | Mean | SD |
| Route | Time (h) | conc (ng/ml) | | | | |
| PO-3 mg/kg | 0.25 | 539 | 93.3 | 11.2 | 215 | 284 |
| | 0.5 | 444 | 153 | 188 | 262 | 159 |
| | 1 | 333 | 193 | 318 | 281 | 76.9 |
| | 2 | 365 | 242 | 266 | 291 | 65.2 |
| | 3 | 212 | 143 | 269 | 208 | 63.1 |
| | 4 | 151 | 62.7 | 118 | 111 | 44.6 |
| | 6 | 90.3 | 24.0 | 64.0 | 59.4 | 33.4 |
| | 8 | 12.0 | 4.87 | 15.6 | 10.8 | 5.46 |
| | 24 | BQL | BQL | BQL | NA | NA |

Table 8 Pharmacokinetic parameters after intragastric administration of 3 mg/kg DC411151 in Beagle dogs

| Route | ID | $T_{max}$ (h) | $C_{max}$ (ng/mL) | $AUC_{0-t}$ (ng·h/mL) | $AUC_{0-\infty}$ (ng·h/mL) | $MRT_{0-\infty}$ (h) | $t_{1/2}$ (h) |
|---|---|---|---|---|---|---|---|
| PO-3 mg/kg | 4 | 0.25 | 539 | 1550 | 1570 | 2.58 | 1.32 |
| | 5 | 2 | 242 | 757 | 765 | 2.44 | 1.06 |
| | 6 | 1 | 318 | 1170 | 1200 | 2.93 | 1.3 |
| | Mean | 1.08 | 366 | 1160 | 1180 | 2.65 | 1.23 |
| | SD | 0.878 | 154 | 395 | 403 | 0.251 | 0.142 |
| | CV% | 81 | 42.1 | 34.1 | 34.2 | 9.48 | 11.6 |

**Experimental Example 6 In vivo anti-obesity, insulin resistance, NASH pharmacological activity test**

In this experiment, we investigated the effects of long-term oral administration of compound A1 on obesity, insulin resistance, hepatic lipid accumulation, inflammation and fibrosis in mice with a triple phenotype of obesity, insulin resistance and NASH induced by high fat, high cholesterol and high fructose (Gubra amylin, GAN).

Animal experiment: Male C57BL/6 mice were fed with GAN diet to induce the NASH model, and 12 weeks after modeling, the mice were randomly divided into 3 groups: low-fat diet control group (LFD, n = 6), model control group (Model, n = 10), and A1 group (10 mg/kg, n = 10), and administered by gavage at a dose of 10 mg/kg and a volume of 10 mL /kg, once a day.During the period of administration, the food intake and body weight of animals were monitored; after 10 weeks of administration, glucose tolerance test (GTT) and insulin tolerance test (ITT) were performed; after 11

weeks of administration, fasting glucose and insulin were measured; after 12 weeks of administration, core body temperature and energy metabolism were measured; after 15 weeks of administration, body composition of mice was measured, and mice were sacrificed by dislocation after blood sampling via the posterior ocular venous plexus, and liver, subcutaneous fat, epididymal fat and brown fat were weighed, part the liver was fixed in 4% paraformaldehyde, and all tissues and serum were stored at -80°C.This experiment evaluated whether the compounds have the effect of weight loss by detecting energy metabolism, body composition, and fat weight of mice; whether the compounds have the effect of improving insulin resistance by GTT, ITT, detecting random blood glucose, fasting blood glucose and fasting insulin, and calculating HOMA-IR index; whether the compounds have the effect of improving insulin resistance by detecting serum liver function indexes ALT, AST, triglyceride, total cholesterol (T- CHO), LDL-C, HDL-C levels in serum, triglyceride, T-CHO, and hydroxyproline (characteristic amino acids of collagen) levels in liver, and by measuring mRNA expression levels of fatty acid ab initio synthesis and transport related genes (*Srebp1c, Scd1, Fasn, Acaca, Cd36*),β-oxidative metabolic pathways (*Ppara*,*Cyp4**A1***,*Cpt1b*,*Acot1*,*Acox1*), inflammation related genes (*Tnfa, Il1b, Casp1, Nlrp3, Pycard,Panx1, Ccl2, Ccl3*) and fibrosis-related genes (*Asma, Tgfb, Col1**A1**, Col3**A1**, Col4**A1**, Col4a2, Col5a2*) and liver pathological changes (H&E staining, Oil Red O staining and Sirius Scarlet staining) and other indicators to evaluate whether the compounds have the effect of alleviating the disorder of lipid metabolism, inflammation and fibrosis in the pathological state of NASH.

The results showed that compound **A1** of the present invention significantly slowed down body weight gain and reduced body fat content in mice by increasing energy metabolism and promoting thermogenesis without affecting food intake (Figure 1); A1 significantly reduced fasting glucose, insulin and HOMA-IR index, and significantly improved glucose tolerance and insulin sensitivity in mice (Figure 2); A1 significantly reduced NASH mice's liver weight, liver-to-body ratio, and serum levels ALT, AST, triglycerides, T-CHO, LDL-C, and HDL-C and liver levels of triglycerides, T-CHO and hydroxyproline (Figure 3); at the gene level,**A1** significantly upregulated the mRNA expression of fatty acid oxidation-related genes (*Ppara,Cyp4**A1***,*Acot1, andAcox1*), and downregulated inflammation (*Tnfa,Il1b,Casp1,Pycard,Ccl2,* and*Ccl3*) and fibrosis-related genes (*Asma,Tgfb,Col1**A1**,Col3**A1**,* and*Col5a2*) mRNA expression (Figure 4); quantitative analysis of H&E-stained liver pathology sections shows that A1 significantly decreased NAS scores, significantly alleviated liver ballooning degeneration and inflammation, and had an ameliorating (but not significant) effect on lipid accumulation in the liver (Figure 5); in quantitative analysis of liver pathological sections stained with Oil Red O, A1 significantly reduced liver lipid accumulation, mainly by reducing lipid droplet size (Figure 5); in quantitative analysis of liver pathological sections stained with Sirius Scarlet, A1 significantly reduced liver collagen deposition (Figure 5).

In summary, long-term administration of subject A1 can significantly alleviate GAN diet-induced obesity and insulin resistance in mice, improve energy metabolism and insulin sensitivity, improve liver function, promote hepatic fatty acid oxidation, reduce inflammation and fibrosis, and has a significant alleviating effect on NASH.

**Experimental Example 7 Pharmacological Activity Test in carbon tetrachloride ($CCl_4$) induced liver fibrosis model**

Male C57BL/6 mice were injected intraperitoneally with 5% $CCl_4$(dissolved in corn oil) at 5 mL/kg twice a week to induce liver fibrosis model.Three weeks after modeling, mice were randomly divided into five groups: model control group (Model, n = 12), positive compound FTY720 group (2 mg/kg, n = 9), positive compound OCA group (30 mg/kg, n = 10), low dose group (3 mg/kg), high dose group (10 mg/kg), FTY720 was administered by intraperitoneal injection and the rest of the groups were administered by oral gavage, once a day.After 5 weeks of administration, the mice were sacrificed by dislocation after blood was taken via the posterior ophthalmic plexus, liver and spleen were taken and weighed. Part of the liver was fixed in 4% paraformaldehyde, and part of the liver and serum were stored at - 80°C.10 mice from the same litter were injected intraperitoneally with the same dose of corn oil throughout the experiment as a systemic control.

In the $CCl_4$-induced liver fibrosis model, compound A1 significantly reduced ALT levels in serum without affecting liver and spleen organ weights (Figure 6); at the gene level, high dose A1 significantly downregulated the mRNA expression of liver fibrosis-related genes (*Tgfb, Col1a1*) (Figure 7); pathological analysis of liver tissues showed that high dose A1 significantly alleviated hydroxyproline content in the liver, and pathological section analysis showed that A1 significantly reduced collagen deposition in the liver (Figure 8).

**Experimental Example 8 Pharmacological activity test in methionine-choline deficiency combined with high fat model diet (MCD-HFD) induced NASH model**

Male C57BL/6 mice were fed with MCD-HFD diet to induce the NASH model, and 4 weeks after modeling, the mice were randomly divided into 3 groups: normal diet control group (Control, n = 9), model control group (Model, n = 10), positive compound BAF312 group (0.3 mg/kg, n = 10), low dose group (0.3 mg/ kg, n = 10), medium dose group (3 mg/kg, n = 10), and high dose group (10 mg/kg, n = 10), administered by gavage in a volume of 5 mL/kg once a day.The animals were monitored for food intake and body weight during administration; 4 weeks after administration,

the mice were sacrificed by dislocation after blood was taken via the posterior ophthalmic plexus, livers and spleens were weighed, part the liver was fixed in 4% paraformaldehyde, and all tissues and serum were stored at -80°C.

In the methionine-choline deficiency combined with high-fat model feed diet (MCD-HFD)-induced NASH model compound A1 significantly reduced serum ALT and AST levels in the high-dose group without affecting body weight, while significantly alleviating TG accumulation in the liver (Figure 9); at the gene level, high/ medium/low doses of A1 significantly downregulated the mRNA expression of liver inflammation-related genes (*Tnfa,Il1b,Nlrp3, Ccl2*) to different degrees, and analysis of H&E-stained liver pathology sections showed that high-dose A1 was effective in alleviating hepatic lipid accumulation and inflammatory infiltration (Figure 10); high/medium-dose A1 was also effective in alleviating hydroxyproline levels in the liver to different degrees and significantly downregulated the expression levels of fibrosis-related genes (*Asma,Tgfb,* and*Col1a1*) at the gene level (Figure 11).

**Experimental Example 9 Pharmacological activity test in dextran sodium sulfate (DSS)-induced IBD models**

The mice were randomly divided into 5 groups: normal control group (Control,n = 5), model control group (Model,n = 11), positive compound BAF312 group (0.3 mg/kg,n = 10), low dose group (0.03 mg/kg,n = 10), medium dose group (0.3 mg/kg,n = 10), high dose group (3 mg/kg, n = 10), and each group was administered by gavage in a volume of 5 mL/kg once a day.DSS with an average molecular weight of 36000-50000 Da was dissolved in drinking water to make 2% DSS solution, and the mice in the modeling group were allowed to drink it freely for 6 days, while the drug administration treatments were carried out simultaneously.The body weight, fecal hardness and occult blood of mice in each group were observed every day, and the scores of each index were summed to obtain the disease activity index of each animal.After 6 days of completion of modeling and drug administration, the modeling reagents were withdrawn for a 1-day remission period, and the mice were sacrificed by dislocation after blood was taken via the posterior ophthalmic plexus. Colon tissue and spleen were measured for length or weighed, part of the colon was fixed in 4% paraformaldehyde, and all tissues and serum were stored at -80°C.

In the dextran sodium sulfate (DSS)-induced IBD model, high/medium/low doses of A1 were effective in alleviating the disease process to different degrees (Figure 12); in the analysis of lesioned colon sites, high/medium/low doses of A1 were effective in alleviating the shorten of colon length in IBD mice to different degrees without affecting the weight of the spleen; and in the analysis of H&E-stained colon pathology sections, high/medium doses of A1 could effectively improve the impaired crypt and inflammatory infiltration in colon sites (Figure 13).

In conclusion, long-term administration of subject A1 can significantly alleviate GAN diet-induced obesity and insulin resistance in mice, improve energy metabolism and insulin sensitivity, improve liver function, promote hepatic fatty acid oxidation, reduce inflammation and fibrosis, and has significant alleviating effects on NASH.It also exerted effective alleviating effects in the CC14-induced liver fibrosis model and the MCD-HFD diet-induced NASH model.Besides, the subject A1 was also able to exert significant anti-inflammatory as well as pathological ameliorating effects in the IBD model.

All documents mentioned in the present invention are cited by reference in this application, just as each document is cited by reference.Further, it should be understood that upon reading the above teaching of the present invention, various modifications or modifications may be made to the present invention by those skilled in the art, and those equivalents also fall within the scope defined by the appended claims of the present application.

## Claims

1. A compound of the following formula I, a pharmaceutically acceptable salt thereof, a racemate, an R-isomer, an S-isomer or a mixture thereof:

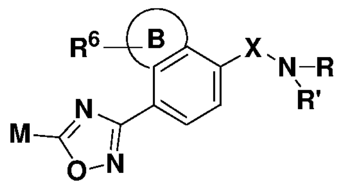

I

wherein,

M is selected from the group consisting of substituted or unsubstituted C1-C6 alkyl, substituted or unsubstituted

C1-C6 alkenyl, substituted or unsubstituted 3- to 12-membered saturated aliphatic ring, substituted or unsubstituted 3- to 12-membered unsaturated aliphatic ring, substituted or unsubstituted 3- to 12-membered aliphatic ring containing 1 to 8 heteroatoms, substituted or unsubstituted 9- to 12-membered aromatic fused ring, substituted or unsubstituted C6- C10 aromatic ring (preferably a benzene ring) and substituted or unsubstituted 5-12-membered aromatic heterocycle containing 1 to 4 heteroatoms selected from group consisting of oxygen, sulfur and nitrogen; wherein the substituted in M group means the group may be substituted by one or more $R^1$ groups; and

each $R^1$ is independently selected from the group consisting of hydrogen, deuterium, tritium, halogen, cyano, amino, hydroxyl, nitro, aldehyde group, substituted or unsubstituted guanyl,substituted or unsubstituted C1 to C6 alkyl (includingtrifluoromethyl), substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl 5- to 7-membered heteroaryl, substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^4$, $-OCOR^4$, and $SO_2R^4$;

X is selected from the group consisting of $CHR^2$, $NR^2$, O and S;

$R^2$ is selected from the group consisting of: hydrogen, deuterium, tritium, halogen, cyano, amino, hydroxyl, nitro, aldehyde group, substituted or unsubstituted guanyl, substituted or unsubstituted guanidino, substituted or unsubstituted C1 to C6 alkyl (containing trifluoromethyl), C1 to C3 alkyl substituted by 1-7 fluorine atoms, substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl 5- to 7-membered heteroaryl, substituted or unsubstituted C3 to C12 cycloalkyl, substituted or unsubstituted C2 to C10 acyl, substituted or unsubstituted C2 to C10 ester, substituted or unsubstituted C1 to C6 amide, - $SO_2R^5$, and $-COR^5$;

R and R' are independently selected from the group consisting of: H, substituted or unsubstituted C1-C6 alkyl, substituted or unsubstituted 3-12 member saturated aliphatic ring; or R, R' and the N atom to which they connect together form the following structure:

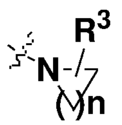

n is 1, 2, 3, 4, 5, 6, or 7;

$R^3$ is selected from the group consisting of: carboxyl, sulfonic acid group ($-SO_2H$), phosphate group ($-PO_3H$), substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^5$,$-OCOR^5$, $-C(O)R^5$,$-C(O)OR^5$,$-SO_2R^5$, and $-PO(OR^5)_2$;

ringB is selected from the group consisting of: 5-to 10-membered saturated or unsaturated carbonring, 5- to 10-membered saturated or unsaturated heterocycle; wherein the heterocycle has one or more N atoms as ring member;

$R^4$ and $R^5$ are independently selected from the group consisting of hydrogen, deuterium, tritium, amino, hydroxyl, substituted or unsubstituted C1 to C6 alkyl (including trifluoromethyl), C1 to C3 alkyl containing 1-7 fluorine atoms, substituted or unsubstituted C6 to C10 aryl, substituted or unsubstituted 5-7 membered heterocycle, substituted or unsubstituted C1 to C6 alkyl phenyl, substituted or unsubstituted C1 to C6 alkyl 5-7 membered heteroaryl, substituted or unsubstituted C3 to C12 cycloalkyl, substituted or unsubstituted C2 to C10 acyl, substituted or unsubstituted C2 to C10 ester, and substituted or unsubstituted C1 to C6 amide;

$R^6$ is one or more groups on the ring

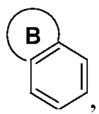,

and selected from the group consisting of hydrogen, deuterium, tritium, substituted or unsubstituted C1 to C6 alkyl;

wherein, the substitutedmeans one or more hydrogen atoms on the group being substituted by a substituent selected from the group consisting of: halogen, hydroxyl, carboxyl, benzyl, $C_1$-$C_6$alkoxycarbonyl, ami-

no, $C_1$-$C_6$amido, nitro, cyano, unsubstituted or halogenated $C_1$-$C_6$alkyl, $C_2$-$C_{10}$alkenyl, $C_1$-$C_6$alkoxy, $C_1$-$C_6$alkylamine, $C_6$-$C_{10}$aryl, five-membered or six-membered heteroaryl, -O-($C_6$-$C_{10}$aryl), and -O-(five-membered or six-membered heteroaryl).

**2.** The compound of formula I according to claim 1, a pharmaceutically acceptable salt thereof, a racemate, an R-isomer, an S-isomer or a mixture thereof, wherein the compound has the structure shown in formula II:

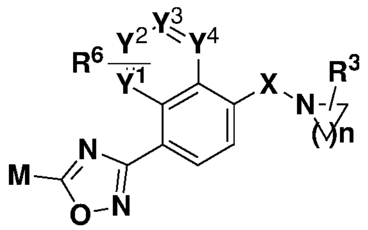

II

wherein,

$Y^1$, $Y^2$, $Y^3$, and $Y^4$are each independently selected from N or CH, and when $Y^1$, $Y^2$, $Y^3$, or $Y^4$ is CH, the CH may be substituted by $R^6$.

**3.** The compound of formula I according to claim 1, a pharmaceutically acceptable salt thereof, a racemate, an R-isomer, an S-isomer or a mixture thereof, wherein the compound has the structure shown in formula III:

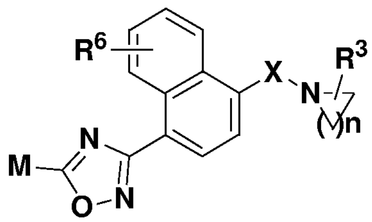

III

wherein, Xis $CHR^2$.

**4.** The compound of formula I according to claim 1, a pharmaceutically acceptable salt thereof, a racemate, an R-isomer, an S-isomer or a mixture thereof, wherein M has the following structure:

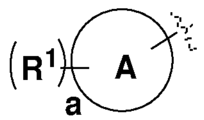

wherein,

A is selected from the group consisting of substituted or unsubstituted 3-12 membered saturated aliphatic ring, substituted or unsubstituted 3-12 membered unsaturated aliphatic rings, substituted or unsubstituted 3-12 membered aliphatic rings containing 1-8 heteroatoms, substituted or unsubstituted 7-12 membered aromatic fused ring, substituted or unsubstituted C6-C10 aromatic ring (preferably benzene ring), and substituted or unsubstituted 5-12 membered aromatic heterocycle containing 1 to 4 heteroatoms selected from the group consisting of oxygen, sulfur and nitrogen;

each $R^1$ is independently selected from the group consisting of hydrogen, deuterium, tritium, halogen, cyano, amino, hydroxyl, nitro, aldehyde group, substituted or unsubstituted C1 to C6 alkyl (including trifluoromethyl), C1 to C3 alkyl containing 1-7 fluorine atoms, substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl (5- to 7-membered heteroaryl), substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^4$, $-OCOR^4$, and $SO_2R^4$;
a is 1, 2, 3, 4, 5, 6, 7, 8, 9, 10 or 11.

**5.** The compound of formula I according to claim 4, a pharmaceutically acceptable salt thereof, a racemate, an R-isomer, an S-isomer or a mixture thereof, wherein ring A is selected from the group consisting of substituted or unsubstituted benzene ring, substituted or unsubstituted 5-7 membered saturated aliphatic ring, substituted or unsubstituted 5- to 10-membered aromatic heterocycle containing 1 to 4 heteroatoms selected from oxygen, sulfur and nitrogen, substituted or unsubstituted partially unsaturated 5- to 10- membered heterocycle containing 1 to 4 heteroatoms selected from oxygen, sulfur and nitrogen;

each $R^1$ is independently selected from the group consisting of: hydrogen, deuterium, tritium, halogen, cyano, amino, hydroxyl, nitro, aldehyde group, substituted or unsubstituted C1 to C6 alkyl (includingtrifluoromethyl), C1 to C3 alkyl containing 1-7 fluorine atoms, substituted or unsubstituted C1 to C6 alkoxy, substituted or unsubstituted C6 to C10 aryl, substituted or unsubstituted 5- to 7-membered heterocycle, substituted or unsubstituted C1 to C6 alkyl phenyl, substituted or unsubstituted C1 to C6 alkyl (5- to 7-membered heteroaryl), substituted or unsubstituted C3 to C12 cycloalkyl, substituted or unsubstituted C2 to C10 acyl, substituted or unsubstituted C2 to C10 ester, substituted or unsubstituted C2 to C10 aryl ether, substituted or unsubstituted C1 to C6 amide, $-OSO_2R^4$, $-OCOR^4$, and $SO_2R^4$;
a is 1, 2, 3, 4 or 5.

**6.** The compound of formula I according to claim 1, a pharmaceutically acceptable salt thereof, a racemate, an R-isomer, an S-isomer or a mixture thereof, wherein the compound is selected from the group consisting of:

| No. | Name | Structure |
|---|---|---|
| A1 | 1-((4-(5-(3-cyano-4-isopropoxyphenyl)-1,2,4-oxadiazol-3-yl) naphthalen-1-yl)methyl)azetidine-3-carboxylic acid hydrochloric acid | 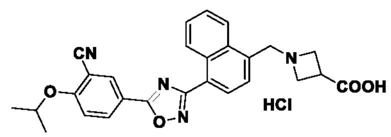 |
| A2 | 1-((4-(5-(3-cyano-4-isopropoxyphenyl)-1,2,4-oxadiazol-3-yl) naphthalen-1-yl)methyl) pyrrolidin-3-carboxylic acid hydrochloric acid | 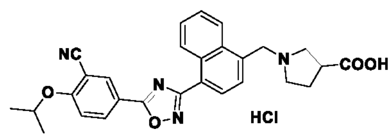 |
| A3 | ((4-(5-(3-cyano-4-isopropoxyphenyl)-1,2,4-oxadiazol-3-yl) naphthalen-1-yl) methyl) proline hydrochloric acid | 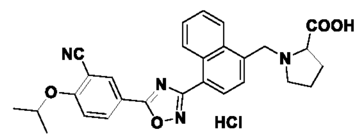 |
| A4 | (cis)-3-(((4-(5-(3-cyano-4-isopropyloxyphenyl)-1,2,4-oxadiazol-3-yl)naphthalen-1-yl)methyl)amino) cyclobutane-1-carboxylic acid hydrochloric acid | 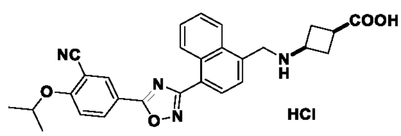 |
| A5 | *N*-((4-(5-(3-cyano-4-isopropoxyphenyl)-1,2,4-oxadiazol-3-yl) naphthalen-1-yl)methyl)-N-methylglycine hydrochloride | 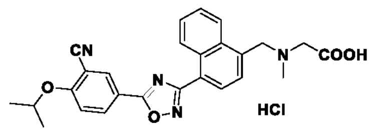 |

(continued)

| No. | Name | Structure |
|---|---|---|
| A6 | ((4-(5-(3-cyano-4-isopropoxyphenyl)-1,2,4-oxadiazol-3-yl) naphthalen-1-yl) methyl) alanine hydrochloric acid | 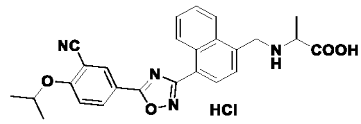 |
| A7 | ((4-(5-(3-cyano-4-isopropoxyphenyl)-1,2,4-oxadiazol-3-yl) naphthalen-1-yl) methyl) glycine hydrochloride | 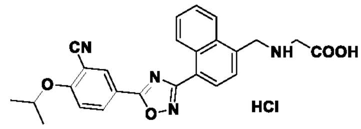 |
| A8 | 3-(((4-(5-(3-cyano-4-isopropoxyphenyl)-1,2,4-oxadiazol-3-yl) naphthalen-1-yl)methyl)amino) propionic acid hydrochloric acid | 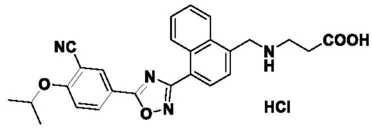 |
| A9 | 2-(((4-(5-(3-cyano-4-isopropoxyphenyl)-1,2,4-oxadiazol-3-yl) naphthalen-1-yl)methyl)amino) malonic acid hydrochloric acid | 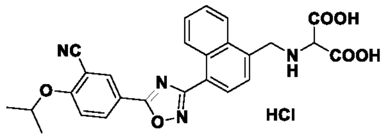 |
| A10 | 1-((4-(5-(3-chloro-4-isopropoxyphenyl)-1,2,4-oxadiazol-3-yl) naphthalen-1-yl)methyl)azetidine-3-carboxylic acid hydrochloric acid | 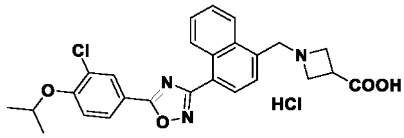 |
| A11 | 1-((4-(5-(3-bromo-4-isopropoxyphenyl)-1,2,4-oxadiazol-3-yl) naphthalen-1-yl)methyl)azetidine-3-carboxylic acid hydrochloric acid | 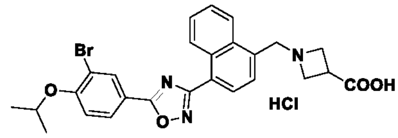 |
| A12 | 1-((4-(5-(4-isopropoxy-3-(trifluoromethyl)phenyl)-1,2,4-oxadiazol-3-yl)naphthalen-1-yl) methyl)azetidine-3-carboxylic acid hydrochloric acid | 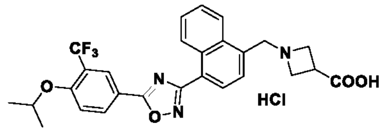 |
| A13 | 1-((4-(5-(4-isopropoxy-3-methoxyphenyl)-1,2,4-oxadiazol-3-yl) naphthalen-1-yl)methyl)azetidine-3-carboxylic acid hydrochloric acid | 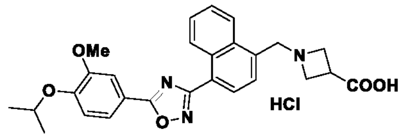 |
| A14 | 1-((4-(5-(4-isopropoxyphenyl)-1,2,4-oxadiazol-3-yl) naphthalen-1-yl) methyl)azetidine-3-carboxylic acid hydrochloric acid | 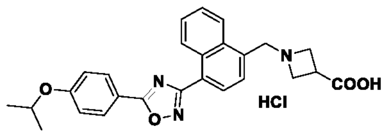 |
| A15 | 1-((4-(5-(4-(tert-butoxy) phenyl)-1,2, 4-oxadiazol-3-yl) naphthalen-1-yl) methyl)azetidine-3-carboxylic acid hydrochloric acid | 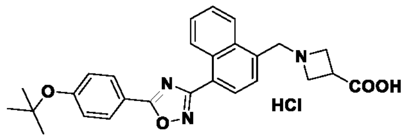 |

(continued)

| No. | Name | Structure |
|---|---|---|
| A16 | 1-((4-(5-(4-methoxyphenyl)-1,2,4-oxadiazol-3-yl)naphthalen-1-yl) methyl)azetidine-3-carboxylic acid hydrochloric acid | 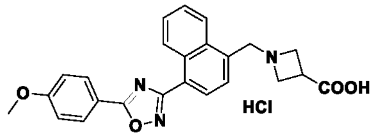 |
| A17 | 1-((4-(5-(4-propoxyphenyl)-1,2,4-oxadiazol-3-yl)naphthalen-1-yl) methyl)azetidine-3-carboxylic acid hydrochloric acid | 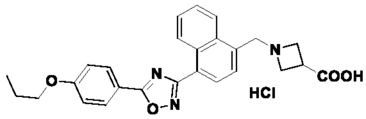 |
| A18 | 1-((4-(5-(4-(benzyloxy) phenyl)-1,2,4-oxadiazol-3-yl) naphthalen-1-yl) methyl)azetidine-3-carboxylic acid hydrochloric acid | 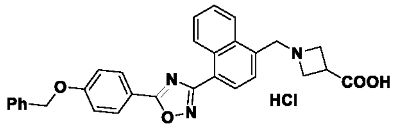 |
| A19 | 1-((4-(5-(4-(trifluoromethoxy)phenyl)-1,2,4-oxadiazol-3-yl) naphthalen-1-yl) methyl)azetidine-3-carboxylic acid hydrochloric acid | 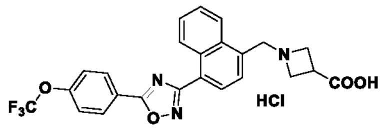 |
| A20 | 1-((4-(5-(4-isopropylphenyl)-1,2,4-oxadiazol-3-yl)naphthalen-1-yl) methyl)azetidine-3-carboxylic acid hydrochloric acid | 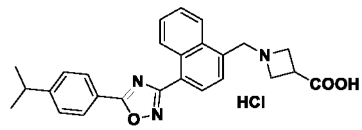 |
| A21 | 1-((4-(5-(4-propylphenyl)-1,2,4-oxadiazol-3-yl)naphthalen-1-yl) methyl)azetidine-3-carboxylic acid hydrochloric acid | 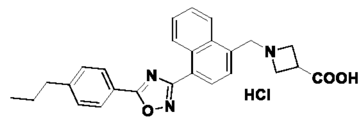 |
| A22 | 1-((4-(5-(4-cyanophenyl)-1,2,4-oxadiazol-3-yl)naphthalen-1-yl) methyl)azetidine-3-carboxylic acid hydrochloric acid | 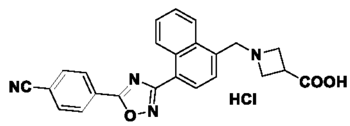 |
| A23 | 1-((4-(5-(4-fluorophenyl)-1,2,4-oxadiazol-3-yl)naphthalen-1-yl) methyl)azetidine-3-carboxylic acid hydrochloric acid | 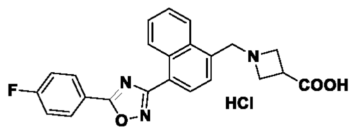 |
| A24 | 1-((4-(5-(3-cyanophenyl)-1,2,4-oxadiazol-3-yl)naphthalen-1-yl) methyl)azetidine-3-carboxylic acid hydrochloric acid | 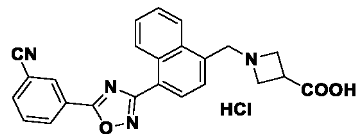 |
| A25 | 1-((4-(5-(3-fluorophenyl)-1,2,4-oxadiazol-3-yl)naphthalen-1-yl) methyl)azetidine-3-carboxylic acid hydrochloric acid | 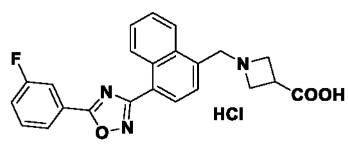 |
| A26 | 1-((4-(5-phenyl-1,2,4-oxadiazol-3-yl) naphthalen-1-yl)methyl) azetidine-3-carboxylic acid hydrochloric acid | 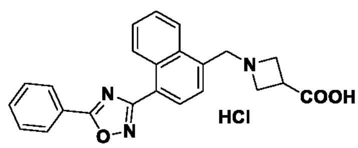 |

(continued)

| No. | Name | Structure |
|---|---|---|
| A27 | 1-((4-(5-(pyridin-4-yl)-1,2,4-oxadiazol-3-yl)naphthalen-1-yl) methyl)azetidine-3-carboxylic acid hydrochloric acid | 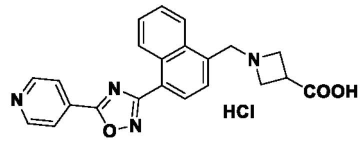 |
| A28 | 1-((4-(5-(pyridin-3-yl)-1,2,4-oxadiazol-3-yl)naphthalen-1-yl) methyl)azetidine-3-carboxylic acid hydrochloric acid | 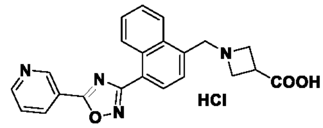 |
| A29 | 1-((4-(5-(pyridin-2-yl)-1,2,4-oxadiazol-3-yl) naphthalen-1-yl) methyl)azetidine-3-carboxylicacid hydrochloric acid | 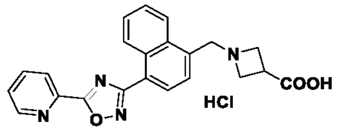 |
| A30 | 1-((4-(5-(5-methylisothiazol-3-yl)-1,2, 4-oxadiazol-3-yl) naphthalen-1-yl) methyl)azetidine-3-carboxylic acid hydrochloric acid | 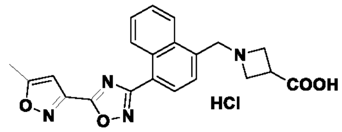 |
| A31 | 1-((4-(5-(5-(benzo[*d*][1,3]dioxazol-5-yl)-1,2,4-oxadiazol-3-yl) naphthalen-1-yl)methyl)azetidine-3-carboxylic acid hydrochloric acid | 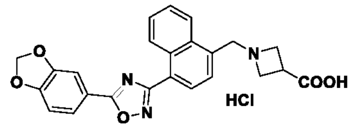 |
| A32 | 1-((4-(5-(2,3-dihydrobenzo[*b*][1,4] dioxin-6-yl)-1,2,4-oxadiazol-3-yl) naphthalen-1-yl)methyl)azetidine-3-carboxylic acid hydrochloric acid | 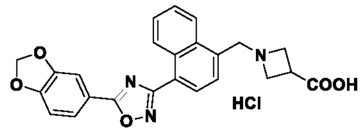 |
| A33 | 1-((4-(5-(quinolin-3-yl)-1,2,4-oxadiazol-3-yl)naphthalen-1-yl) methyl)azetidine-3-carboxylic acid hydrochloric acid | 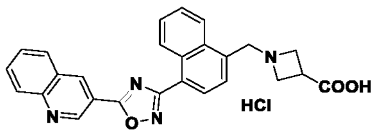 |
| A34 | 1-((4-(5-(quinoxaline-2-yl)-1,2,4-oxadiazol-3-yl)naphthalen-1-yl) methyl)azetidine-3-carboxylic acid hydrochloric acid | 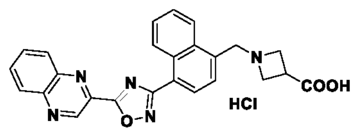 |
| A35 | 1-((4-(5-(1*H*-indole-5-yl)-1,2,4-oxadiazol-3-yl)naphthalen-1-yl) methyl)azetidine-3-carboxylic acid hydrochloric acid | 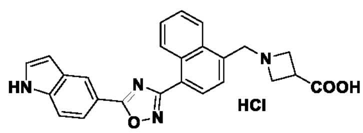 |
| A36 | 1-((4-(5-(1*H*-indole-2-yl)-1,2,4-oxadiazol-3-yl)naphthalen-1-yl) methyl)azetidine-3-carboxylic acid hydrochloric acid | 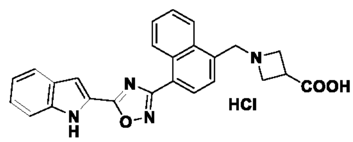 |

(continued)

| No. | Name | Structure |
|---|---|---|
| A37 | 1-((4-(5-(1-methyl-1*H*-indole-2-yl)-1,2,4-oxadiazol-3-yl) naphthalen-1-yl) methyl)azetidine-3-carboxylic acid hydrochloric acid | 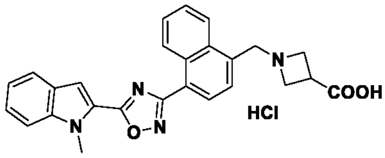 |
| A38 | (*E*)-1-((4-(5-styryl-1,2,4-oxadiazol-3-yl)naphthalen-1-yl)methyl) azetidine-3-carboxylic acid hydrochloric acid | 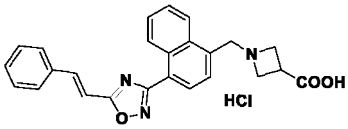 |
| A39 | 1-((4-(5-cyclohexyl-1,2,4-oxadiazol-3-yl)naphthalen-1-yl) methyl) azetidine-3-carboxylic acid hydrochloric acid | 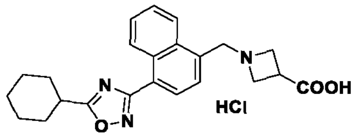 |
| A40 | 1-((4-(5-(3-cyano-4-isopropoxyphenyl)-1,2,4-oxadiazol-3-yl) naphthalen-1-yl)methyl) piperidine-4-carboxylic acid hydrochloric acid | 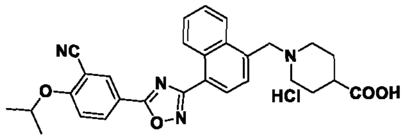 |
| A41 | 1-((4-(5-(3-cyano-4-isopropoxyphenyl)-1,2,4-oxadiazol-3-yl) naphthalen-1-yl)methyl) piperidine-3-carboxylic acid hydrochloric acid | 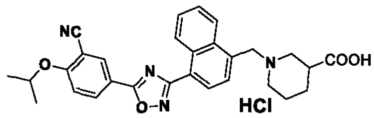 |
| A42 | 1-((4-(5-(3-cyano-4-isopropoxyphenyl)-1,2,4-oxadiazol-3-yl) naphthalen-1-yl)methyl) piperidine-3,4-carboxylic acid hydrochloric acid | 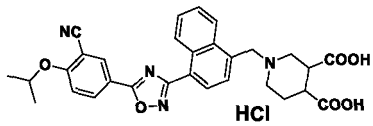 |
| A43 | 1-((4-(5-(3-cyano-4-isopropoxyphenyl)-1,2,4-oxadiazol-3-yl) naphthalen-1-yl) methyl)pyrrolidin-3-sulfonic acid | 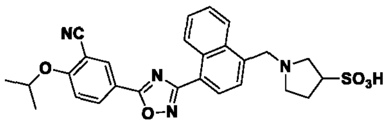 |
| A44 | Methyl 1-((4-(5-(3-cyano-4-isopropoxyphenyl)-1,2,4-oxadiazol-3-yl)naphthalen-1-yl)methyl)azelaic acid-3-carboxylate | 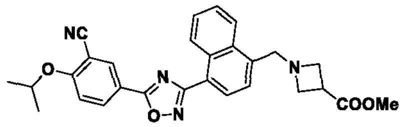 |
| A45 | Ethyl 1-((4-(5-(3-cyano-4-isopropoxyphenyl)-1,2,4-oxadiazol-3-yl)naphthalen-1-yl)methyl)azelaic acid-3-carboxylate | 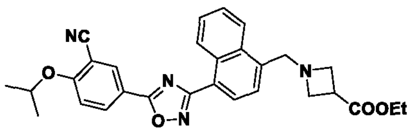 |
| A46 | Isopropyl 1-((4-(5-(3-cyano-4-isopropoxyphenyl)-1,2,4-oxadiazol-3-yl) naphthalen-1-yl)methyl)azelaic acid-3-carboxylate | 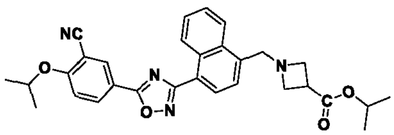 |

(continued)

| No. | Name | Structure |
|---|---|---|
| A47 | 47 1-((4-(5-(3-cyano-4-isopropoxyphenyl)-1,2,4-oxadiazol-3-yl) naphthalen-1-yl)methyl)azelaic acid-3-carboxamide | 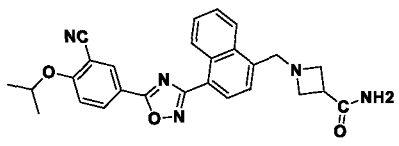 |
| A48 | 1-((4-(5-(3-cyano-4-isopropoxyphenyl)-1,2,4-oxadiazol-3-yl) naphthalen-1-yl) methyl)-N-methyl azetidine-3-formamide | 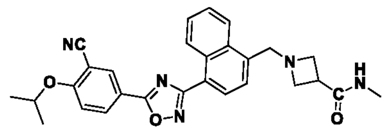 |
| A49 | 1-((4-(5-(3-cyano-4-isopropoxyphenyl)-1,2,4-oxadiazol-3-yl) naphthalen-1-yl) methyl)-N, N-dimethylazetidine-3-formamide | 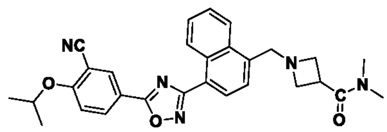 |
| A50 | -(3-(3-(3-(1H-tetrazol-5-yl)azelaic acid-1-yl)methyl)naphthalen-1-yl)-1,2,4-oxadiazol-5-yl)-2-isopropoxybenzonitrile | 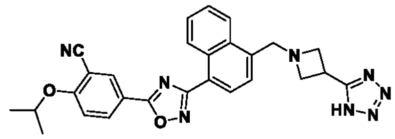 |
| A51 | 3-(4-(3-(1H-tetrazol-1-yl)azelaic acid-1-yl)methyl)naphthalen-1-yl)-5-(4-isopropoxy-3-(trifluoromethyl)phenyl)-1,2,4-oxadiazole | 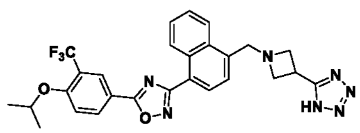 |
| A52 | 3-(4-(3-(1H-tetrazol-1-yl)azelaic acid-1-yl)methyl)naphthalen-1-yl)-5-(1H-indole-5-yl)-1,2,4-oxadiazole | 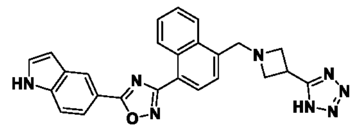 |
| A53 | 3-(4-(3-(1H-tetrazol-1-yl)azelaic acid -1-yl)methyl)naphthalen-1-yl)-5-(1H-indole-6-yl)-1,2,4-oxadiazole | 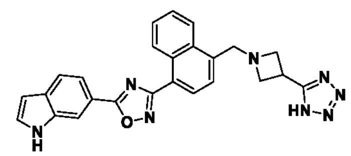 |
| A54 | 3-(4-(3-(1H-tetrazol-1-yl)azelaic acid -1-yl)methyl)naphthalen-1-yl)-5-(1H-indole-2-yl)-1,2,4-oxadiazole | 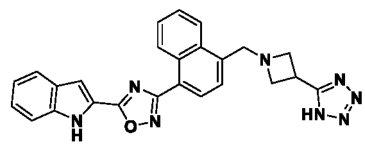 |
| A55 | Diethyl (1-(4-(5-(3-cyano-4-isopropoxyphenyl)-1,2,4-oxadiazol-3-yl) naphthalen-1-yl)methyl)azelaic acid-3-yl)phosphonate | 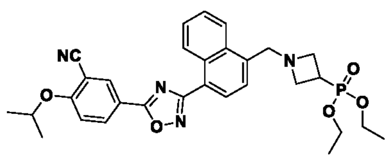 |
| A56 | 1-((5-(5-(3-cyano-4-isopropoxyphenyl)-1,2,4-oxadiazol-3-yl) isoquinoline-8-yl) methyl)azelaic acid-3-carboxylic acid hydrochloric acid | 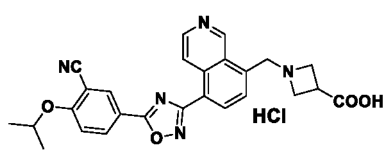 |

(continued)

| No. | Name | Structure |
|---|---|---|
| A57 | 1-((5-(5-(3-cyano-4-isopropoxyphenyl)-1,2,4-oxadiazol-3-yl) isoquinoline-8-yl) methyl)azelaic acid-3-carboxylic acid hydrochloric acid | 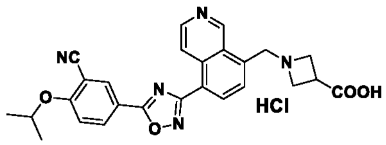 |
| A58 | 1-((8-(5-(3-cyano-4-isopropoxyphenyl)-1,2,4-oxadiazol-3-yl) quinoxaline-5-yl) methyl)azelaic acid-3-carboxylic acid hydrochloric acid | 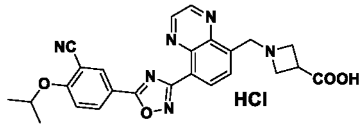 |
| A59 | 1-((4-(5-(thiophen-2-yl)-1,2,4-oxadiazol-3-yl)naphthalen-1-yl) methyl)azelaic acid-3-carboxylic acid hydrochloric acid | 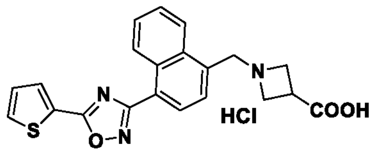 |
| A60 | 1-((4-(5-(4-methylthiophen-2-yl)-1,2, 4-oxadiazol-3-yl) naphthalen-1-yl) methyl)azelaic acid-3-carboxylic acid hydrochloric acid | 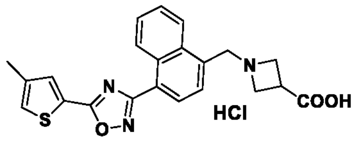 |
| A61 | 1-((4-(5-(5-methoxy-4-methylthiophen-2-yl)-1,2,4-oxadiazol-3-yl)naphthalen-1-yl)methyl) azelaic acid-3-carboxylic acid hydrochloric acid | 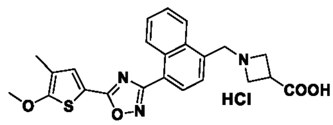 |
| A62 | 1-((4-(5-(furan-2-yl)-1,2,4-oxadiazol-3-yl)naphthalen-1-yl) methyl)azelaic acid-3-carboxylic acid hydrochloric acid | 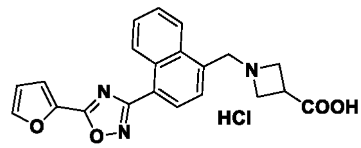 |
| A63 | 1-((4-(5-(4-(trifluoromethyl)furan-2-yl)-1,2,4-oxadiazol-3-yl) naphthalen-1-yl)methyl)azelaic acid-3-carboxylic acid hydrochloric acid | 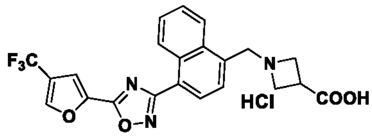 |
| A64 | 1-((4-(5-(4-cyanofuran-2-yl)-1,2,4-oxadiazol-3-yl)naphthalen-1-yl) methyl)azelaic acid-3-carboxylic acid hydrochloric acid | 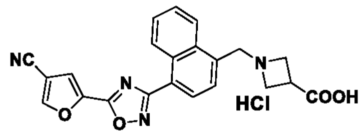 |
| A65 | 1-((4-(5-(4-isopropoxyfuran-2-yl)-1,2, 4-oxadiazol-3-yl) naphthalen-1-yl) methyl)azelaic acid-3-carboxylic acid hydrochloric acid | 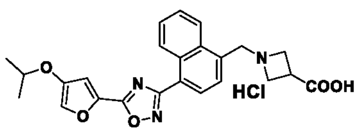 |
| A66 | 1-((4-(5-([1,1'-biphenyl]-4-yl)-1,2,4-oxadiazol-3-yl)naphthalen-1-yl) methyl)azelaic acid-3-carboxylic acid hydrochloric acid | 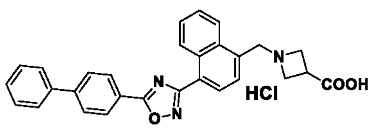 |

(continued)

| No. | Name | Structure |
|---|---|---|
| A67 | 1-((4-(5-([1,1'-biphenyl]-4-yl)-1,2,4-oxadiazol-3-yl)naphthalen-1-yl) methyl)azelaic acid-3-carboxylic acid hydrochloric acid | 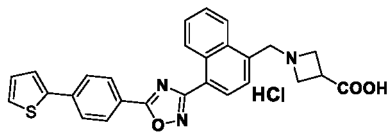 |
| A68 | 1-((4-(4-(4-(1H-pyrrol-2-yl)phenyl)-1,2,4-oxadiazol-3-yl) naphthalen-1-yl) methyl)azelaic acid-3-carboxylic acid hydrochloric acid | 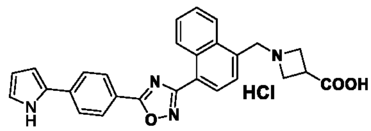 |
| A69 | 1-((4-(5-(4-(furan-2-yl) phenyl)-1,2,4-oxadiazol-3-yl)naphthalen-1-yl) methyl)azelaic acid-3-carboxylic acid hydrochloric acid | 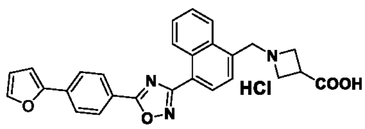 |
| A70 | 1-((4-(5-(3-Cyano-4-(cyclopentyloxy) phenyl)-1,2,4-oxadiazol-3-yl) naphthalen-1-yl)methyl)azelaic acid- 3-carboxylic acid hydrochloric acid | 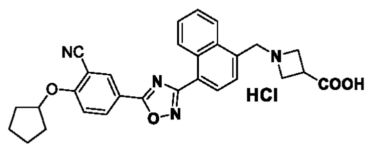 |

**7.** A pharmaceutical composition, wherein the pharmaceutical composition comprises one or more of the compounds of formula I according to claim 1, a pharmaceutically acceptable salt thereof, a racemate, an R-isomers, an S-isomers and a mixture thereof, and one or more pharmaceutically acceptable carriers, excipients, adjuvants, ingredients and/or diluents.

**8.** A use of the compound of formula I according to claim 1, a pharmaceutically acceptable salt thereof, a racemate, an R-isomer, an S-isomer or a mixture thereof, in the preparation of a pharmaceutical composition for the treatment or prevention of diseases associated with S1P agonists.

**9.** The use of claim 8, wherein the disease is selected from the group consisting ofnon-alcoholic fatty liver disease, liver fibrosis, diabetes, hyperlipidemia, multiple sclerosis (includingrelapsing multiple sclerosis, relapsing-remitting multiple sclerosis, active secondary progressive multiple sclerosis), psoriasis, ulcerative colitis, lupus erythematosus, Crohn's disease, immune disorders, wet age-related macular degeneration, atopic dermatitis, inflammatory bowel disease, and clinical isolated syndrome.

**10.** A method for preparing the compound of formula I according to claim 1, a pharmaceutically acceptable salt thereof, a racemate, an R-isomer, an S-isomer or a mixture thereof, including the steps:

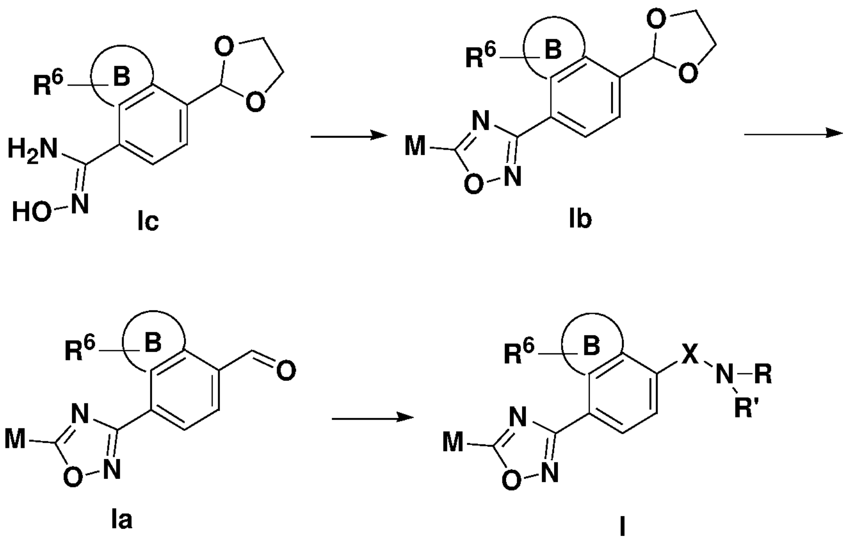

Ic Ib Ia I

(1) in DMF, reacting the compound of formula Ic with M-COOH, HOBT, EDCI and potassium carbonate to give the compound of formula Ib;
(2) in acetone/diluted hydrochloric acid, reacting the compound of formula Ib to obtain the compound of formula Ia;
(3) in a methanol/dichloromethane mixture reacting the compound of formula Ia with the hydrochlorideof

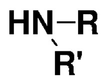
,

DIPEA, acetic acid and sodium cyanoborohydride to give the compound of formula I; wherein X is CH.

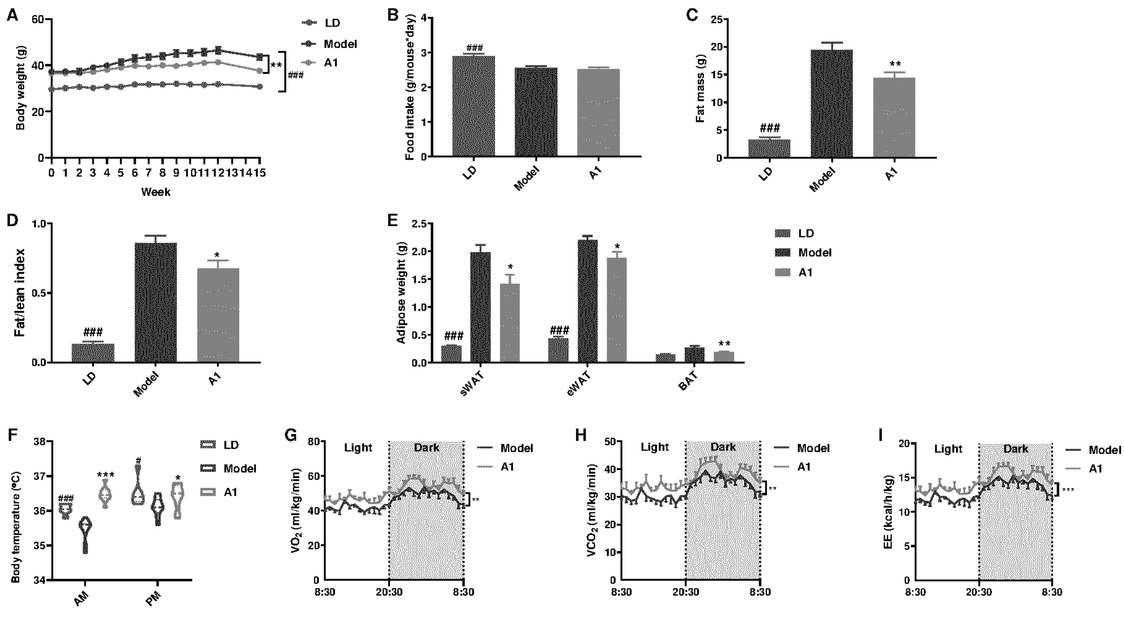


Figure 1

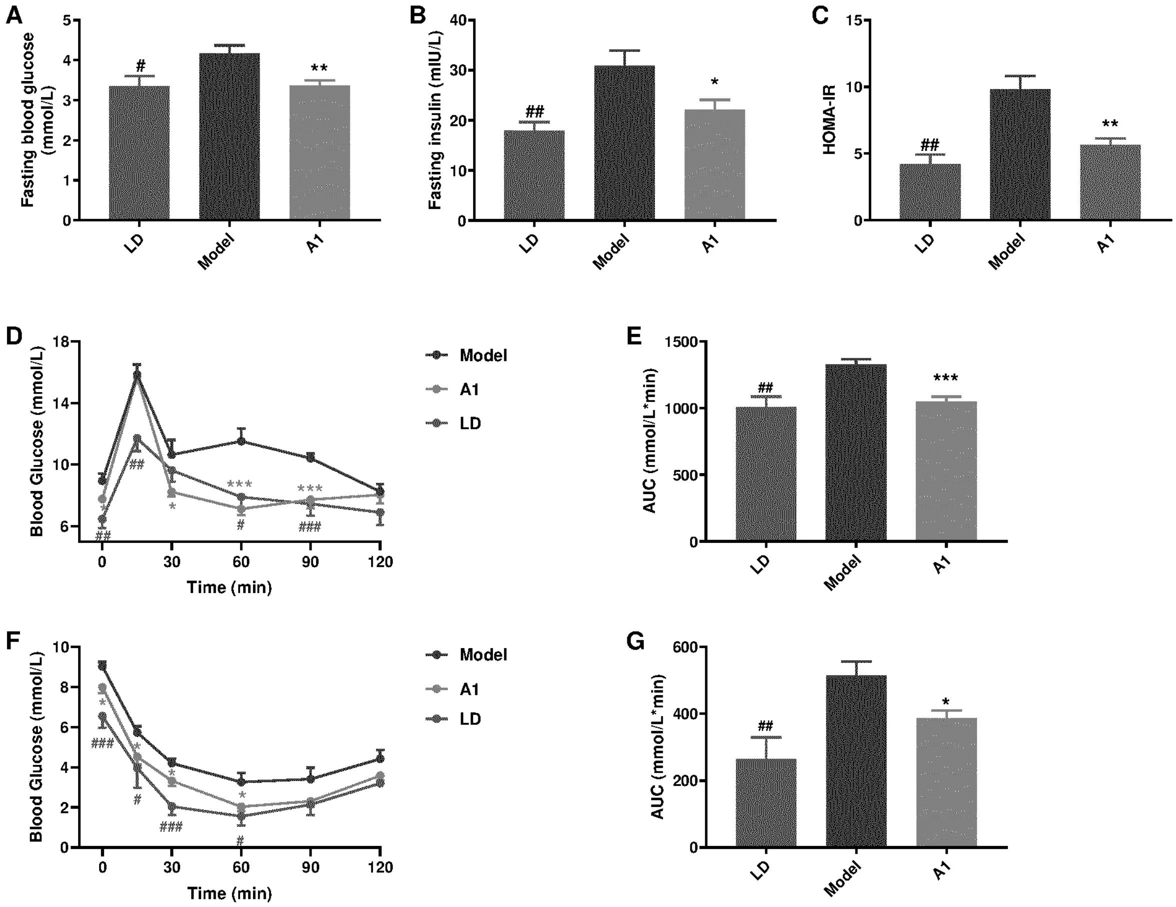


Figure 2

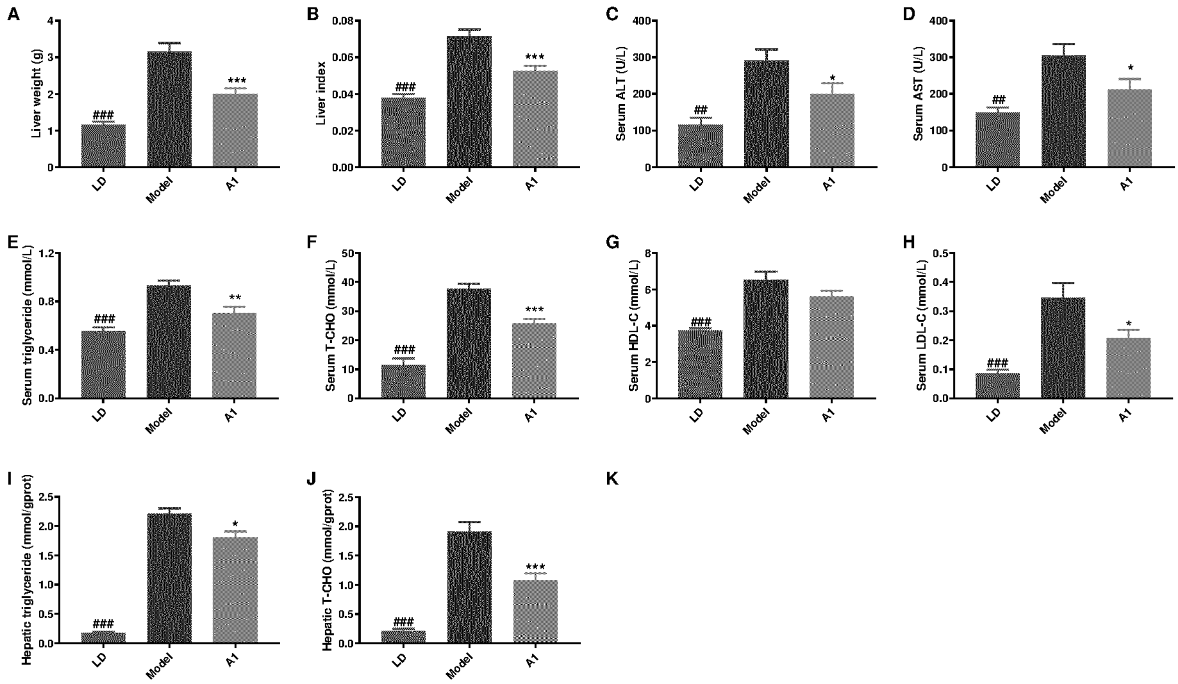
A
Liver weight (g)
###
***
B
Liver index
###
***
C
Serum ALT (U/L)
##
*
D
Serum AST (U/L)
##
*
E
Serum triglyceride (mmol/L)
###
**
F
Serum T-CHO (mmol/L)
###
***
G
Serum HDL-C (mmol/L)
###
H
Serum LDL-C (mmol/L)
###
*
I
Hepatic triglyceride (mmol/gprot)
###
*
J
Hepatic T-CHO (mmol/gprot)
###
***
K
LD
Model
A1

Figure 3

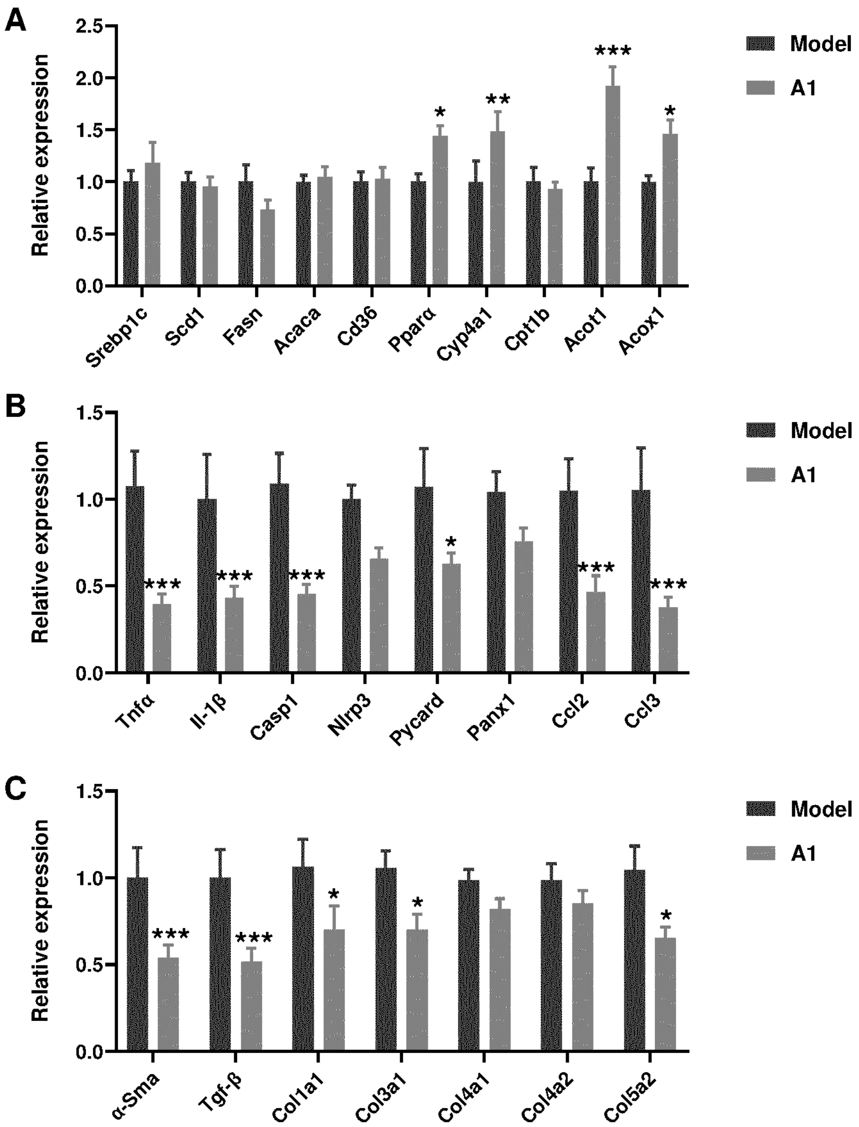
A
Relative expression
2.5
2.0
1.5
1.0
0.5
0.0
Model
A1
*
**
***
*
Srebp1c
Scd1
Fasn
Acaca
Cd36
Pparα
Cyp4a1
Cpt1b
Acot1
Acox1
B
Relative expression
1.5
1.0
0.5
0.0
Model
A1
***
***
***
*
***
***
Tnfα
Il-1β
Casp1
Nlrp3
Pycard
Panx1
Ccl2
Ccl3
C
Relative expression
1.5
1.0
0.5
0.0
Model
A1
***
***
*
*
*
α-Sma
Tgf-β
Col1a1
Col3a1
Col4a1
Col4a2
Col5a2

**Figure 4**

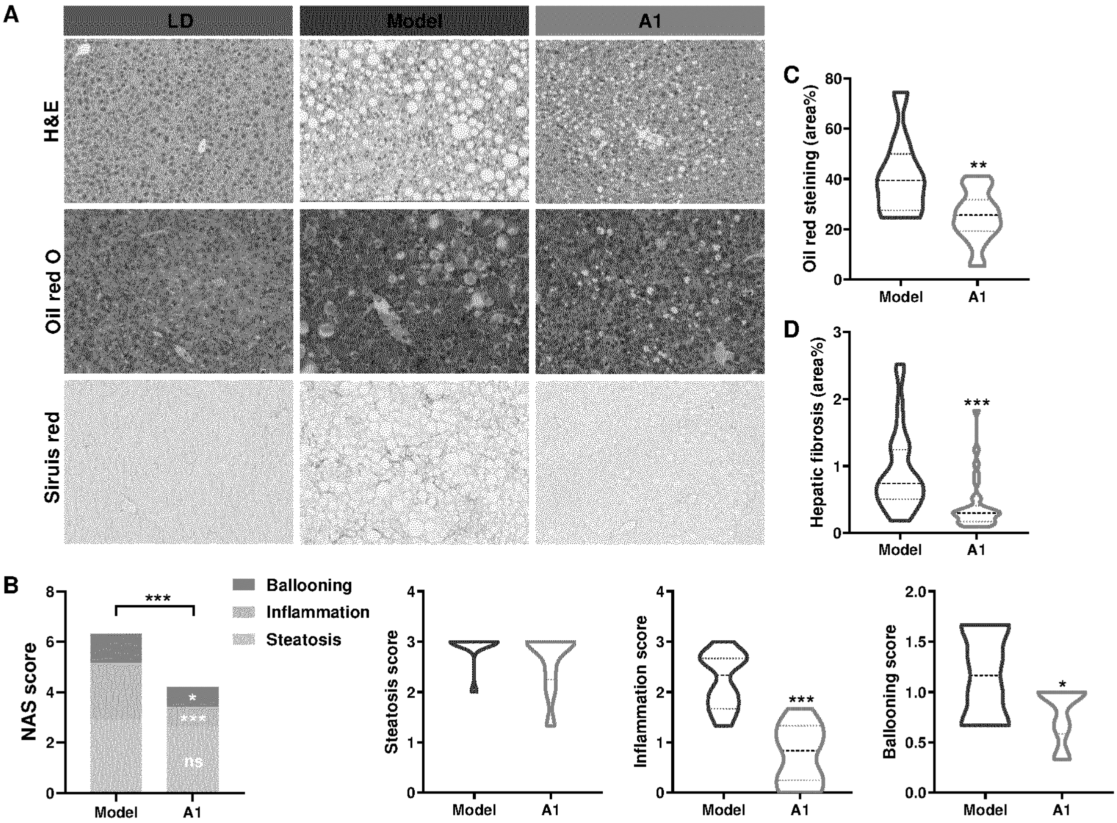


Figure 5

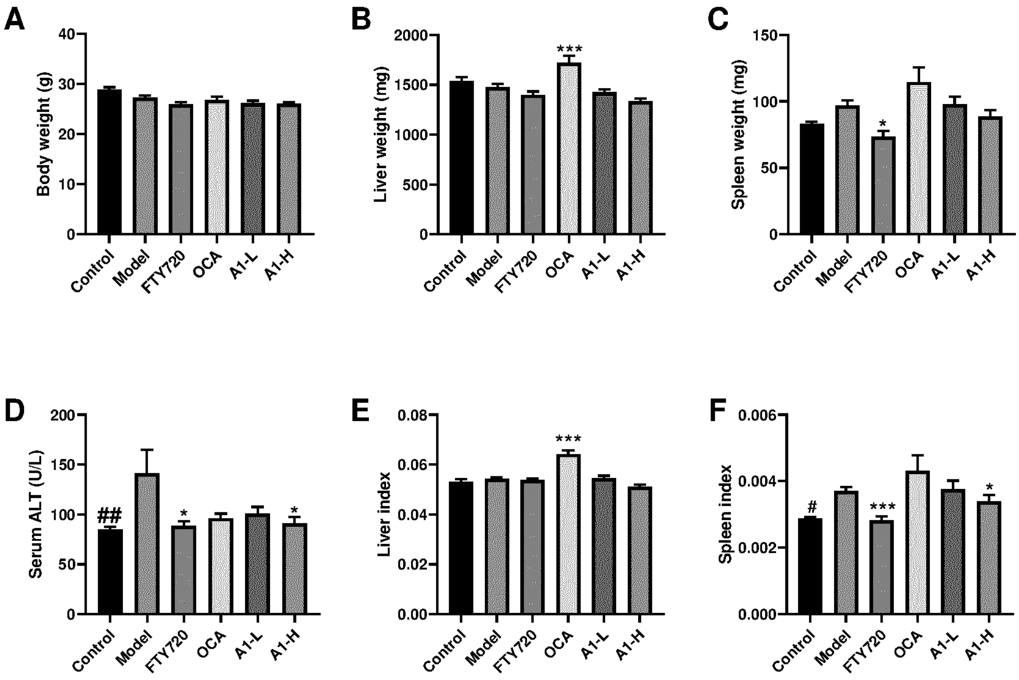


Figure 6

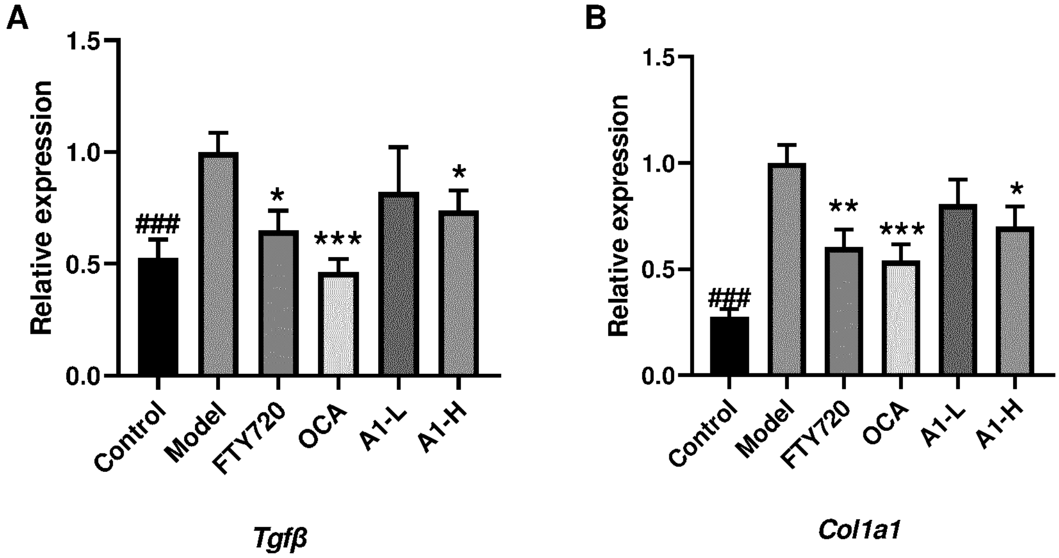


Figure 7

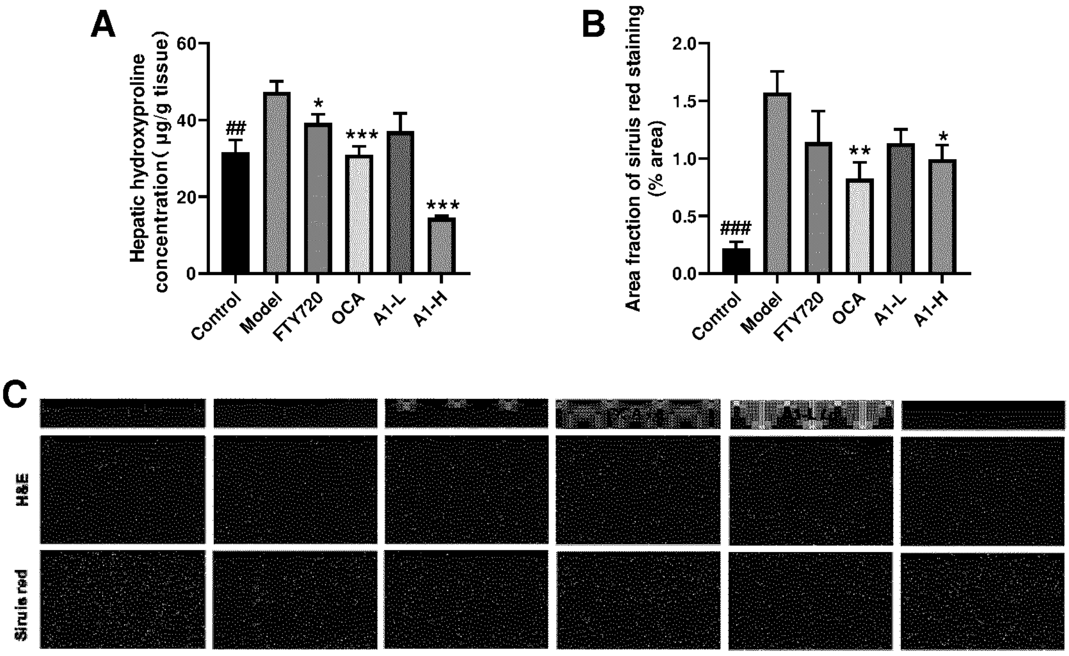


Figure 8

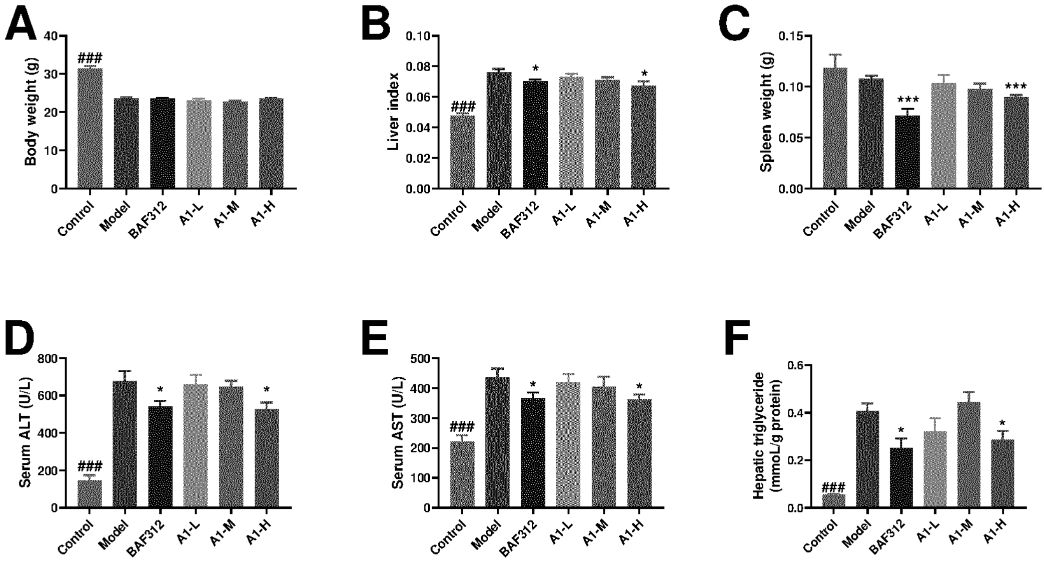


Figure 9

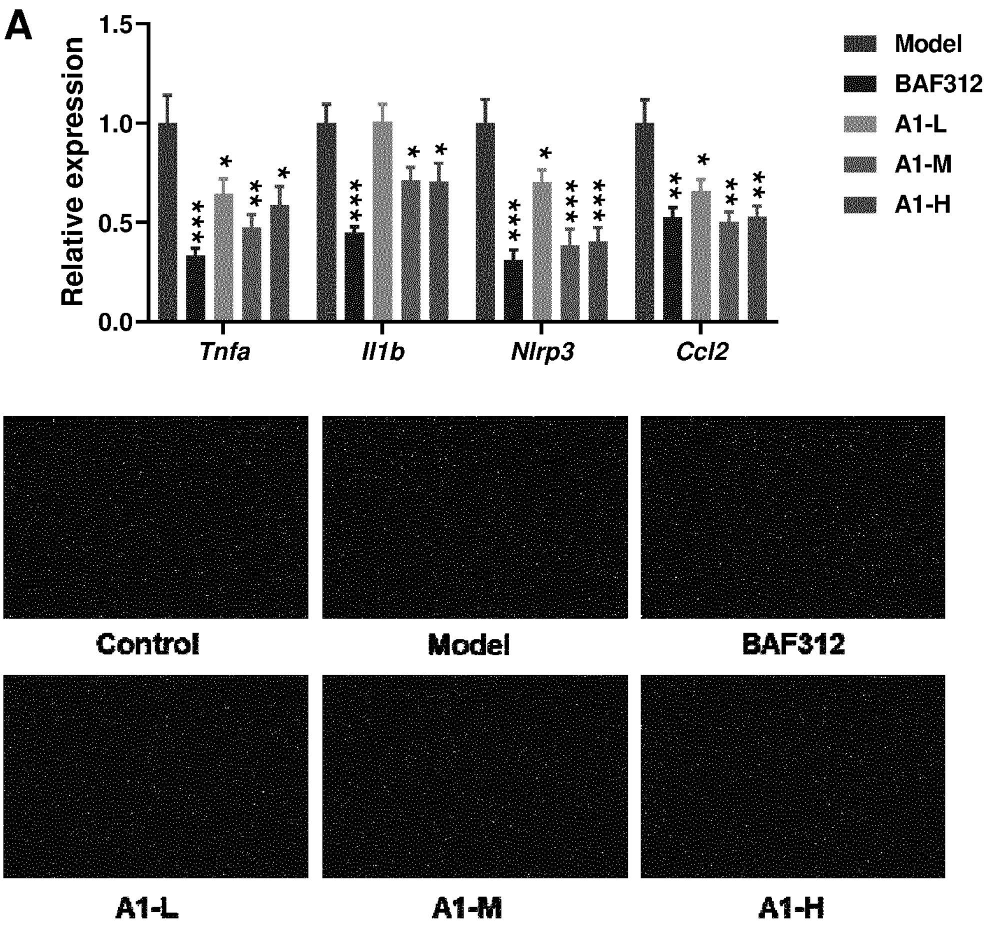


Figure 10

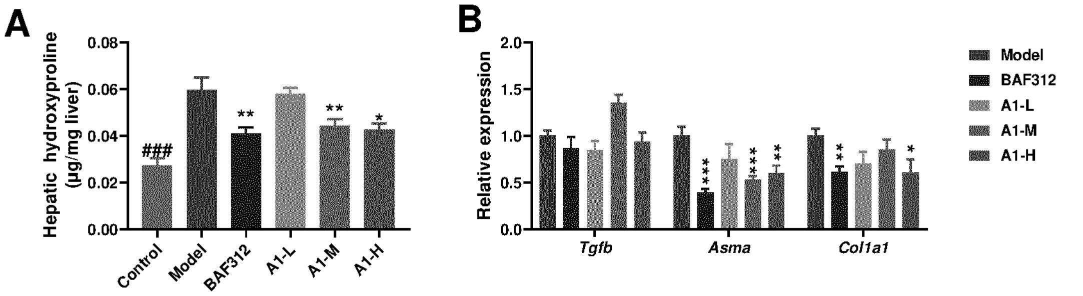


Figure 11

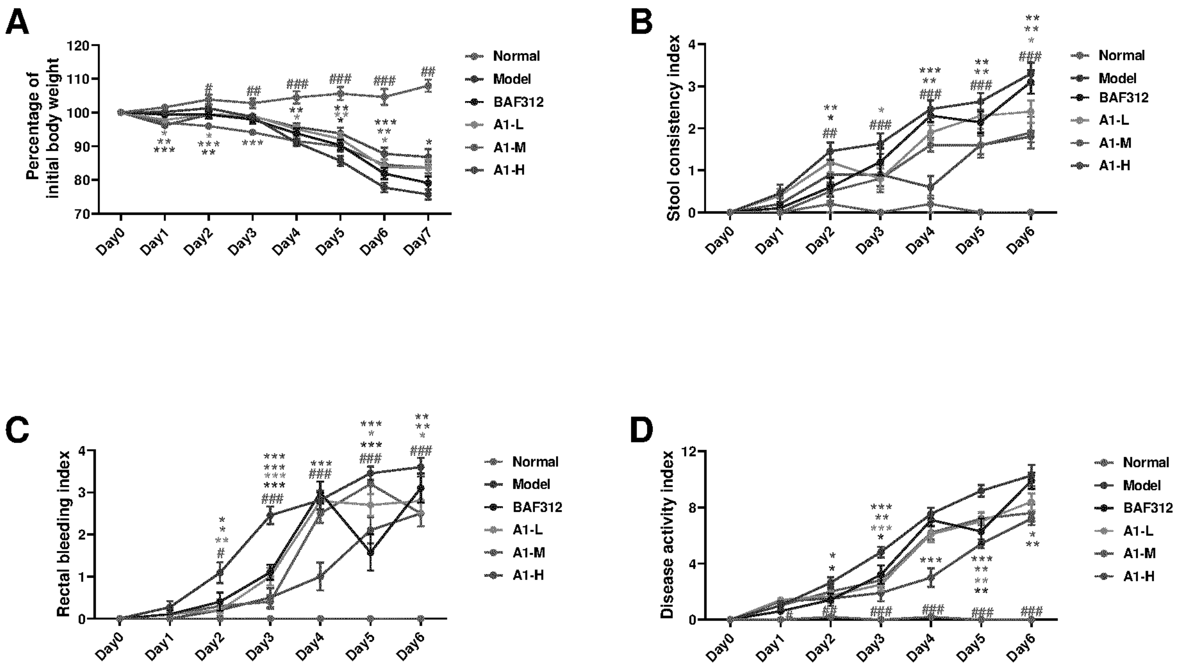


Figure 12

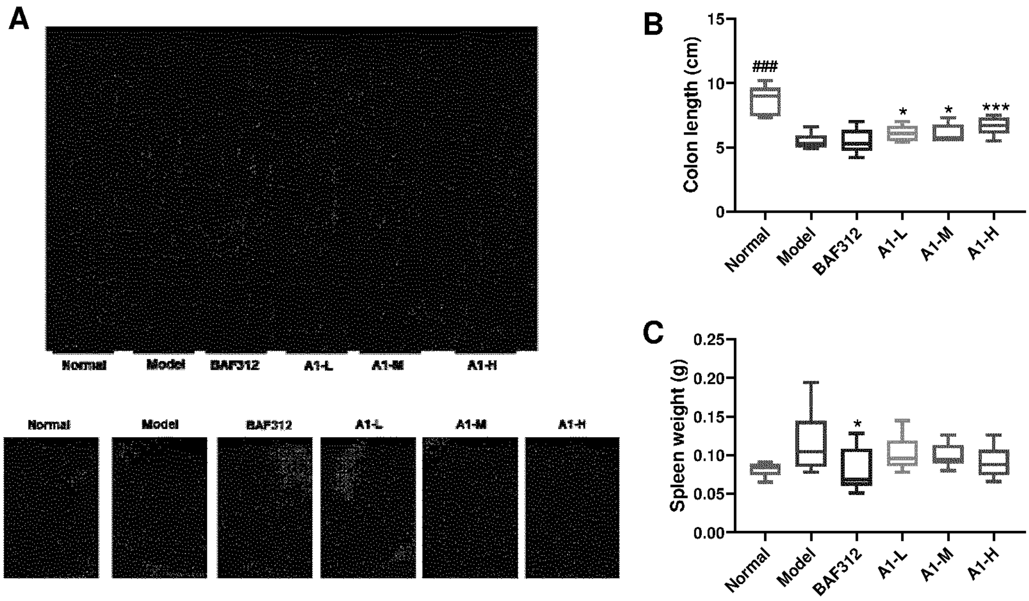
A
Normal
Model
BAF312
A1-L
A1-M
A1-H
B
Colon length (cm)
15
10
5
0
###
*
*
***
C
Spleen weight (g)
0.25
0.20
0.15
0.10
0.05
0.00
D
H&E

Figure 13

**INTERNATIONAL SEARCH REPORT**

International application No.
**PCT/CN2021/103263**

**A. CLASSIFICATION OF SUBJECT MATTER**

C07D 413/14(2006.01)i; C07D 413/12(2006.01)i; C07D 271/06(2006.01)i; A61K 31/4245(2006.01)i; A61P 3/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; CNKI; 万方; STN: 中国科学院上海药物研究所, 柳红, 王江, 谢岑, 刘雅萌, 胡树雷, 李翠娜, 高峰, 王康龙, 汪勇, 钟先春, 石俞强, 蒋华良, 陈凯先, 噁二唑, 1-磷酸鞘氨醇, S1P, 代谢, 炎症, 脂肪肝, 糖尿病, 高血脂, 银屑症, +oxadiazole+, sphingosine 1-phosphate?, metabolism?, inflammat+, fatty liver?, diabete?, hyperlipidemia?, immunity?, psoriasis, structural formula

**C. DOCUMENTS CONSIDERED TO BE RELEVANT**

| Category* | Citation of document, with indication, where appropriate, of the relevant passages | Relevant to claim No. |
|---|---|---|
| X | WO 2014055911 A1 (ALLERGAN, INC.) 10 April 2014 (2014-04-10) claims 1-6, description page 1 lines 13-18, description page 22 lines 5-16, page 24 lines 5-7, page 25 lines 1, 2, page 26 lines 5-13, page 28 lines 5-14, description page 30 | 1-10 |
| X | CN 108939074 A (BEIJING WEILAN ZHIYUAN MEDICAL TECHNOLOGY CO., LTD.) 07 December 2018 (2018-12-07) description paragraph [0056], paragraphs [0085-0087] | 1-9 |
| A | WO 2015073140 A1 (ALLERGAN, INC.) 21 May 2015 (2015-05-21) entire document | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 August 2021** | **15 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/103263**

| Patent document cited in search report | | | Publication date (day/month/year) | Patent family member(s) | | | Publication date (day/month/year) |
|---|---|---|---|---|---|---|---|
| WO | 2014055911 | A1 | 10 April 2014 | EP | 2903984 | A1 | 12 August 2015 |
| | | | | US | 8957051 | B2 | 17 February 2015 |
| | | | | US | 2014100197 | A1 | 10 April 2014 |
| CN | 108939074 | A | 07 December 2018 | WO | 2018214860 | A1 | 29 November 2018 |
| WO | 2015073140 | A1 | 21 May 2015 | None | | | |

Form PCT/ISA/210 (patent family annex) (January 2015)